# EUROPEAN PATENT APPLICATION

(11) **EP 3 744 710 A1**
(43) Date of publication of application: **02.12.2020**
(21) Application number: 19740827.1
(22) Date of filing: 18.01.2019
(51) Int. Cl.: C07C 321/26, C08G 10/04, C09K 3/00, G03F 7/11, G03F 7/20, G03F 7/26

(54) **COMPOUND, RESIN, COMPOSITION, AND PATTERN FORMING METHOD**

(30) Priority: 22.01.2018 JP 2018007892; 26.02.2018 JP 2018032476
(71) Applicant: MITSUBISHI GAS CHEMICAL COMPANY, INC., Chiyoda-ku Tokyo 100-8324 (JP)
(72) Inventor: ECHIGO, Masatoshi, Tokyo 100-8324 (JP); OKADA, Yu, Hiratsuka-shi, Kanagawa 254-0016 (JP); TAKIGAWA, Tomoaki, Tokyo 100-8324 (JP); MAKINOSHIMA, Takashi, Hiratsuka-shi, Kanagawa 254-0016 (JP)
(74) Representative: Müller-Boré & Partner Patentanwälte PartG mbB
(86) International application number: PCT/JP2019/001421
(87) International publication number: WO 2019/142897

(57) **Abstract**

An object of the present invention is to provide a compound and the like that are applicable to a wet process and are useful for forming a photoresist and an underlayer film for photoresists excellent in heat resistance, solubility, and etching resistance. A compound represented by the following formula (1) can solve the problem described above.

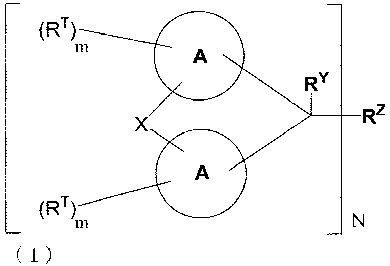

## Description

### Technical Field

The present invention relates to a compound and a resin having a specific structure, and a composition comprising the compound and/or the resin. The present invention relates to pattern formation methods using the composition (a resist pattern formation method and a circuit pattern formation method).

### Background Art

In the production of semiconductor devices, fine processing is practiced by lithography using photoresist materials. In recent years, further miniaturization based on pattern rules has been demanded along with increase in the integration and speed of LSI. The light source for lithography used upon forming resist patterns has been shifted to ArF excimer laser (193 nm) having a shorter wavelength from KrF excimer laser (248 nm). The introduction of extreme ultraviolet (EUV, 13.5 nm) is also expected.

However, because conventional polymer-based resist materials have a molecular weight as large as about 10,000 to 100,000 and also wide molecular weight distribution, in lithography using such a polymer-based resist material, roughness occurs on a pattern surface; the pattern dimension becomes difficult to be controlled; and there is a limitation in miniaturization.

Accordingly, various low molecular weight resist materials have been proposed so far in order to provide resist patterns having higher resolution. The low molecular weight resist materials are expected to provide resist patterns having high resolution and small roughness, because of their small molecular sizes.

Various materials are currently known as such low molecular weight resist materials. For example, an alkaline development type negative type radiation-sensitive composition (see, for example, Patent Literature 1 and Patent Literature 2) using a low molecular weight polynuclear polyphenolic compound as a main component has been suggested; and as a candidate of a low molecular weight resist material having high heat resistance, an alkaline development type negative type radiation-sensitive composition (see, for example, Patent Literature 3 and Non Patent Literature 1) using a low molecular weight cyclic polyphenolic compound as a main component has been suggested as well. Also, as a base compound of a resist material, a polyphenol compound is known to be capable of imparting high heat resistance despite a low molecular weight and useful for improving the resolution and roughness of a resist pattern (see, for example, Non Patent Literature 2).

The present inventors have proposed a resist composition containing a compound having a specific structure and an organic solvent (see Patent Literature 4) as a material that is excellent in etching resistance and is also soluble in a solvent and applicable to a wet process.

Also, as the miniaturization of resist patterns proceeds, the problem of resolution or the problem of collapse of resist patterns after development arises. Therefore, resists have been desired to have a thinner film. However, if resists merely have a thinner film, it is difficult to obtain the film thicknesses of resist patterns sufficient for substrate processing. Therefore, there has been a need for a process of preparing a resist underlayer film between a resist and a semiconductor substrate to be processed, and imparting functions as a mask for substrate processing to this resist underlayer film in addition to a resist pattern.

Various resist underlayer films for such a process are currently known. For example, as a material for realizing resist underlayer films for lithography having the selectivity of a dry etching rate close to that of resists, unlike conventional resist underlayer films having a fast etching rate, an underlayer film forming material for a multilayer resist process containing a resin component having at least a substituent that generates a sulfonic acid residue by eliminating a terminal group under application of predetermined energy, and a solvent has been suggested (see Patent Literature 5). Moreover, as a material for realizing resist underlayer films for lithography having the selectivity of a dry etching rate smaller than that of resists, a resist underlayer film material comprising a polymer having a specific repeat unit has been suggested (see Patent Literature 6). Furthermore, as a material for realizing resist underlayer films for lithography having the selectivity of a dry etching rate smaller than that of semiconductor substrates, a resist underlayer film material comprising a polymer prepared by copolymerizing a repeat unit of an acenaphthylene and a repeat unit having a substituted or unsubstituted hydroxy group has been suggested (see Patent Literature 7).

Meanwhile, as materials having high etching resistance for this kind of resist underlayer film, amorphous carbon underlayer films formed by CVD using methane gas, ethane gas, acetylene gas, or the like as a raw material are well known. However, resist underlayer film materials that can form resist underlayer films by a wet process such as spin coating or screen printing have been demanded from the viewpoint of a process.

The present inventors have proposed an underlayer film forming composition for lithography containing a compound having a specific structure and an organic solvent (see Patent Literature 8) as a material that is excellent in etching resistance, has high heat resistance, and is soluble in a solvent and applicable to a wet process.

As for methods for forming an intermediate layer used in the formation of a resist underlayer film in a three-layer process, for example, a method for forming a silicon nitride film (see Patent Literature 9) and a CVD formation method for a silicon nitride film (see Patent Literature 10) are known. Also, as intermediate layer materials for a three-layer process, materials comprising a silsesquioxane-based silicon compound are known (see Patent Literature 11 and Patent Literature 12).

Various compositions have been further proposed as optical component forming compositions. Examples thereof include acrylic resins (see Patent Literatures 13 and 14).

### Citation List

### Patent Literature

Patent Literature 1: Japanese Patent Laid-Open No. 2005-326838
Patent Literature 2: Japanese Patent Laid-Open No. 2008-145539
Patent Literature 3: Japanese Patent Laid-Open No. 2009-173623
Patent Literature 4: International Publication No. WO 2013/024778
Patent Literature 5: Japanese Patent Laid-Open No. 2004-177668
Patent Literature 6: Japanese Patent Laid-Open No. 2004-271838
Patent Literature 7: Japanese Patent Laid-Open No. 2005-250434
Patent Literature 8: International Publication No. WO 2013/024779
Patent Literature 9: Japanese Patent Laid-Open No. 2002-334869
Patent Literature 10: International Publication No. WO 2004/066377
Patent Literature 11: Japanese Patent Laid-Open No. 2007-226170
Patent Literature 12: Japanese Patent Laid-Open No. 2007-226204
Patent Literature 13: Japanese Patent Laid-Open No. 2010-138393
Patent Literature 14: Japanese Patent Laid-Open No. 2015-174877

### Non Patent Literature

Non Patent Literature 1: T. Nakayama, M. Nomura, K. Haga, M. Ueda: Bull. Chem. Soc. Jpn., 71, 2979 (1998)
Non Patent Literature 2: Shinji Okazaki et al., "New Trends of Photoresists", CMC Publishing Co., Ltd., September 2009, pp. 211-259

### Summary of Invention

### Technical Problem

As mentioned above, a large number of film forming compositions for lithography for resist purposes and film forming compositions for lithography for underlayer film purposes have heretofore been suggested. However, none of these compositions not only have high solvent solubility that permits application of a wet process such as spin coating or screen printing but achieve all of solvent solubility, heat resistance and etching resistance at high dimensions. Thus, the development of novel materials is required.

Also, a large number of compositions for optical members have heretofore been suggested. However, none of these compositions achieve all of heat resistance, transparency, and refractive index at high dimensions. Thus, the development of novel materials is required.

The present invention has been made in order to solve the problems mentioned above. An object of the present invention is to provide a compound, a resin, and a composition (for example, a composition used in film formation for lithography or optical component formation) that are applicable to a wet process and are useful for forming a photoresist and an underlayer film for photoresists excellent in heat resistance, solubility, and etching resistance, as well as pattern formation methods using the composition (a resist pattern formation method and a circuit pattern formation method).

### Solution to Problem

The inventors have, as a result of devoted examinations to solve the above problems, found out that use of a compound or resin having a specific structure can solve the above problems, and reached the present invention. More specifically, the present invention is as follows.
[1] A compound represented by the following formula (1) or (1'): 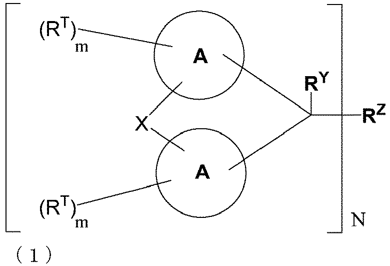 wherein
   R^{Y} is a hydrogen atom, an alkyl group having 1 to 60 carbon atoms and optionally having a substituent and/or a heteroatom, or an aryl group having 6 to 60 carbon atoms and optionally having a substituent and/or a heteroatom;
   R^{Z} is an N-valent group having 1 to 60 carbon atoms and optionally having a substituent and/or a heteroatom, or a single bond;
   or alternatively, R^{Y} and R^{Z} may form, including a carbon atom to which they are bonded, a 4-membered to 30-membered ring optionally having a substituent and/or a heteroatom;
   A is a group exhibiting aromaticity and having 1 to 60 carbon atoms and optionally having a substituent and/or a heteroatom;
   each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a carboxylic acid group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond; and
   X is an oxygen atom, a sulfur atom or not a crosslink;
   wherein
   at least one selected from A, R^{Y}, R^{Z}, R^{T} and X contains a sulfur atom;
   at least one R^{T} contains a thiol group, a hydroxy group, or a group in which a hydrogen atom of a thiol group or a hydroxy group is replaced with an acid crosslinking group or an acid dissociation group;
   each m is independently an integer of 0 to 9; and
   N is an integer of 1 to 4, wherein when N is an integer of 2 or larger, N structural formulas within the parentheses [ ] are the same or different, or 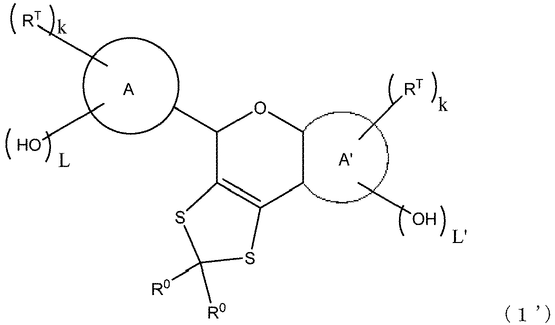 wherein
      each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond;
      each R⁰ is independently a hydrogen atom, an alkyl group having 1 to 30 carbon atoms and optionally having a substituent and/or a heteroatom, or an aryl group having 6 to 30 carbon atoms and optionally having a substituent and/or a heteroatom;
      or alternatively, two R⁰ may form, including a carbon atom to which they are bonded, a 4-membered to 30-membered ring optionally having a substituent and/or a heteroatom; or two R⁰ are a double bond bonded to a carbon atom to which they are bonded, wherein an alkyl group having 1 to 30 carbon atoms and optionally having a substituent and/or a heteroatom, or an aryl group having 6 to 30 carbon atoms and optionally having a substituent and/or a heteroatom may be bonded to the double bond;
      A and A' are each a group exhibiting aromaticity and having 1 to 60 carbon atoms and optionally having a substituent and/or a heteroatom;
      L is an integer of 1 to 9; and
      k and L' are each independently an integer of 0 to 9.
[2] The compound according to [1], wherein the compound represented by the above formula (1) is a compound represented by the following formula (1-1): 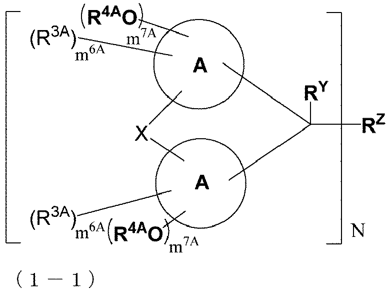 wherein
   R^{Y}, R^{Z}, A, X, and N are as defined in [1];
   each R^{3A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, a halogen atom, a nitro group, an amino group, a carboxylic acid group, or a thiol group; and
   each R^{4A} is independently a hydrogen atom, an acid crosslinking group, or an acid dissociation group;
   wherein, at least one selected from A, R^{Y}, R^{Z}, R^{3A}, R^{4A}, and X contains a sulfur atom;
   each m^{6A} is independently an integer of 0 to 5; and
   each m^{7A} is independently an integer of 0 to 5,
   provided that two m^{7A} are not 0 at the same time.
[3] The compound according to [1], wherein the compound represented by the above formula (1) is a compound represented by the following formula (2): 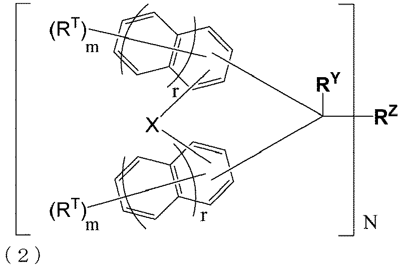 wherein
   R^{Y} is a hydrogen atom, an alkyl group having 1 to 30 carbon atoms and optionally having a substituent, or an aryl group having 6 to 30 carbon atoms and optionally having a substituent;
   R^{Z} is an N-valent group having 1 to 60 carbon atoms or a single bond;
   each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a thiol group, a hydroxy group, or a group in which a hydrogen atom of a hydroxy group is replaced with an acid dissociation group, wherein the alkyl group, the alkenyl group, the alkynyl group and the aryl group each optionally contain an ether bond, a ketone bond or an ester bond and wherein at least one R^{T} is a thiol group;
   X is an oxygen atom, a sulfur atom or not a crosslink;
   each m is independently an integer of 0 to 9, wherein at least one m is an integer of 1 to 9;
   N is an integer of 1 to 4, wherein when N is an integer of 2 or larger, N structural formulas within the parentheses [ ] are the same or different; and
   each r is independently an integer of 0 to 2.
[4] The compound according to [3], wherein the compound represented by the above formula (2) is a compound represented by the following formula (3): 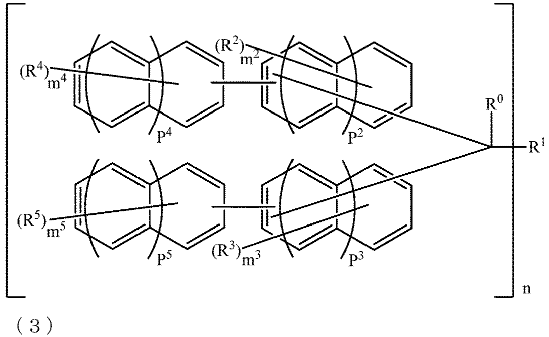 wherein
   R⁰ is as defined in R^{Y} in [3];
   R¹ is an n-valent group having 1 to 60 carbon atoms or a single bond;
   R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one of R² to R⁵ is a thiol group;
   m² and m³ are each independently an integer of 0 to 8;
   m⁴ and m⁵ are each independently an integer of 0 to 9, provided that m², m³, m⁴, and m⁵ are not 0 at the same time;
   n is as defined in N in [3], wherein when n is an integer of 2 or larger, n structural formulas within the parentheses [ ] are the same or different; and
   p² to p⁵ are as defined in r in [3].
[5] The compound according to [3], wherein the compound represented by the above formula (2) is a compound represented by the following formula (4): 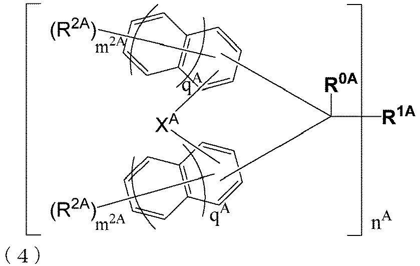 wherein
   R^{0A} is as defined in R^{Y} in [3];
   R^{1A} is an n^{A}-valent group having 1 to 60 carbon atoms or a single bond;
   each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one R^{2A} is a thiol group;
   n^{A} is as defined in N in [3], where when n^{A} is an integer of 2 or larger, n^{A} structural formulas within the parentheses [ ] are the same or different;
   X^{A} is an oxygen atom, a sulfur atom or not a crosslink;
   each m^{2A} is independently an integer of 0 to 7, provided that at least one m^{2A} is an integer of 1 to 7; and
   each q^{A} is independently 0 or 1.
[6] The compound according to [4], wherein the compound represented by the above formula (3) is a compound represented by the following formula (3-1): 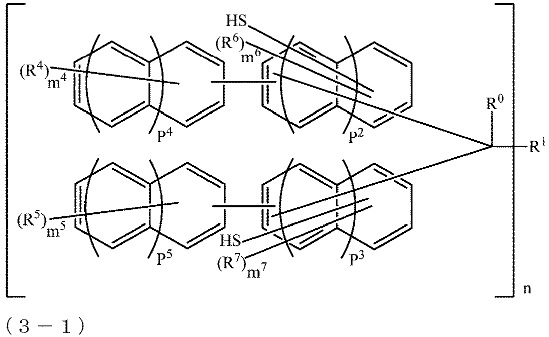 wherein
   R⁰, R¹, R⁴, R⁵, n, p² to p⁵, m⁴, and m⁵ are as defined in [4];
   R⁶ and R⁷ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; and
   m⁶ and m⁷ are each independently an integer of 0 to 7.
[7] The compound according to [6], wherein the compound represented by the above formula (3-1) is a compound represented by the following formula (3-2): 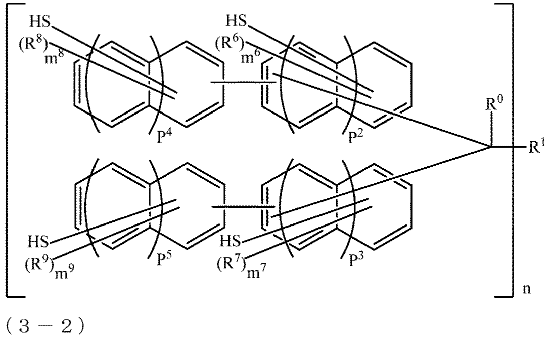 wherein
   R⁰, R¹, n, and p² to p⁵ are as defined in [4];
   R⁶, R⁷, m⁶, and m⁷ are as defined in [6];
   R⁸ and R⁹ are as defined in R⁶ and R⁷; and
   m⁸ and m⁹ are each independently an integer of 0 to 8.
[8] The compound according to [5], wherein the compound represented by the above formula (4) is a compound represented by the following formula (4-1): 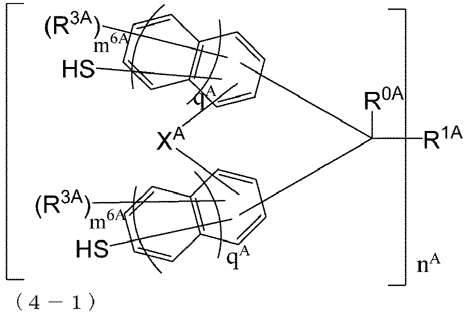 wherein
   R^{0A}, R^{1A}, n^{A}, q^{A}, and X^{A} are as defined in [5];
   each R^{3A} is independently a halogen atom, an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, or an alkoxy group having 1 to 30 carbon atoms and optionally having a substituent; and
   each m^{6A} is independently an integer of 0 to 5.
[9] A resin comprising the compound according to [1] or [2] as a constituent unit.
[10] The resin according to [9], wherein the resin is represented by the following formula (5): 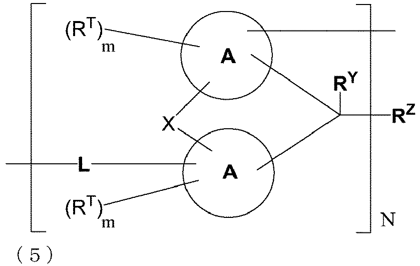 wherein
   R^{Y}, R^{Z}, A, R^{T}, X, and N are as defined in [1]; and
   each m is independently an integer of 0 to 8;
   wherein
   at least one selected from A, R^{Y}, R^{Z}, R^{T} and X contains a sulfur atom;
   at least one R^{T} contains a thiol group, a hydroxy group, or a group in which a hydrogen atom of a thiol group or a hydroxy group is replaced with an acid crosslinking group or an acid dissociation group;
   when N is an integer of 2 or larger, N structural formulas within the parentheses [ ] are the same or different; and
   L is an alkylene group having 1 to 30 carbon atoms and optionally having a substituent and/or a heteroatom, an arylene group having 6 to 30 carbon atoms and optionally having a substituent and/or a heteroatom, an alkoxylene group having 1 to 30 carbon atoms and optionally having a substituent and/or a heteroatom, or a single bond, wherein the alkylene group, the arylene group and the alkoxylene group each optionally contain an ether bond, a thioether bond, a ketone bond or an ester bond.
[11] A resin comprising the compound according to any of [3] to [8] as a constituent unit.
[12] The resin according to [11], wherein the resin has a structure represented by the following formula (6): 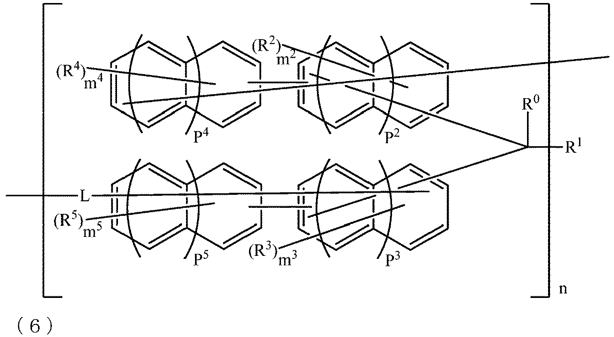 wherein
   L is a linear or branched alkylene group having 1 to 30 carbon atoms and optionally having a substituent, or a single bond;
   R⁰ is as defined in R^{Y} in [3];
   R¹ is an n-valent group having 1 to 60 carbon atoms or a single bond;
   R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one of R² to R⁵ is a thiol group;
   m² and m³ are each independently an integer of 0 to 8;
   m⁴ and m⁵ are each independently an integer of 0 to 9, provided that m², m³, m⁴, and m⁵ are not 0 at the same time;
   n is as defined in N in [3], wherein when n is an integer of 2 or larger, n structural formulas within the parentheses [ ] are the same or different; and
   p² to p⁵ are as defined in r in [3].
[13] The resin according to [11], wherein the resin has a structure represented by the following formula (7): 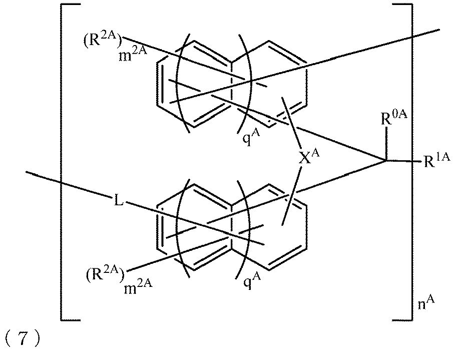 wherein
   L is a linear or branched alkylene group having 1 to 30 carbon atoms and optionally having a substituent, or a single bond;
   R^{0A} is as defined in R^{Y} in [3];
   R^{1A} is an n^{A}-valent group having 1 to 60 carbon atoms or a single bond;
   each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one R^{2A} is a thiol group;
   n^{A} is as defined in N in [3], where when n^{A} is an integer of 2 or larger, n^{A} structural formulas within the parentheses [ ] are the same or different;
   X^{A} is an oxygen atom, a sulfur atom or not a crosslink;
   each m^{2A} is independently an integer of 0 to 7, provided that at least one m^{2A} is an integer of 1 to 6; and
   each q^{A} is independently 0 or 1.
[14] A composition comprising one or more selected from the group consisting of the compound according to any of [1] to [8] and the resin according to any of [9] to [13].
[15] The composition according to [14], further comprising a solvent.
[16] The composition according to [14] or [15], further comprising an acid generating agent.
[17] The composition according to any of [14] to [16], further comprising an acid crosslinking agent.
[18] The composition according to any of [14] to [17], further comprising a radical generating agent.
[19] The composition according to any of [14] to [18], wherein the composition is used in film formation for lithography.
[20] The composition according to [19], wherein the composition is used in resist underlayer film formation.
[21] The composition according to [19], wherein the composition is used in resist film formation.
[22] The composition according to [19], wherein the composition is used in resist permanent film formation.
[23] The composition according to any of [14] to [18], wherein the composition is used in optical component formation.
[24] A method for forming a resist pattern, comprising the steps of:
   forming a photoresist layer on a substrate using the composition according to [19]; and
   irradiating a predetermined region of the photoresist layer with radiation for development.
[25] A method for forming an insulating film, comprising the steps of:
   forming a photoresist layer on a substrate using the composition according to [19]; and
   irradiating a predetermined region of the photoresist layer with radiation for development.
[26] A method for forming a resist pattern, comprising the steps of:
   forming an underlayer film on a substrate using the composition according to [19];
   forming at least one photoresist layer on the underlayer film; and
   irradiating a predetermined region of the photoresist layer with radiation for development.
[27] A method for forming a circuit pattern, comprising the steps of:
   forming an underlayer film on a substrate using the composition according to [19];
   forming an intermediate layer film on the underlayer film using a resist intermediate layer film material;
   forming at least one photoresist layer on the intermediate layer film;
   irradiating a predetermined region of the photoresist layer with radiation for development, thereby forming a resist pattern;
   etching the intermediate layer film with the resist pattern as a mask;
   etching the underlayer film with the obtained intermediate layer film pattern as an etching mask; and
   etching the substrate with the obtained underlayer film pattern as an etching mask, thereby forming a pattern on the substrate.
[28] A method for purifying the compound according to any of [1] to [8] or the resin according to any of [9] to [13], comprising:
   an extraction step in which extraction is carried out by bringing a solution containing the compound or resin, and an organic solvent that does not inadvertently mix with water into contact with an acidic aqueous solution.

### Advantageous Effects of Invention

The present invention can provide a compound, a resin, and a composition (for example, a composition used in film formation for lithography or optical component formation) that are applicable to a wet process and are useful for forming a photoresist and an underlayer film for photoresists excellent in heat resistance, solubility, and etching resistance, as well as pattern formation methods using the composition (a resist pattern formation method and a circuit pattern formation method).

### Description of Embodiments

As mentioned later, the compound and the resin of the present embodiment have high solubility in a safe solvent and have good heat resistance and etching resistance. The resist composition of the present embodiment imparts a good shape to a resist pattern.

Also, the compound and the resin of the present embodiment are applicable to a wet process and can achieve a compound, a resin, and a film forming composition for lithography that are useful for forming a photoresist underlayer film excellent in heat resistance and etching resistance. Furthermore, this film forming composition for lithography employs the compound or the resin having high heat resistance and also high solvent solubility and having a specific structure and can therefore form a resist and an underlayer film that is prevented from deteriorating during high temperature baking and is also excellent in etching resistance against oxygen plasma etching or the like. In addition, the underlayer film thus formed is also excellent in adhesiveness to a resist layer and can therefore form an excellent resist pattern. Moreover, the composition has high refractive index and is prevented from being stained by heat treatment in a wide range from a low temperature to a high temperature. Therefore, the composition is also useful as various optical forming compositions.

Hereinafter, embodiments of the present invention will be described. The embodiments described below are given merely for illustrating the present invention. The present invention is not limited only by these embodiments.

As for structural formulas described in the present specification, for example, when a line indicating a bond to C is in contact with a ring A and a ring B as described below, C is meant to be bonded to any one or both of the ring A and the ring B.

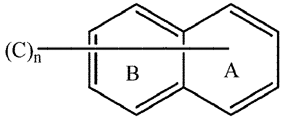

### [Compound represented by formula (1)]

The compound of the present invention is represented by the following formula (1), or the formula (1'), which will be mentioned later. Since the compound of the present invention has such a structure, it has high heat resistance, relatively high carbon concentration, relatively low oxygen concentration, and high solvent solubility. In addition, the compound of the present invention contains a sulfur atom, and thus has a high refractive index. Furthermore, the compound of the present invention has a low viscosity before being cured, and is excellent in smoothing properties. 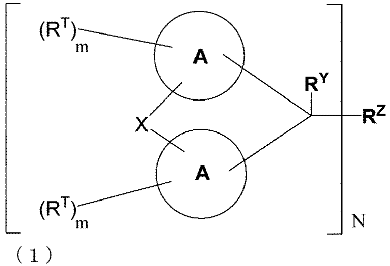 (In the formula (1),
R^{Y} is a hydrogen atom, an alkyl group having 1 to 60 carbon atoms and optionally having a substituent and/or a heteroatom, or an aryl group having 6 to 60 carbon atoms and optionally having a substituent and/or a heteroatom;
R^{Z} is an N-valent group having 1 to 60 carbon atoms and optionally having a substituent and/or a heteroatom, or a single bond;
or alternatively, R^{Y} and R^{Z} may form, including a carbon atom to which they are bonded, a 4-membered to 30-membered ring optionally having a substituent and/or a heteroatom;
A is a group exhibiting aromaticity and having 1 to 60 carbon atoms and optionally having a substituent and/or a heteroatom;
each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a carboxylic acid group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond; and
X is an oxygen atom, a sulfur atom or not a crosslink;
wherein
at least one selected from A, R^{Y}, R^{Z}, R^{T} and X contains a sulfur atom;
at least one R^{T} contains a thiol group, a hydroxy group, or a group in which a hydrogen atom of a thiol group or a hydroxy group is replaced with an acid crosslinking group or an acid dissociation group;
each m is independently an integer of 0 to 9; and
N is an integer of 1 to 4, wherein when N is an integer of 2 or larger, N structural formulas within the parentheses [ ] are the same or different.)

Unless otherwise specified, the term "alkyl group" includes linear, branched and cyclic alkyl groups.

Examples of the "alkyl group" include a methyl group, an ethyl group, a n-propyl group, an i-propyl group, a n-butyl group, an i-butyl group, a t-butyl group, a cyclopropyl group, and a cyclobutyl group.

Examples of the substituent for the alkyl group include a halogen atom, a nitro group, an amino group, a thiol group, a hydroxy group, and a group in which a hydrogen atom of a thiol group or a hydroxy group is replaced with an acid crosslinking group or an acid dissociation group.

Examples of the "aryl group" include a phenyl group and a naphthyl group.

Examples of the substituent for the aryl group include a halogen atom, a nitro group, an amino group, a thiol group, a hydroxy group, a phenyl group, or a group in which a hydrogen atom of a thiol group and a hydroxy group is replaced with an acid crosslinking group or an acid dissociation group.

Examples of the "alkenyl group" include a propenyl group and a butenyl group.

Examples of the substituent for the alkenyl group include a halogen atom, a nitro group, an amino group, a thiol group, a hydroxy group, and a group in which a hydrogen atom of a thiol group or a hydroxy group is replaced with an acid crosslinking group or an acid dissociation group.

Examples of the "alkynyl group" include a propargyl group and a butanyl group.

Examples of the substituent for the alkynyl group include a halogen atom, a nitro group, an amino group, a thiol group, a hydroxy group, and a group in which a hydrogen atom of a thiol group or a hydroxy group is replaced with an acid crosslinking group or an acid dissociation group.

Examples of the "alkoxy group" include a methoxy group, an ethoxy group, a propoxy group, a cyclohexyloxy group, a phenoxy group, and a naphthoxy group.

Examples of the substituent for the alkoxy group include a halogen atom, a nitro group, an amino group, a thiol group, a hydroxy group, and a group in which a hydrogen atom of a thiol group or a hydroxy group is replaced with an acid crosslinking group or an acid dissociation group.

Examples of the "alkylthio" include a methylthio group, an ethylthio group, a n-propylthio group, an i-propylthio group, a n-butylthio group, an i-butylthio group, a t-butylthio group, a cyclopropylthio group, and a cyclobutylthio group.

Examples of the substituent for the alkylthio group include a halogen atom, a nitro group, an amino group, a thiol group, a hydroxy group, and a group in which a hydrogen atom of a thiol group or a hydroxy group is replaced with an acid crosslinking group or an acid dissociation group.

Examples of the "halogen atom" include a fluorine atom, a chlorine atom, a bromine atom, and an iodine atom.

Examples of the "heteroatom" include a sulfur atom, a nitrogen atom, and an oxygen atom.

The "acid dissociation group" refers to a characteristic group that is cleaved in the presence of an acid to generate a functional group that alters solubility, such as an alkali soluble group. Examples of the alkali soluble group include, but not particularly limited to, a phenolic hydroxy group, a carboxyl group, a sulfonic acid group, and a hexafluoroisopropanol group. Among them, a phenolic hydroxy group and a carboxyl group are preferable, and a phenolic hydroxy group is particularly preferable, from the viewpoint of the easy availability of an introduction reagent.

The acid dissociation reactive group preferably has the property of causing chained cleavage reaction in the presence of an acid, for achieving pattern formation with high sensitivity and high resolution. The acid dissociation reactive group is not particularly limited, but can be arbitrarily selected for use from among, for example, those proposed in hydroxystyrene based resins, (meth)acrylic acid based resins, and the like for use in chemically amplified resist compositions for KrF or ArF.

Preferable examples of the acid dissociation reactive group include a group selected from the group consisting of a substituted methyl group, a 1-substituted ethyl group, a 1-substituted n-propyl group, a 1-branched alkyl group, a silyl group, an acyl group, a 1-substituted alkoxymethyl group, a cyclic ether group, an alkoxycarbonyl group, and an alkoxycarbonylalkyl group which have the property of being dissociated by an acid. From the viewpoint of roughness, it is preferable that the acid dissociation reactive group has no crosslinkable functional group.

The substituted methyl group is not particularly limited, but is usually a substituted methyl group having 2 to 20 carbon atoms and is preferably a substituted methyl group having 4 to 18 carbon atoms and more preferably a substituted methyl group having 6 to 16 carbon atoms. Specific examples of the substituted methyl group can include, but not limited to, a methoxymethyl group, a methylthiomethyl group, an ethoxymethyl group, a n-propoxymethyl group, an isopropoxymethyl group, a n-butoxymethyl group, a t-butoxymethyl group, a 2-methylpropoxymethyl group, an ethylthiomethyl group, a methoxyethoxymethyl group, a phenyloxymethyl group, a 1-cyclopentyloxymethyl group, a 1-cyclohexyloxymethyl group, a benzylthiomethyl group, a phenacyl group, a 4-bromophenacyl group, a 4-methoxyphenacyl group, a piperonyl group, and a substituent group represented by the following formula (Y-1).

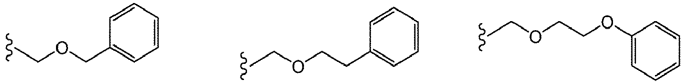

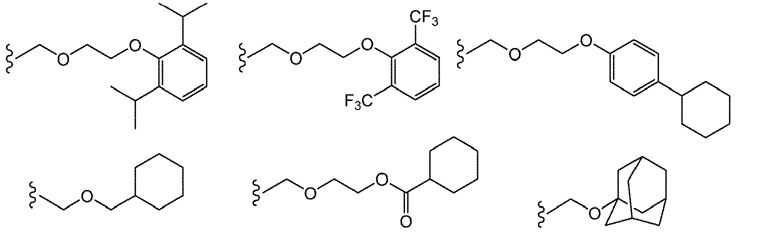

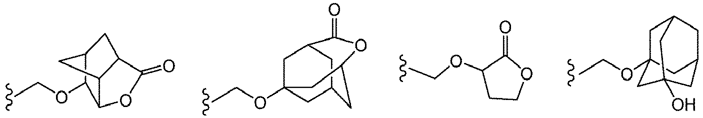

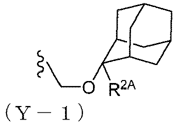

In the above formula (Y-1), R^{2A} is an alkyl group having 1 to 4 carbon atoms. Specific examples of R^{2A} include, but not limited to, a methyl group, an ethyl group, an isopropyl group, a n-propyl group, a t-butyl group, and a n-butyl group.

The 1-substituted ethyl group is not particularly limited, but is usually a 1-substituted ethyl group having 3 to 20 carbon atoms and is preferably a 1-substituted ethyl group having 5 to 18 carbon atoms and more preferably a substituted ethyl group having 7 to 16 carbon atoms. Specific examples of the 1-substituted ethyl group can include, but not limited to, a 1-methoxyethyl group, 1-methylthioethyl group, a 1,1-dimethoxyethyl group, a 1-ethoxyethyl group, a 1-ethylthioethyl group, a 1,1-diethoxyethyl group, a n-propoxyethyl group, an isopropoxyethyl group, a n-butoxyethyl group, a t-butoxyethyl group, a 2-methylpropoxyethyl group, a 1-phenoxyethyl group, a 1-phenylthioethyl group, a 1,1-diphenoxyethyl group, a 1-cyclopentyloxyethyl group, a 1-cyclohexyloxyethyl group, a 1-phenylethyl group, a 1,1-diphenylethyl group, and a substituent group represented by the following formula (Y-2).

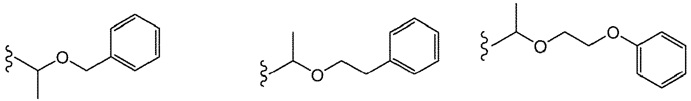

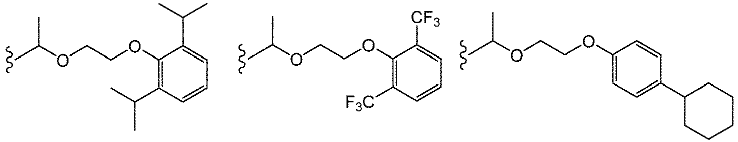

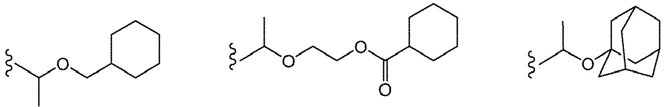

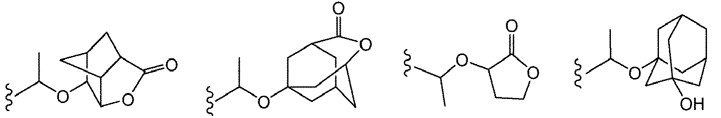

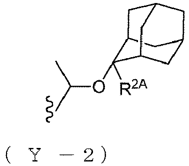

In the above formula (Y-2), R^{2A} is as defined above.

The 1-substituted n-propyl group is not particularly limited, but is usually a 1-substituted n-propyl group having 4 to 20 carbon atoms and is preferably a 1-substituted n-propyl group having 6 to 18 carbon atoms and more preferably a 1-substituted n-propyl group having 8 to 16 carbon atoms. Specific examples of the 1-substituted n-propyl group can include, but not limited to, a 1-methoxy-n-propyl group and a 1-ethoxy-n-propyl group.

The 1-branched alkyl group is not particularly limited, but is usually a 1-branched alkyl group having 3 to 20 carbon atoms and is preferably a 1-branched alkyl group having 5 to 18 carbon atoms and more preferably a branched alkyl group having 7 to 16 carbon atoms. Specific examples of the 1-branched alkyl group can include, but not limited to, an isopropyl group, a sec-butyl group, a tert-butyl group, a 1,1-dimethylpropyl group, a 1-methylbutyl group, a 1,1-dimethylbutyl group, a 2-methyladamantyl group, and a 2-ethyladamantyl group.

The silyl group is not particularly limited, but is usually a silyl group having 1 to 20 carbon atoms and is preferably a silyl group having 3 to 18 carbon atoms and more preferably a silyl group having 5 to 16 carbon atoms. Specific examples of the silyl group can include, but not limited to, a trimethylsilyl group, an ethyldimethylsilyl group, a methyldiethylsilyl group, a triethylsilyl group, a tert-butyldimethylsilyl group, a tert-butyldiethylsilyl group, a tert-butyldiphenylsilyl group, a tri-tert-butylsilyl group, and a triphenylsilyl group.

The acyl group is not particularly limited, but is usually an acyl group having 2 to 20 carbon atoms and is preferably an acyl group having 4 to 18 carbon atoms and more preferably an acyl group having 6 to 16 carbon atoms. Specific examples of the acyl group can include, but not limited to, an acetyl group, a phenoxyacetyl group, a propionyl group, a butyryl group, a heptanoyl group, a hexanoyl group, a valeryl group, a pivaloyl group, an isovaleryl group, a lauroyl group, an adamantylcarbonyl group, a benzoyl group, and a naphthoyl group.

The 1-substituted alkoxymethyl group is not particularly limited, but is usually a 1-substituted alkoxymethyl group having 2 to 20 carbon atoms and is preferably a 1-substituted alkoxymethyl group having 4 to 18 carbon atoms and more preferably a 1-substituted alkoxymethyl group having 6 to 16 carbon atoms. Specific examples of the 1-substituted alkoxymethyl group can include, but not limited to, a 1-cyclopentylmethoxymethyl group, a 1-cyclopentylethoxymethyl group, a 1-cyclohexylmethoxymethyl group, a 1-cyclohexylethoxymethyl group, a 1-cyclooctylmethoxymethyl group, and a 1-adamantylmethoxymethyl group.

The cyclic ether group is not particularly limited, but is usually a cyclic ether group having 2 to 20 carbon atoms and is preferably a cyclic ether group having 4 to 18 carbon atoms and more preferably a cyclic ether group having 6 to 16 carbon atoms. Specific examples of the cyclic ether group can include, but not limited to, a tetrahydropyranyl group, a tetrahydrofuranyl group, a tetrahydrothiopyranyl group, a tetrahydrothiofuranyl group, a 4-methoxytetrahydropyranyl group, and a 4-methoxytetrahydrothiopyranyl group.

The alkoxycarbonyl group is usually an alkoxycarbonyl group having 2 to 20 carbon atoms and is preferably an alkoxycarbonyl group having 4 to 18 carbon atoms and more preferably an alkoxycarbonyl group having 6 to 16 carbon atoms. Specific examples of the alkoxycarbonyl group can include, but not limited to, a methoxycarbonyl group, an ethoxycarbonyl group, a n-propoxycarbonyl group, an isopropoxycarbonyl group, a n-butoxycarbonyl group, a tert-butoxycarbonyl group, and a group of acid dissociation reactive groups represented by the following formula (Y-3) wherein n = 0.

The alkoxycarbonylalkyl group is not particularly limited, but is usually an alkoxycarbonylalkyl group having 2 to 20 carbon atoms and is preferably an alkoxycarbonylalkyl group having 4 to 18 carbon atoms and more preferably an alkoxycarbonylalkyl group having 6 to 16 carbon atoms. Specific examples of the alkoxycarbonylalkyl group can include, but not limited to, a methoxycarbonylmethyl group, an ethoxycarbonylmethyl group, a n-propoxycarbonylmethyl group, an isopropoxycarbonylmethyl group, a n-butoxycarbonylmethyl group, and a group of acid dissociation reactive groups represented by the following formula (Y-3) wherein n = 1 to 4.

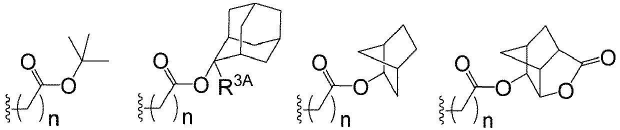

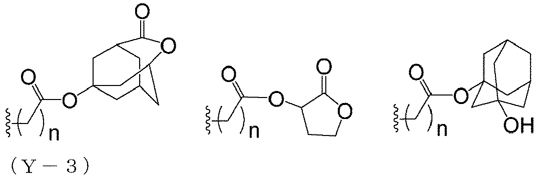

In the above formula (Y-3), R^{3A} is a hydrogen atom or a linear or branched alkyl group having 1 to 4 carbon atoms; and n is an integer of 0 to 4.

Among these acid dissociation reactive groups, a substituted methyl group, a 1-substituted ethyl group, a 1-substituted alkoxymethyl group, a cyclic ether group, an alkoxycarbonyl group, and an alkoxycarbonylalkyl group are preferable. From the viewpoint of exerting higher sensitivity, a substituted methyl group, a 1-substituted ethyl group, an alkoxycarbonyl group, and an alkoxycarbonylalkyl group are more preferable, and an acid dissociation reactive group having a structure selected from a cycloalkane having 3 to 12 carbon atoms, a lactone, and an aromatic ring having 6 to 12 carbon atoms is further preferable.

The cycloalkane having 3 to 12 carbon atoms may be monocyclic or polycyclic and is preferably polycyclic.

Specific examples of the cycloalkane having 3 to 12 carbon atoms include, but not limited to, monocycloalkanes, bicycloalkanes, tricycloalkanes, and tetracycloalkanes. More specific examples thereof include, but not limited to: monocycloalkanes such as cyclopropane, cyclobutane, cyclopentane, and cyclohexane; and polycycloalkanes such as adamantane, norbornane, isobornane, tricyclodecane, and tetracyclodecane. Among them, adamantane, tricyclodecane, and tetracyclodecane are preferable, and adamantane and tricyclodecane are more preferable. The cycloalkane having 3 to 12 carbon atoms may have a substituent group.

Examples of the lactone include, but not limited to, a butyrolactone or cycloalkane groups having 3 to 12 carbon atoms and having lactone group.

Examples of the aromatic ring having 6 to 12 carbon atoms include, but not limited to, a benzene ring, a naphthalene ring, an anthracene ring, a phenanthrene ring, and a pyrene ring. A benzene ring and a naphthalene ring are preferable, and a naphthalene ring is more preferable.

Among those described above, an acid dissociation reactive group selected from the group consisting of groups represented by the following formula (Y-4) is particularly preferable because of a tendency that resolution is higher.

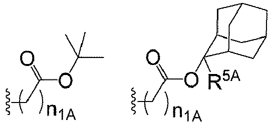

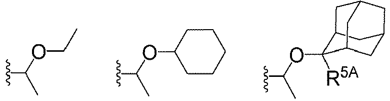

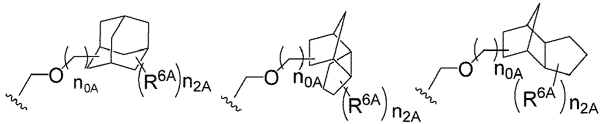

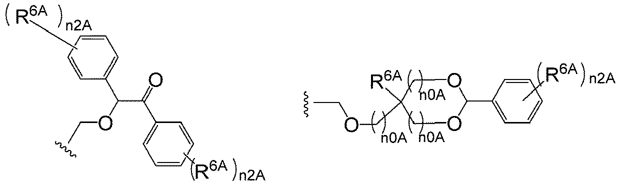

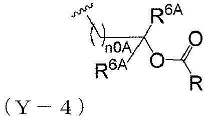

In the above formula (Y-4), R^{5A} is a hydrogen atom or a linear or branched alkyl group having 1 to 4 carbon atoms; R^{6A} is a hydrogen atom, a linear or branched alkyl group having 1 to 4 carbon atoms, a cyano group, a nitro group, a heterocyclic group, a halogen atom, or a carboxyl group; n_{1A} is an integer of 0 to 4; n_{2A} is an integer of 1 to 5; and n_{0A} is an integer of 0 to 4.

In the present specification, the "acid crosslinking group" is a group that crosslinks in the presence of a catalyst or without a catalyst. Examples of the acid crosslinking group include, but not particularly limited to, an alkoxy group having 1 to 20 carbon atoms, a group having an allyl group, a group having a (meth)acryloyl group, a group having an epoxy (meth)acryloyl group, a group having a hydroxy group, a group having a urethane (meth)acryloyl group, a group having a glycidyl group, a group having a vinyl-containing phenylmethyl group, a group having various alkynyl groups, a group having a carbon-carbon double bond, and a group having a carbon-carbon triple bond.

Examples of the group having an allyl group include, but not particularly limited to, a group represented by the following general formula (X-1). 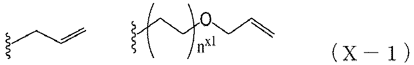 (In the general formula (X-1), n^{X1} is an integer of 1 to 5.)

Examples of the group having a (meth)acryloyl group include, but not particularly limited to, a group represented by the following general formula (X-2). 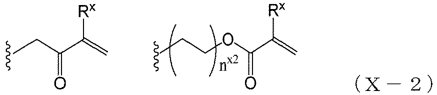 (In the general formula (X-2), n^{X2} is an integer of 1 to 5; and R^{X} is a hydrogen atom or a methyl group.)

Examples of the group having an epoxy (meth)acryloyl group include, but not particularly limited to, a group represented by the following general formula (X-3). Here, the epoxy (meth)acryloyl group refers to a group generated by having an epoxy (meth)acrylate to react with a hydroxy group. 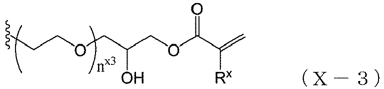 (In the general formula (X-3), n^{X3} is an integer of 0 to 5; and R^{X} is a hydrogen atom or a methyl group.)

Examples of the group having a urethane (meth)acryloyl group include, but not particularly limited to, a group represented by the following general formula (X-4). 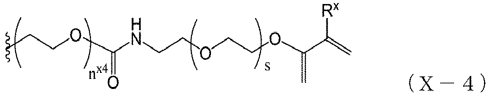 (In the general formula (X-4), n^{X4} is an integer of 0 to 5; s is an integer of 0 to 3; and R^{X} is a hydrogen atom or a methyl group.)

Examples of the group having a hydroxy group include, but not particularly limited to, a group represented by the following general formula (X-5). 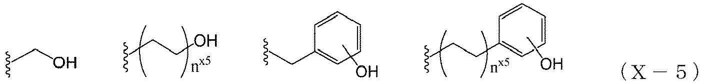 (In the general formula (X-5), n^{X5} is an integer of 1 to 5.)

Examples of the group having a glycidyl group include, but not particularly limited to, a group represented by the following general formula (X-6). 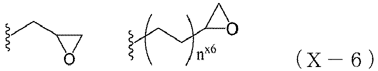 (In the general formula (X-6), n^{X6} is an integer of 1 to 5.)

Examples of the group having a vinyl-containing phenylmethyl group include, but not particularly limited to, a group represented by the following general formula (X-7) . 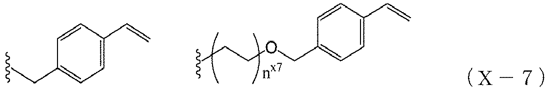 (In the general formula (X-7), n^{X7} is an integer of 1 to 5.)

Examples of the group having various alkynyl groups include, but not particularly limited to, a group represented by the following general formula (X-8). 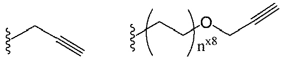 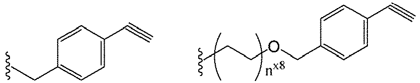 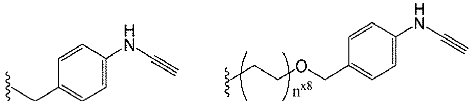 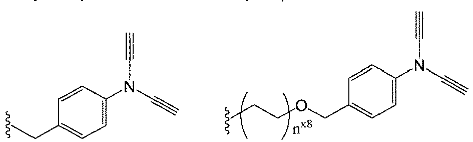 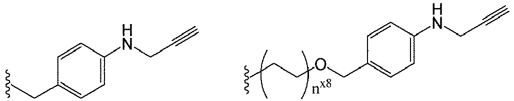 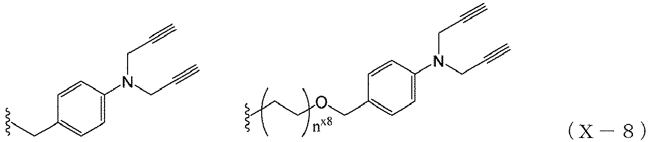 (In the general formula (X-8), n^{X8} is an integer of 1 to 5.)

Examples of the above carbon-carbon double bond containing group include a (meth)acryloyl group, a substituted or unsubstituted vinylphenyl group, and a group represented by the following formula (X-9-1). In addition, examples of the above carbon-carbon triple bond containing group include a substituted or unsubstituted ethynyl group, a substituted or unsubstituted propargyl group, and a group represented by the following formulas (X-9-2) and (X-9-3).

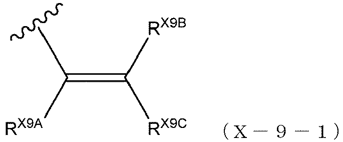

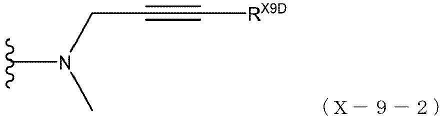

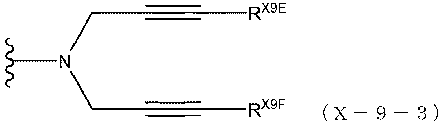

In the above formula (X-9-1), R^{X9A}, R^{X9B} and R^{X9C} are each independently a hydrogen atom or a monovalent hydrocarbon group having 1 to 20 carbon atoms. In the above formulas (X-9-2) and (X-9-3), R^{X9D}, R^{X9E} and R^{X9F} are each independently a hydrogen atom or a monovalent hydrocarbon group having 1 to 20 carbon atoms.

Among the above, a group having a (meth)acryloyl group, an epoxy (meth)acryloyl group, a urethane (meth)acryloyl group, or a glycidyl group, and a group containing a styrene group are preferable; a (meth)acryloyl group, an epoxy (meth)acryloyl group, and a urethane (meth)acryloyl group are more preferable; and a (meth)acryloyl group is further preferable, from the viewpoint of ultraviolet curability. In addition, from the viewpoint of heat resistance, a group having various alkynyl groups are also preferable.

R^{Y} is preferably a hydrogen atom or an alkyl group having 1 to 6 carbon atoms and optionally having a substituent and/or a heteroatom, more preferably a hydrogen atom or an alkyl group having 1 to 6 carbon atoms, and further preferably a hydrogen atom or an alkyl group having 1 to 3 carbon atoms.

R^{Z} is preferably an N-valent group having 1 to 30 carbon atoms, more preferably an N-valent group having 3 to 30 carbon atoms and containing a ring, further preferably an N-valent group having 3 to 12 carbon atoms and containing a ring, and particularly preferably an N-valent group having 3 to 12 carbon atoms and containing an aromatic ring. The N-valent group may contain a sulfur atom. The ring or the aromatic ring may contain a sulfur atom and/or a nitrogen atom as a ring member atom. The ring or the aromatic ring may be substituted by a substituent containing a sulfur atom (for example, an alkylthio group), a hydroxy group, a phenyl group, or an alkyl group having 1 to 6 carbon atoms. The N-valence is preferably monovalence to trivalence, and more preferably monovalence or divalence.

R^{Z} may be, for example, an alkyl group having 1 to 60 carbon atoms, an alkylene group having 1 to 60 carbon atoms, an alkanetriyl group having 2 to 60 carbon atoms, an alkanetetrayl group having 3 to 60 carbon atoms, or an aromatic group having 6 to 60 carbon atoms. These groups may have an alicyclic hydrocarbon group, a double bond, a heteroatom, or an aromatic group having 6 to 60 carbon atoms. The above alicyclic hydrocarbon group may be a bridged alicyclic hydrocarbon group.

R^{Y} and R^{Z} preferably form, including a carbon atom to which they are bonded, a 4-membered to 21-membered ring, and more preferably form, including a carbon atom to which they are bonded, a 6-membered to 13-membered ring. The ring may be an alicyclic hydrocarbon ring, an aromatic ring, or a fused ring thereof. The alicyclic hydrocarbon ring may be a bridged alicyclic hydrocarbon ring. The ring may contain a sulfur atom and/or a nitrogen atom as a ring member atom. The ring may be substituted by a substituent containing a sulfur atom (for example, an alkylthio group), a hydroxy group, a phenyl group, or an alkyl group having 1 to 6 carbon atoms.

Examples of R^{Z} or R^{Y}-C-R^{Z} can include those having the following skeletons. From the viewpoint of improving the refractive index, it is preferable that a sulfur atom be contained in the skeleton.

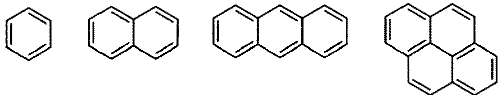

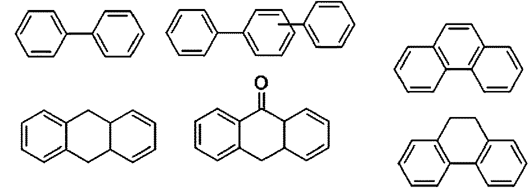

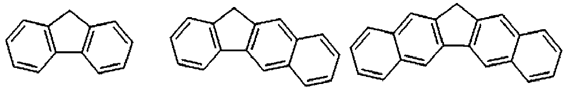

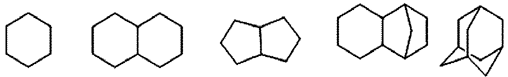

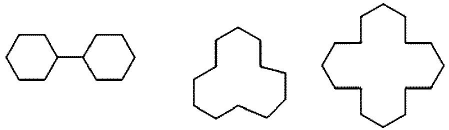

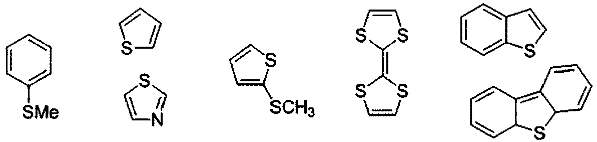

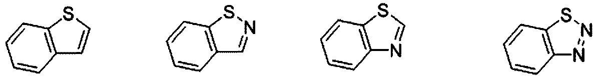

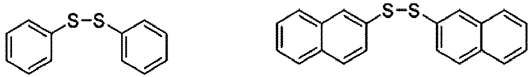

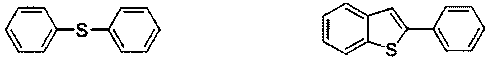

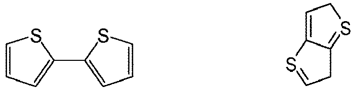

A is preferably an aryl group having 6 to 14 carbon atoms, and more preferably a phenyl group or a naphthyl group.

Preferably, each R^{T} is independently a thiol group, a hydroxy group or a phenyl group. The phenyl group may be substituted with a thiol group and/or a hydroxy group. The phenyl group may be bonded to A via a sulfide bond or a disulfide bond.

X is preferably an oxygen atom or not a crosslink.

Preferably, each m is independently an integer of 1 to 4, more preferably, each independently an integer of 1 to 3, and further preferably, each independently 1 or 2.

N is preferably an integer of 1 to 3, and more preferably 1 or 2.

The compound represented by the above formula (1) has high heat resistance attributed to its rigid structure, in spite of its relatively low molecular weight, and can therefore be used even under high temperature baking conditions. The compound is suitably used as a film forming composition for lithography that can be used in film production for lithography.

Furthermore, the compound represented by the above formula (1) has high solubility in a safe solvent and has good heat resistance and etching resistance. Therefore, the resist forming composition for lithography comprising the compound represented by the above formula (1) can impart a good shape to a resist pattern. In addition, the compound contains a sulfur atom, and thus, a sensitization function can be expected in EUV lithography application.

Moreover, the compound represented by the formula (1) has a relatively low molecular weight and a low viscosity and therefore facilitates enhancing film smoothness while uniformly and completely filling even the steps of an uneven substrate (particularly having fine space, hole pattern, etc.). As a result, an underlayer film forming composition for lithography using this compound has good embedding and smoothing properties. Moreover, the compound has a relatively high carbon concentration and contains a sulfur atom. Therefore, the compound can also impart high etching resistance.

In addition, the compound represented by the formula (1) has high refractive index ascribable to its high aromatic density and containment of a sulfur atom, and is prevented from being stained by heat treatment in a wide range from a low temperature to a high temperature. Therefore, the compound represented by the formula (1) is also useful as various optical component forming compositions. The optical component is used in the form of a film or a sheet and is also useful as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, a photosensitive optical waveguide, a liquid crystal display, an organic electroluminescent (EL) display, an optical semiconductor (LED) element, a solid state image sensing element, an organic thin film solar cell, a dye sensitized solar cell, and an organic thin film transistor (TFT). It can be particularly suitably utilized as an embedded film and a smoothed film on a photodiode, a smoothed film in front of or behind a color filter, a microlens, and a smoothed film and a conformal film on a microlens, all of which are components of a solid state image sensing element, to which high refractive index is particularly demanded.

The compound represented by the above formula (1) is preferably a compound represented by the following formula (1-1) from the viewpoint of solubility in an organic solvent.

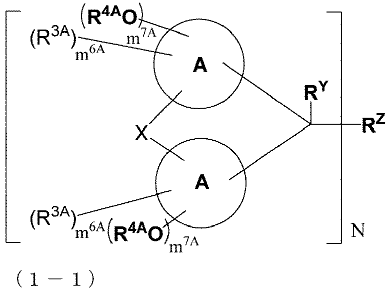

In the above (1-1),
R^{Y}, R^{Z}, A, X, and N are as defined in the formula (1) ;
each R^{3A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, a halogen atom, a nitro group, an amino group, a carboxylic acid group, or a thiol group; and
each R^{4A} is independently a hydrogen atom, an acid crosslinking group, or an acid dissociation group;
wherein, at least one selected from A, R^{Y}, R^{Z}, R^{3A}, R^{4A}, and X contains a sulfur atom;
each m^{6A} is independently an integer of 0 to 5; and
each m^{7A} is independently an integer of 0 to 5.

Specific examples of the compound represented by the above formula (1) will be further listed below. However, the compound represented by the formula (1) is not limited to the specific examples listed below.

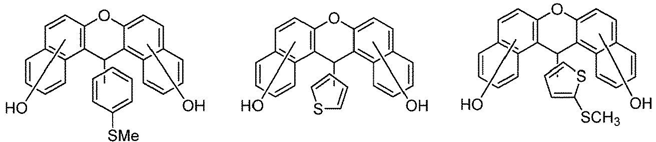

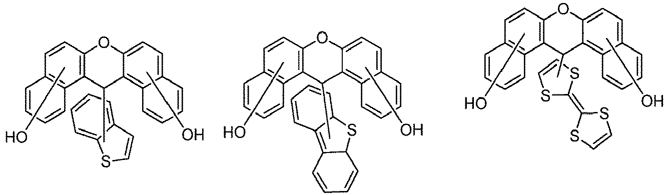

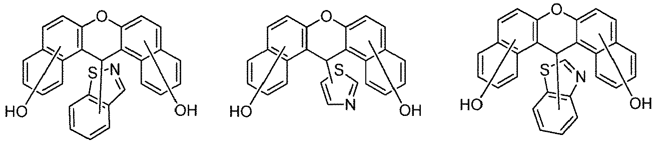

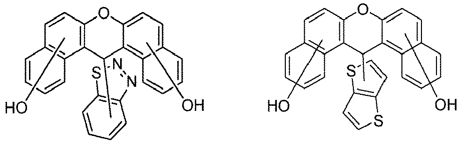

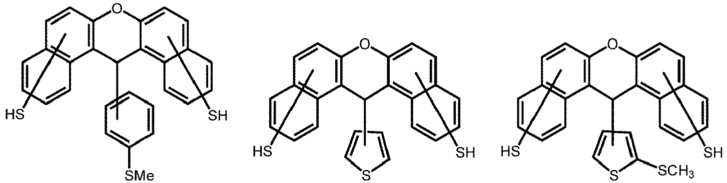

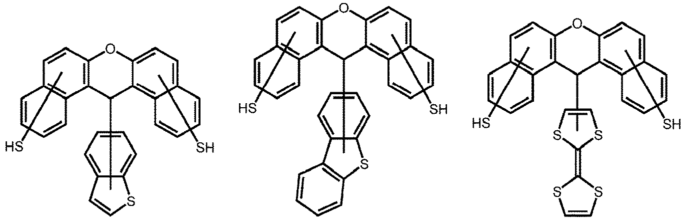

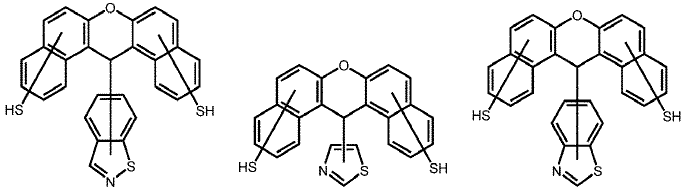

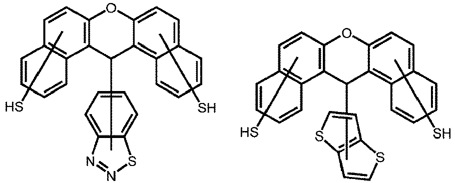

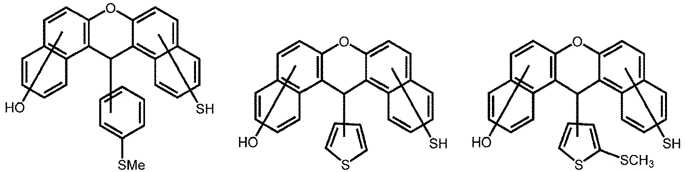

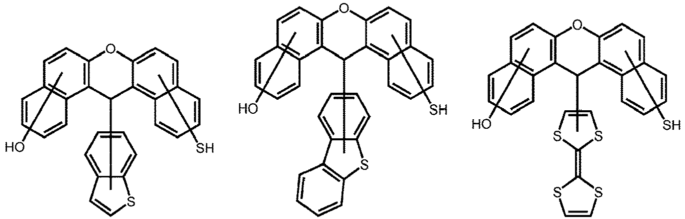

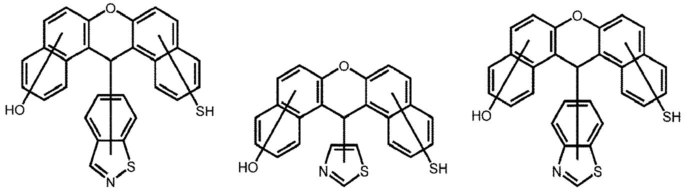

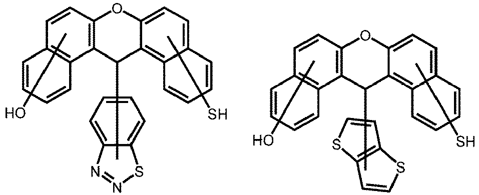

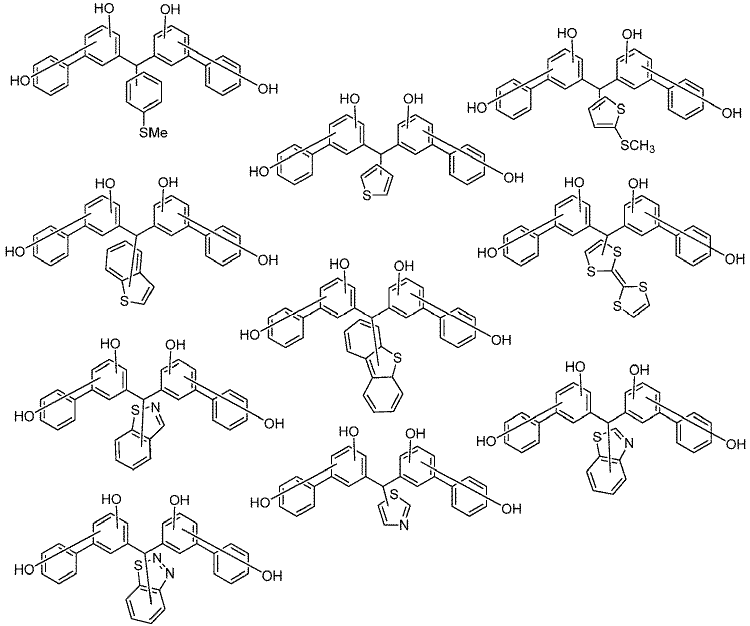

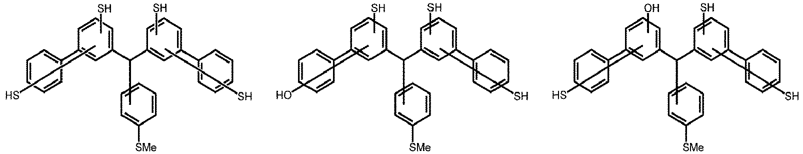

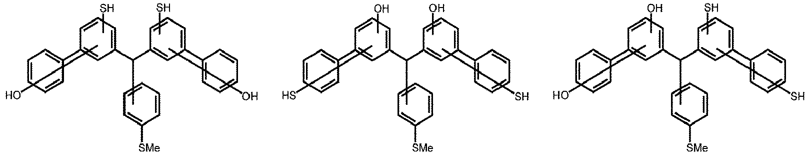

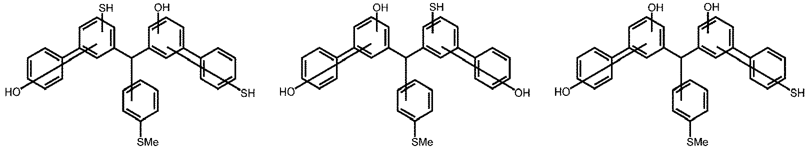

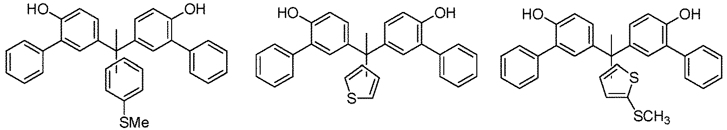

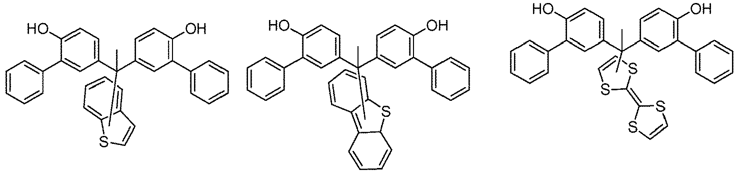

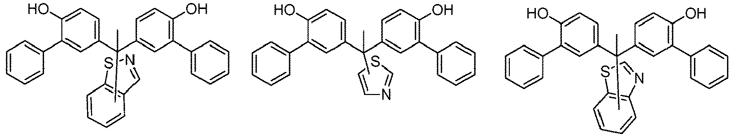

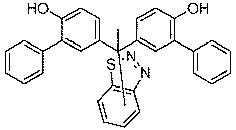

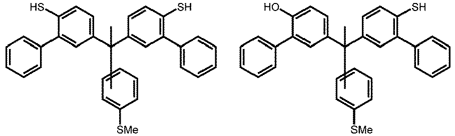

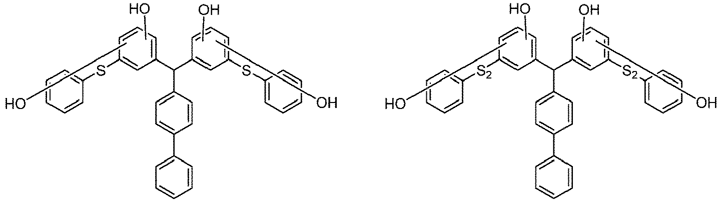

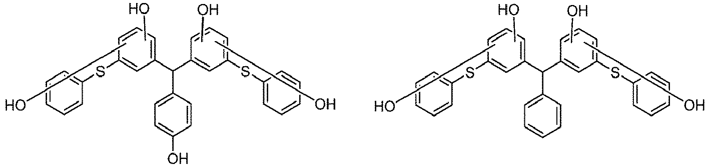

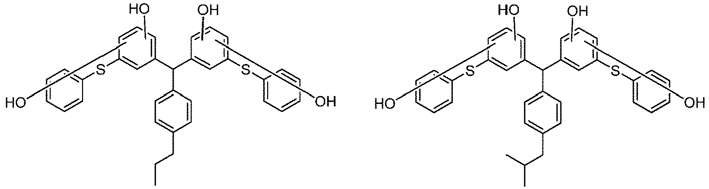

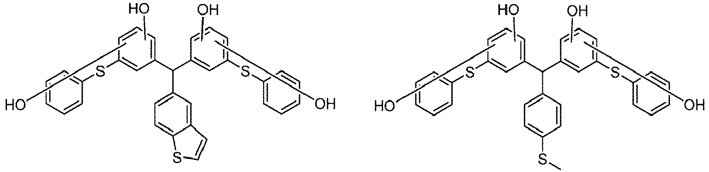

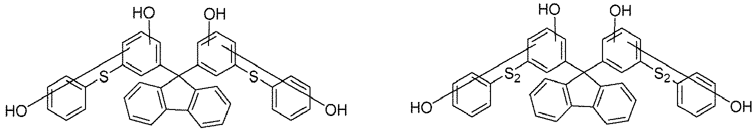

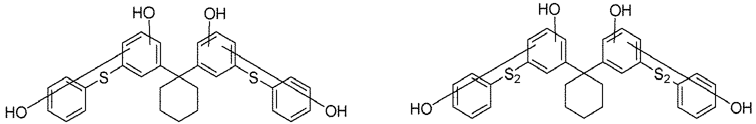

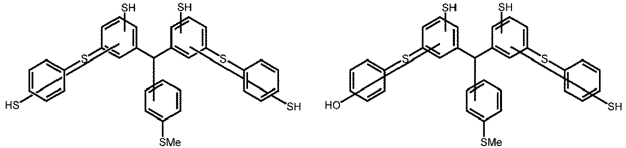

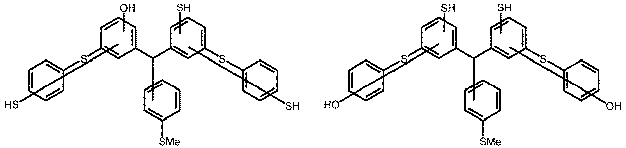

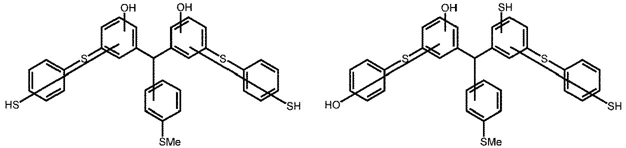

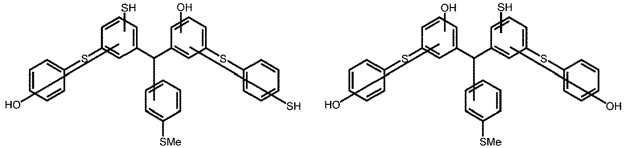

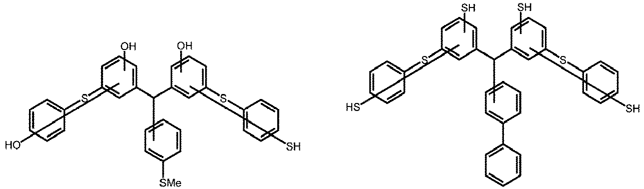

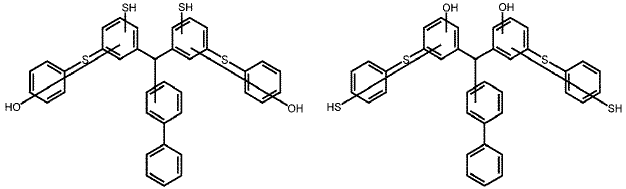

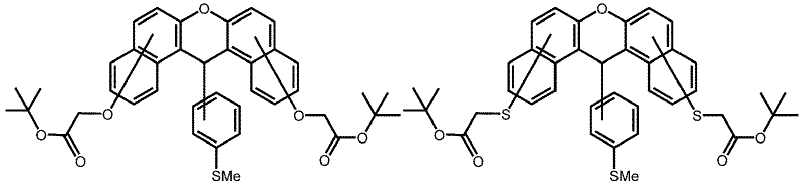

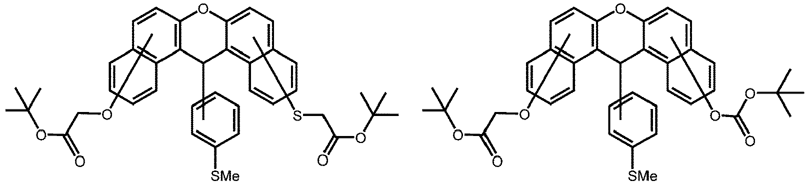

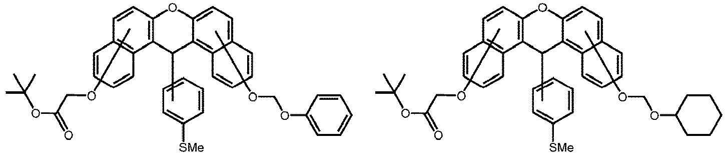

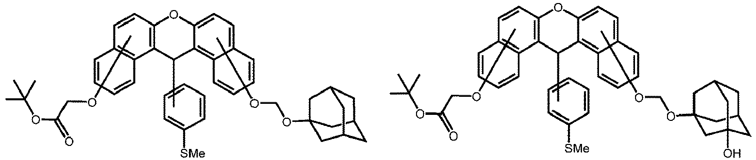

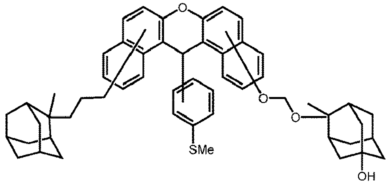

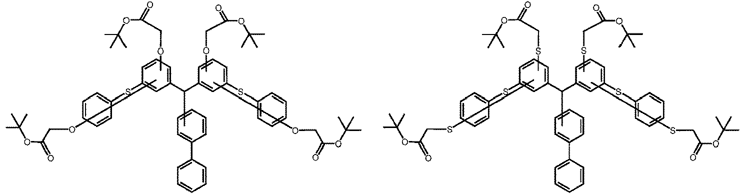

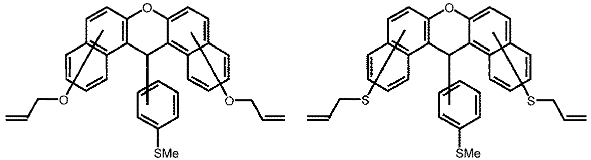

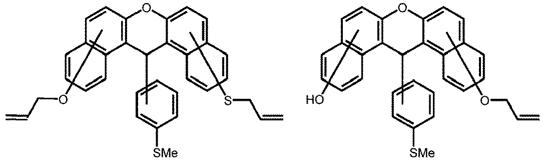

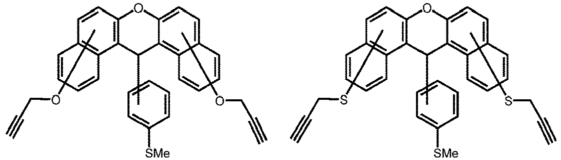

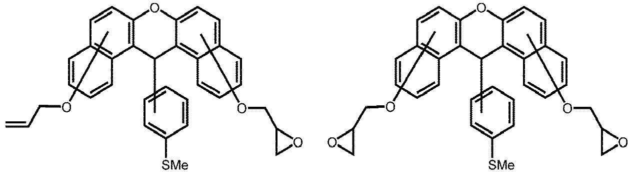

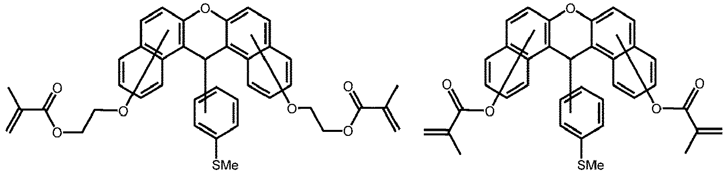

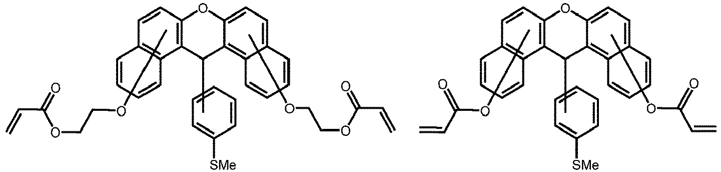

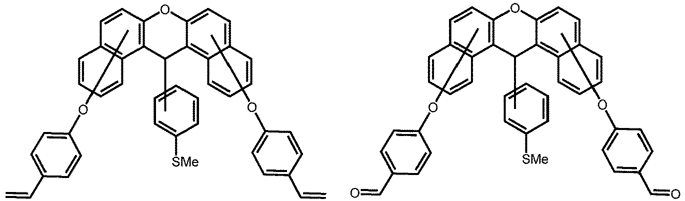

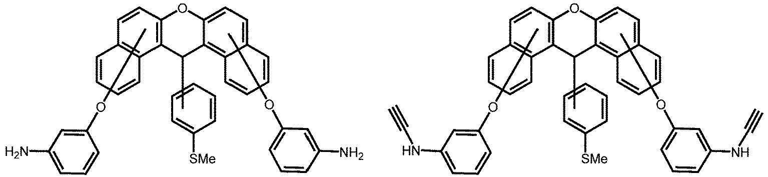

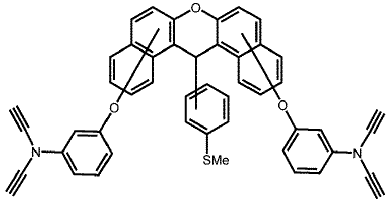

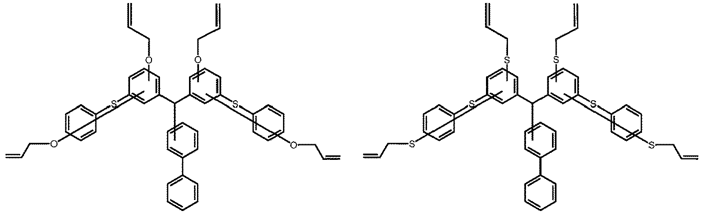

The above formula (1-1) is preferably the following formula (1-2), (1-3), (1-3-1), (1-3-2), (1-4), or (1-4-1), from the viewpoint of heat resistance and solubility. 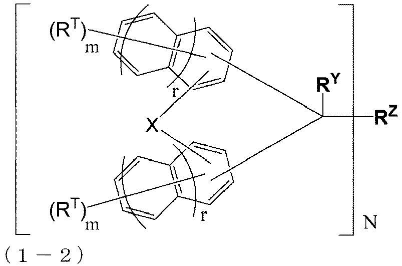 (In the formula (1-2),
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms or a single bond;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a thiol group, a hydroxy group, or a group in which a hydrogen atom of a hydroxy group is replaced with an acid dissociation group, wherein the alkyl group, the alkenyl group, the alkynyl group and the aryl group each optionally contain an ether bond, a ketone bond or an ester bond; and
X is an oxygen atom, a sulfur atom or not a crosslink;
   wherein, at least one selected from R^{T}, R^{Y}, R^{Z}, and X contains a sulfur atom;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 0 to 2.)

In addition, although the compound represented by the formula (1-2) is not particularly limited, it is preferable that one or more of the following (a) to (e) be satisfied from the viewpoint of suppressing colorability and suppressing the degradability of the compound.
(a): In the formula (1-2), r in the structural formula within the parentheses [ ] is the same, that is, two sites represented by the aryl structure in the structural formula within the parentheses [ ] have the same structure.
(b): In the formula (1-2), R^{T} bonded to the site represented by each aryl structure in the structural formula [ ] is the same, and furthermore, the binding site in the site represented by each aryl structure is the same.
(c): In the formula (1-2), N is 1 to 2, and furthermore, N is 1.
(d): In the formula (1-2), R^{Y} is a linear alkyl group having 1 to 30 carbon atoms or a phenyl group, and furthermore, a methyl group or a phenyl group.
(e): In the formula (1-2), R^{Z} is an N-valent group having 1 to 60 carbon atoms.

When the compound has the following structure, the heat resistance is further increased and the solvent solubility is also increased.

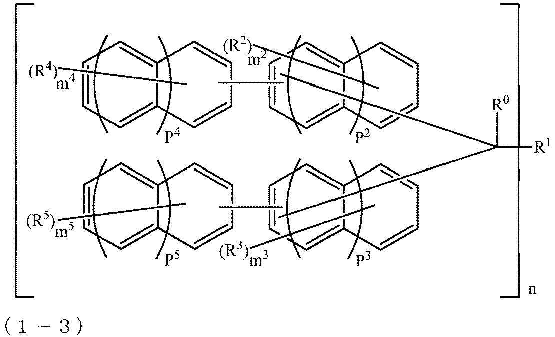

In the above formula (1-3), R⁰ is as defined in the above R^{Y}, and is a hydrogen atom, an alkyl group having 1 to 30 carbon atoms and optionally having a substituent, or an aryl group having 6 to 30 carbon atoms and optionally having a substituent. When R⁰ is an alkyl group having 1 to 30 carbon atoms and optionally having a substituent or an aryl group having 6 to 30 carbon atoms and optionally having a substituent, the compound is prevented from being oxidatively decomposed and stained, has high heat resistance, and improves solvent solubility.

R¹ is an n-valent group having 1 to 60 carbon atoms or a single bond, and each aromatic ring is bonded via this R¹.

R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group. Herein, at least one selected from R⁰, R¹, R², R³, R⁴, and R⁵ contains a sulfur atom; and
m² and m³ are each independently an integer of 0 to 8, and m⁴ and m⁵ are each independently an integer of 0 to 9. However, m², m³, m⁴, and m⁵ are not 0 at the same time.

n is an integer of 1 to 4.

p² to p⁵ are each independently an integer of 0 to 2. When p² to p⁵ are 0, the site represented by the naphthalene structure in the formula (3) represents a benzene structure; when p² to p⁵ are 1, the site represents a naphthalene structure; and when p² to p⁵ are 2, the site represents a tricyclic structure such as anthracene or phenanthrene.

n is as defined in the above N. When n is an integer of 2 or larger, n structural formulas within the parentheses [ ] are the same or different.

The above n-valent group refers to an alkyl group having 1 to 60 carbon atoms when n is 1, an alkylene group having 1 to 60 carbon atoms when n is 2, an alkanetriyl group having 2 to 60 carbon atoms when n is 3, and an alkanetetrayl group having 3 to 60 carbon atoms when n is 4. Examples of the above n-valent group include groups having a linear hydrocarbon group, a branched hydrocarbon group, and an alicyclic hydrocarbon group. Herein, the above alicyclic hydrocarbon group also includes a bridged alicyclic hydrocarbon group. Also, the above n-valent group may have an aromatic group having 6 to 60 carbon atoms.

In addition, the above n-valent hydrocarbon group may have an alicyclic hydrocarbon group, a double bond, a heteroatom, or an aromatic group having 6 to 60 carbon atoms. Herein, the above alicyclic hydrocarbon group also includes a bridged alicyclic hydrocarbon group.

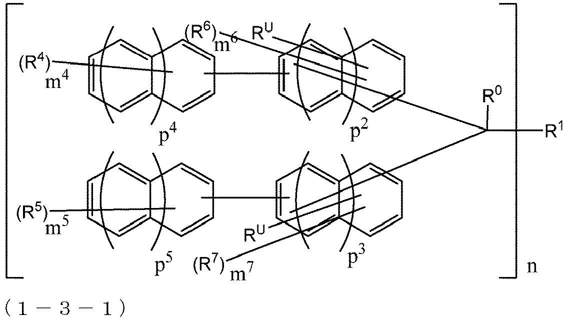

In the formula (1-3-1),
R⁰, R¹, R⁴, R⁵, n, p² to p⁵, m⁴, and m⁵ are as defined in the description of the formula (1-3);
R⁶ and R⁷ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group;
each R^{U} is independently a hydroxy group or a thiol group;
at least one selected from R⁰, R¹, R⁴, R⁵, R⁶, R⁷, and R^{U} contains a sulfur atom; and
m⁶ and m⁷ are each independently an integer of 0 to 7.

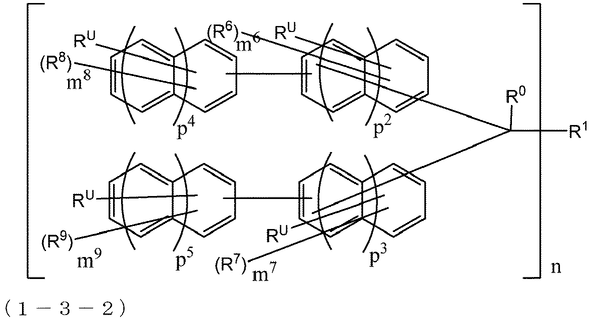

In the formula (1-3-2),
R⁰, R¹, n, and p² to p⁵ are as defined in the description of the formula (1-3), and R⁶, R⁷, m⁶, and m⁷ are as defined in the description of the formula (1-3-1);
R⁸ and R⁹ are as defined in the above R⁶ and R⁷;
each R^{U} is independently a hydroxy group or a thiol group;
at least one selected from R⁰, R¹, R⁶, R⁷, R⁸, R⁹, and R^{U} contains a sulfur atom; and
m⁸ and m⁹ are each independently an integer of 0 to 8.

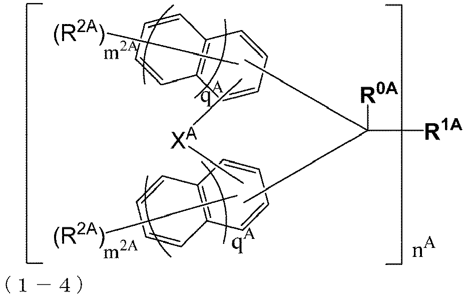

In the formula (1-4), R^{0A} is as defined in the above R^{Y}, and is a hydrogen atom, an alkyl group having 1 to 30 carbon atoms and optionally having a substituent, or an aryl group having 6 to 30 carbon atoms and optionally having a substituent.

R^{1A} is an n^{A}-valent group having 1 to 60 carbon atoms or a single bond;
each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group;
in the formula (1-4), at least one selected from R^{0A}, R^{1A} and R^{2A} contains a sulfur atom; and
n^{A} is an integer of 1 to 4, where when n^{A} is an integer of 2 or larger in the formula (1-4), n^{A} structural formulas within the parentheses [ ] are the same or different.

Each X^{A} is independently an oxygen atom, a sulfur atom or not a crosslink. Herein, X^{A} is preferably an oxygen atom or a sulfur atom and more preferably an oxygen atom, because there is a tendency to exhibit high heat resistance. Preferably, X^{A} is not a crosslink from the viewpoint of solubility.

Each m^{2A} is independently an integer of 0 to 6. However, at least one m^{2A} is an integer of 1 to 6.

Each q^{A} is independently 0 or 1. When q^{A} is 0, the site represented by the naphthalene structure in the formula (1-4) represents a benzene structure, and when q^{A} is 1, the site represents a naphthalene structure.

The above n^{A}-valent group refers to an alkyl group having 1 to 60 carbon atoms when n^{A} is 1, an alkylene group having 1 to 30 carbon atoms when n^{A} is 2, an alkanetriyl group having 2 to 60 carbon atoms when n^{A} is 3, and an alkanetetrayl group having 3 to 60 carbon atoms when n^{A} is 4. Examples of the above n^{A}-valent group include groups having a linear hydrocarbon group, a branched hydrocarbon group, and an alicyclic hydrocarbon group. Herein, the above alicyclic hydrocarbon group also includes a bridged alicyclic hydrocarbon group. Also, the above n^{A}-valent group may have an aromatic group having 6 to 60 carbon atoms.

In addition, the above n^{A}-valent hydrocarbon group may have an alicyclic hydrocarbon group, a double bond, a heteroatom, or an aromatic group having 6 to 60 or 6 to 30 carbon atoms. Herein, the above alicyclic hydrocarbon group also includes a bridged alicyclic hydrocarbon group.

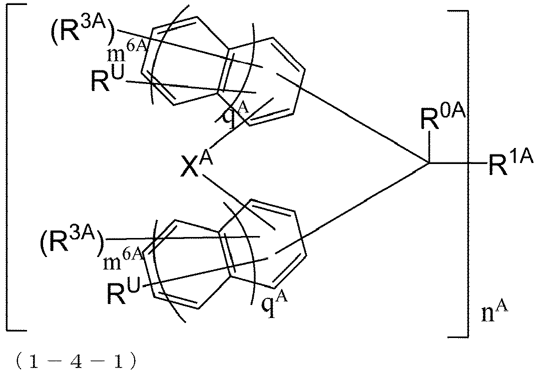

In the formula (1-4-1), R^{0A}, R^{1A}, n^{A}, q^{A}, and X^{A} are as defined in the description of the above formula (1-4).

Each R^{3A} is independently a halogen atom, an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, or an alkoxy group having 1 to 30 carbon atoms and optionally having a substituent;
each R^{U} is independently a hydroxy group or a thiol group;
at least one selected from R^{0A}, R^{1A}, R^{3A}, and R^{U} contains a sulfur atom; and
each m^{6A} is independently an integer of 0 to 5.

### [Compound represented by formula (1')]

The compound of the present invention is also represented by the following formula (1'). Since the compound of the present invention has such a structure, it has high heat resistance, relatively high carbon concentration, relatively low oxygen concentration, and high solvent solubility. In addition, the compound of the present invention contains a sulfur atom, and thus has a high refractive index. Furthermore, the compound of the present invention has a low viscosity before being cured, and is excellent in smoothing properties. 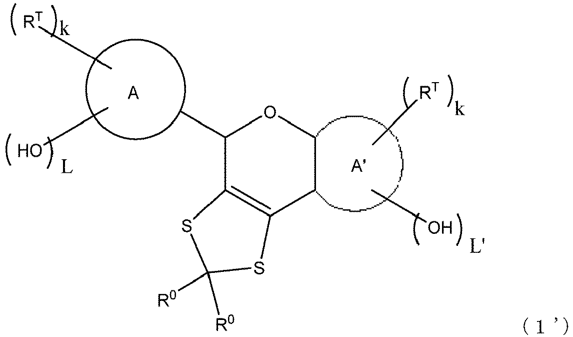 (In the formula (1'),
each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond;
each R⁰ is independently a hydrogen atom, an alkyl group having 1 to 30 carbon atoms and optionally having a substituent and/or a heteroatom, or an aryl group having 6 to 30 carbon atoms and optionally having a substituent and/or a heteroatom;
or alternatively, two R⁰ may form, including a carbon atom to which they are bonded, a 4-membered to 30-membered ring optionally having a substituent and/or a heteroatom; or two R⁰ are a double bond bonded to a carbon atom to which they are bonded, wherein an alkyl group having 1 to 30 carbon atoms and optionally having a substituent and/or a heteroatom, or an aryl group having 6 to 30 carbon atoms and optionally having a substituent and/or a heteroatom may be bonded to the double bond;
A and A' are each a group exhibiting aromaticity and having 1 to 60 carbon atoms and optionally having a substituent and/or a heteroatom;
L is an integer of 1 to 9; and
k and L' are each independently an integer of 0 to 9.)

The terms in the above formula (1') such as the "alkyl group", the "aryl group", the "alkenyl group", the "alkynyl group", the "alkoxy group", the "alkylthio group", the "halogen atom", the "heteroatom", the "acid dissociation", and the "acid crosslinking group" are as defined in the description of the formula (1).

The compound represented by the above formula (1') is preferably a compound represented by the following formula (1-1') from the viewpoint of solubility in an organic solvent. 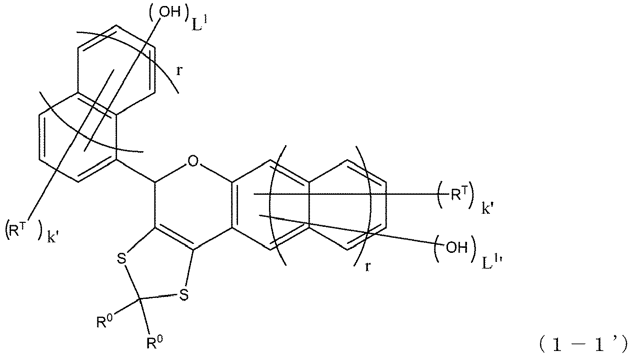 (In the formula (1-1'),
each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond;
each R⁰ is independently a hydrogen atom, an alkyl group having 1 to 30 carbon atoms and optionally having a substituent and/or a heteroatom, or an aryl group having 6 to 30 carbon atoms and optionally having a substituent and/or a heteroatom;
or alternatively, two R⁰ may form, including a carbon atom to which they are bonded, a 4-membered to 30-membered ring optionally having a substituent and/or a heteroatom; or two R⁰ are a double bond bonded to a carbon atom to which they are bonded, wherein an alkyl group having 1 to 30 carbon atoms and optionally having a substituent and/or a heteroatom, or an aryl group having 6 to 30 carbon atoms and optionally having a substituent and/or a heteroatom may be bonded to the double bond;
each r is independently an integer of 0 to 2;
L¹ is an integer of 1 to (4 + 2r); and
k' and L¹' are each independently an integer of 0 to (3 + 2r).)

The compound represented by the above formula (1-1') is not particularly limited, and examples thereof include the following formula (1-2').

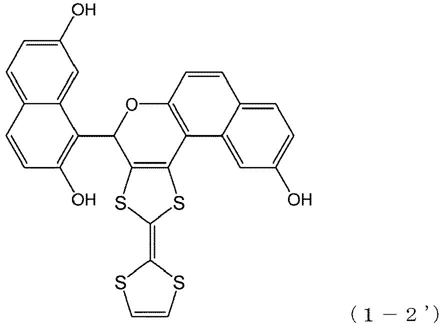

The above formula (1-2') has high etching resistance, high refractive index, and high transmittance.

### [Compound represented by formula (2)]

The compound of the present embodiment is preferably represented by the following formula (2). 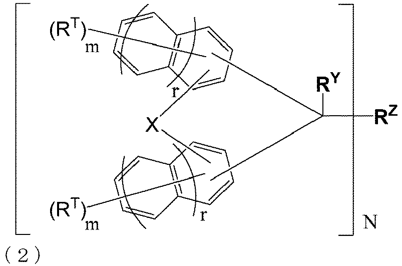 (In the formula (2),
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms or a single bond;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a thiol group, a hydroxy group, or a group in which a hydrogen atom of a hydroxy group is replaced with an acid dissociation group, wherein the alkyl group, the alkenyl group, the alkynyl group and the aryl group each optionally contain an ether bond, a ketone bond or an ester bond and wherein at least one R^{T} is a thiol group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 0 to 2.)

In addition, although the compound represented by the formula (2) is not particularly limited, it is preferable that one or more of the following (a) to (e) be satisfied from the viewpoint of suppressing colorability and suppressing the degradability of the compound.
(a): In the formula (2), r in the structural formula within the parentheses [ ] is the same, that is, two sites represented by the aryl structure in the structural formula within the parentheses [ ] have the same structure.
(b): In the formula (2), R^{T} bonded to the site represented by each aryl structure in the structural formula [ ] is the same, and furthermore, the binding site in the site represented by each aryl structure is the same.
(c): In the formula (2), N is 1 to 2, and furthermore, N is 1.
(d): In the formula (2), R^{Y} is a linear alkyl group having 1 to 30 carbon atoms or a phenyl group, and furthermore, a methyl group or a phenyl group.
(e): In the formula (2), R^{Z} is an N-valent group having 1 to 60 carbon atoms.

The compound represented by the above formula (2) is preferably a compound represented by the following formula (2-1) from the viewpoint of easy crosslinking and solubility in an organic solvent. 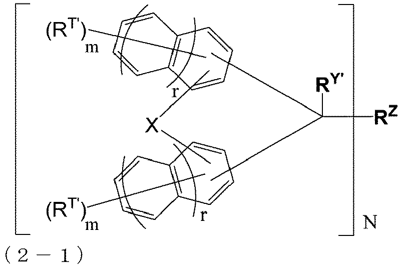 (In the formula (2-1),
R^{Y'} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms or a single bond;
each R^{T'} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one R^{T'} is a thiol group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 0 to 2.)

Hereinafter, the compound represented by the formula (2) and the compound represented by the formula (2-1) will be described with a focus on the compound represented by the formula (3) and the compound represented by the formula (4). However, the compound represented by the formula (2) and the compound represented by the formula (2-1) are not limited to the compounds described below.

### [Compound represented by formula (3)]

The compound of the present embodiment is preferably represented by the following formula (3). When the compound of the present embodiment has the following structure, the heat resistance is further increased and the solvent solubility is also increased.

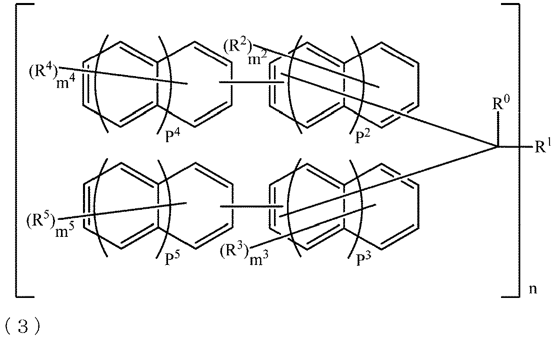

In the above formula (3), R⁰ is as defined in the above R^{Y}, and is a hydrogen atom, an alkyl group having 1 to 30 carbon atoms and optionally having a substituent, or an aryl group having 6 to 30 carbon atoms and optionally having a substituent. When R⁰ is an alkyl group having 1 to 30 carbon atoms and optionally having a substituent or an aryl group having 6 to 30 carbon atoms and optionally having a substituent, the compound is prevented from being oxidatively decomposed and stained, has high heat resistance, and improves solvent solubility.

R¹ is an n-valent group having 1 to 60 carbon atoms or a single bond, and each aromatic ring is bonded via this R¹.

R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group. However, in the formula (3), at least one of R² to R⁵ is a thiol group.

m² and m³ are each independently an integer of 0 to 8, and m⁴ and m⁵ are each independently an integer of 0 to 9. However, m², m³, m⁴, and m⁵ are not 0 at the same time.

n is an integer of 1 to 4.

p² to p⁵ are each independently an integer of 0 to 2. When p² to p⁵ are 0, the site represented by the naphthalene structure in the formula (3) represents a benzene structure; when p² to p⁵ are 1, the site represents a naphthalene structure; and when p² to p⁵ are 2, the site represents a tricyclic structure such as anthracene or phenanthrene.

n is as defined in the above N. When n is an integer of 2 or larger, n structural formulas within the parentheses [ ] are the same or different.

The above n-valent group refers to an alkyl group having 1 to 60 carbon atoms when n is 1, an alkylene group having 1 to 60 carbon atoms when n is 2, an alkanetriyl group having 2 to 60 carbon atoms when n is 3, and an alkanetetrayl group having 3 to 60 carbon atoms when n is 4. Examples of the above n-valent group include groups having a linear hydrocarbon group, a branched hydrocarbon group, and an alicyclic hydrocarbon group. Herein, the above alicyclic hydrocarbon group also includes a bridged alicyclic hydrocarbon group. Also, the above n-valent group may have an aromatic group having 6 to 60 carbon atoms.

In addition, the above n-valent hydrocarbon group may have an alicyclic hydrocarbon group, a double bond, a heteroatom, or an aromatic group having 6 to 60 carbon atoms. Herein, the above alicyclic hydrocarbon group also includes a bridged alicyclic hydrocarbon group.

The compound represented by the above formula (3) has high heat resistance attributed to its rigid structure, in spite of its relatively low molecular weight, and can therefore be used even under high temperature baking conditions. Also, the compound represented by the above formula (3) has tertiary carbon or quaternary carbon in the molecule, which inhibits crystallinity, and is thus suitably used as a film forming composition for lithography that can be used in film production for lithography. It is preferable that the compound have quaternary carbon from the viewpoint of inhibiting crystallinity.

Furthermore, the compound represented by the above formula (3) has high solubility in a safe solvent and has good heat resistance and etching resistance. Therefore, the resist forming composition for lithography of the present embodiment imparts a good shape to a resist pattern.

Moreover, the compound represented by the formula (3) has a relatively low molecular weight and a low viscosity and therefore facilitates enhancing film smoothness while uniformly and completely filling even the steps of an uneven substrate (particularly having fine space, hole pattern, etc.). As a result, an underlayer film forming composition for lithography using this compound is capable of relatively advantageously enhancing embedding and smoothing properties. Moreover, the compound has a relatively high carbon concentration and therefore also imparts high etching resistance.

The compound represented by the formula (3) has high refractive index and is prevented from being stained by heat treatment in a wide range from a low temperature to a high temperature. Therefore, the composition is also useful as various optical component forming compositions. The optical component is used in the form of a film or a sheet and is also useful as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, a photosensitive optical waveguide, a liquid crystal display, an organic electroluminescent (EL) display, an optical semiconductor (LED) element, a solid state image sensing element, an organic thin film solar cell, a dye sensitized solar cell, and an organic thin film transistor (TFT). It can be particularly suitably utilized as an embedded film and a smoothed film on a photodiode, a smoothed film in front of or behind a color filter, a microlens, and a smoothed film and a conformal film on a microlens, all of which are components of a solid state image sensing element, to which high refractive index is demanded.

The compound represented by the above formula (3) is preferably a compound represented by the following formula (3-1) from the viewpoint of easy crosslinking and solubility in an organic solvent.

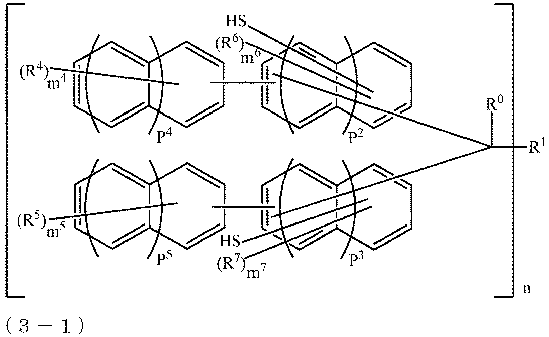

In the formula (3-1),
R⁰, R¹, R⁴, R⁵, n, p² to p⁵, m⁴, and m⁵ are as defined in the description of the formula (3);
R⁶ and R⁷ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; and
m⁶ and m⁷ are each independently an integer of 0 to 7.

In addition, the compound represented by the above formula (3-1) is preferably a compound represented by the following formula (3-2) from the viewpoint of further easy crosslinking and solubility in an organic solvent.

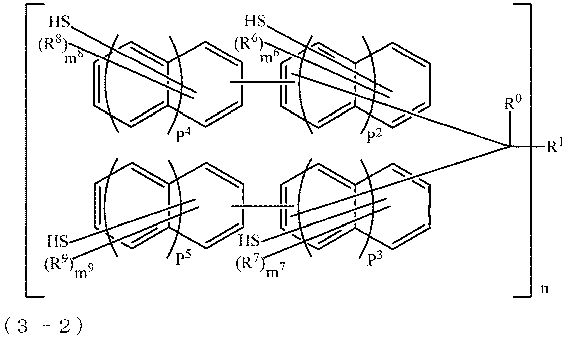

In the formula (3-2),
R⁰, R¹, n, and p² to p⁵ are as defined in the description of the formula (3), and R⁶, R⁷, m⁶, and m⁷ are as defined in the description of the formula (3-1);
R⁸ and R⁹ are as defined in the above R⁶ and R⁷; and
m⁸ and m⁹ are each independently an integer of 0 to 8.

In addition, the compound is preferably a compound represented by the following formula (3a) from the viewpoint of the supply of raw materials.

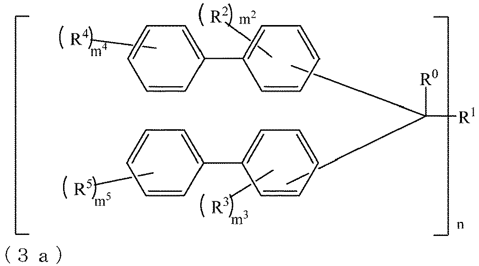

In the above formula (3a), R⁰ to R⁵, m² to m⁵, and n are as defined in the description of the above formula (3) .

The compound represented by the above formula (3a) is more preferably a compound represented by the following formula (3b) from the viewpoint of solubility in an organic solvent.

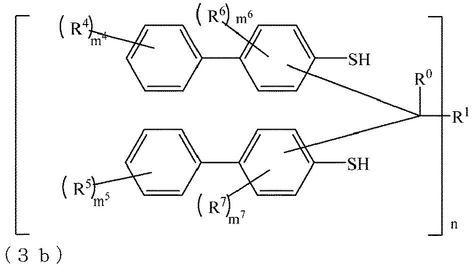

In the above formula (3b), R⁰, R¹, R⁴, R⁵, m⁴, m⁵, and n are as defined in the description of the above formula (3), and R⁶, R⁷, m⁶, and m⁷ are as defined in the description of the above formula (3-1).

The compound represented by the above formula (3a) is further preferably a compound represented by the following formula (3b') from the viewpoint of reactivity.

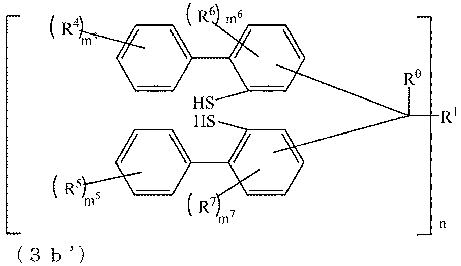

In the above formula (3b'), R⁰, R¹, R⁴, R⁵, m⁴, m⁵, and n are as defined in the description of the above formula (3), and R⁶, R⁷, m⁶, and m⁷ are as defined in the description of the above formula (3-1).

The compound represented by the above formula (3b) is further preferably a compound represented by the following formula (3c) from the viewpoint of solubility in an organic solvent.

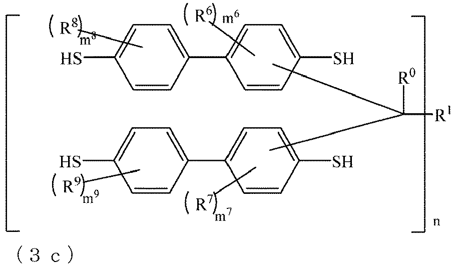

In the above formula (3c), R⁰, R¹, and n are as defined in the description of the formula (3); R⁶, R⁷, m⁶, and m⁷ are as defined in the description of the formula (3-1); and R⁸, R⁹, m⁸, and m⁹ are as defined in the description of the formula (3-2).

The compound represented by the above formula (3b') is further preferably a compound represented by the following formula (3c') from the viewpoint of reactivity.

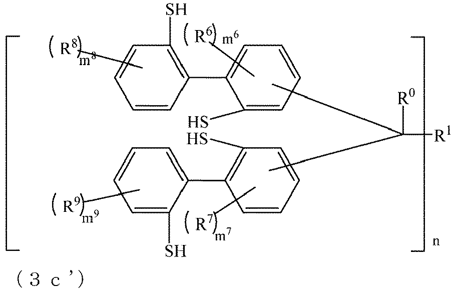

In the above formula (3c'), R⁰, R¹, and n are as defined in the description of the formula (3); R⁶, R⁷, m⁶, and m⁷ are as defined in the description of the formula (3-1); and R⁸, R⁹, m⁸, and m⁹ are as defined in the description of the formula (3-2).

The compound represented by the above formula (3) is particularly preferably a compound represented by the following formula (3d-1) or (3d-2) from the viewpoint of further solubility in an organic solvent.

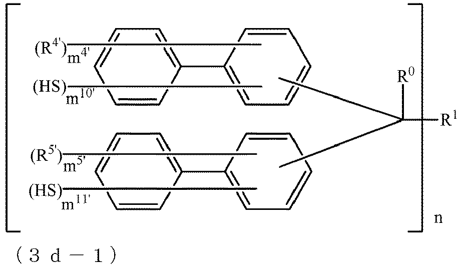

In the above formula (3d-1), R⁰, R¹, and n are as defined in the description of the formula (3); and R^{4'} and R^{5'} are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, or a halogen atom. m^{4'} and m^{5'} are each an integer of 0 to 8; m^{10'} and m^{11'} are each an integer of 1 to 9; and m^{4'} + m^{10'} and m^{5'} + m^{11'} are each independently an integer of 1 to 9.

Examples of R⁰ include a hydrogen atom, a methyl group, an ethyl group, a propyl group, a butyl group, a pentyl group, a hexyl group, a heptyl group, an octyl group, a nonyl group, a decyl group, an undecyl group, a dodecyl group, a triacontyl group, a phenyl group, a naphthyl group, an anthracenyl group, a pyrenyl group, a biphenyl group, and a heptacenyl group.

Examples of R^{4'} and R^{5'} include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracenyl group, a pyrenyl group, a biphenyl group, a heptacenyl group, a vinyl group, an allyl group, a triacontenyl group, a methoxy group, an ethoxy group, a triacontyloxy group, a fluorine atom, a chlorine atom, a bromine atom, an iodine atom, and a thiol group.

R⁰, R^{4'}, and R^{5'} listed above include isomers. For example, a butyl group includes a n-butyl group, an isobutyl group, a sec-butyl group, and a tert-butyl group.

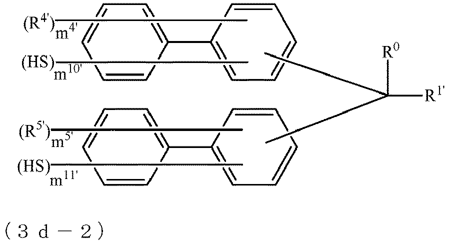

In the above formula (3d-2), R⁰, R^{4'}, R^{5'}, m^{4'}, m^{5'}, m^{10'}, and m^{11'} are as defined in the description of the above formula (3d-1), and R^{1'} is a monovalent group having 1 to 60 carbon atoms.

As the compound represented by the formula (3), the compounds represented by the following formulas can be used.

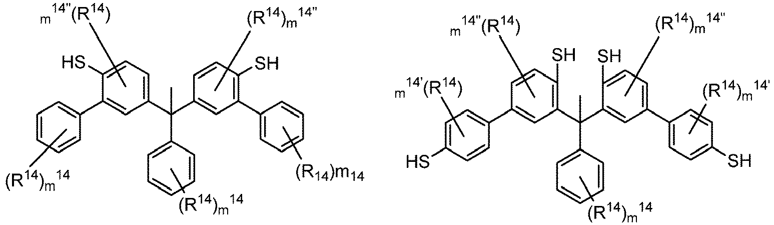

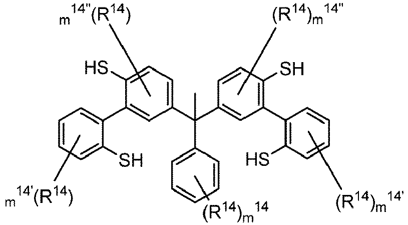

In the above compounds, each R¹⁴ is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; m¹⁴ is an integer of 0 to 5; m^{14'} is an integer of 0 to 4; and m^{14''} is an integer of 0 to 3.

Examples of R¹⁴ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracenyl group, a pyrenyl group, a biphenyl group, a heptacenyl group, a vinyl group, an allyl group, a triacontenyl group, a methoxy group, an ethoxy group, a triacontyloxy group, a fluorine atom, a chlorine atom, a bromine atom, an iodine atom, and a thiol group.

R¹⁴ listed above includes isomers. For example, a butyl group includes a n-butyl group, an isobutyl group, a sec-butyl group, and a tert-butyl group.

As the compound represented by the formula (3), the compounds represented by the following formulas can be further used.

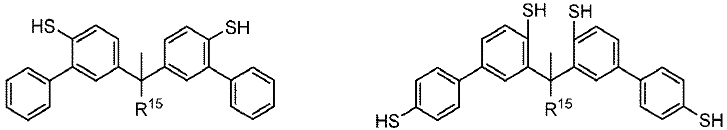

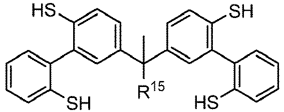

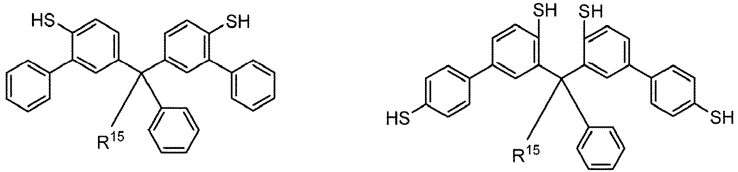

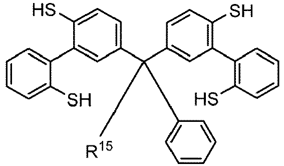

In the above chemical formulas, R¹⁵ is an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group.

Examples of R¹⁵ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracenyl group, a pyrenyl group, a biphenyl group, a heptacenyl group, a vinyl group, an allyl group, a triacontenyl group, a methoxy group, an ethoxy group, a triacontyloxy group, a fluorine atom, a chlorine atom, a bromine atom, an iodine atom, and a thiol group.

R¹⁵ listed above includes isomers. For example, a butyl group includes a n-butyl group, an isobutyl group, a sec-butyl group, and a tert-butyl group.

As the compound represented by the formula (3), the compounds represented by the following formulas can be further used.

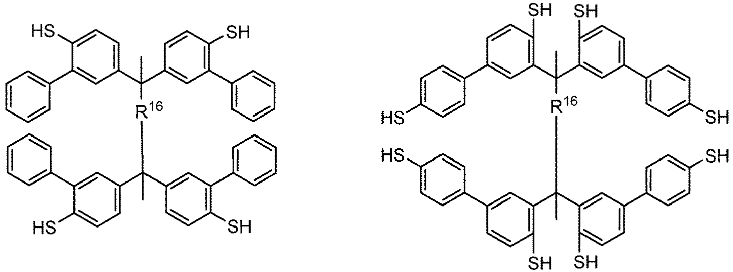

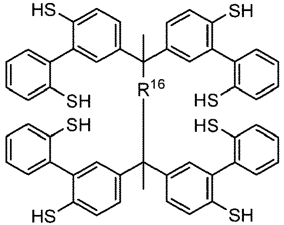

In the above compounds, R¹⁶ is a linear, branched or cyclic alkylene group having 1 to 30 carbon atoms and optionally having a substituent, a divalent aryl group having 6 to 30 carbon atoms and optionally having a substituent, a divalent alkenyl group having 2 to 30 carbon atoms and optionally having a substituent, or an alkynyl group having 2 to 30 carbon atoms and optionally having a substituent.

Examples of R¹⁶ include a methylene group, an ethylene group, a propene group, a butene group, a pentene group, a hexene group, a heptene group, an octene group, a nonene group, a decene group, an undecene group, a dodecene group, a triacontene group, a cyclopropene group, a cyclobutene group, a cyclopentene group, a cyclohexene group, a cycloheptene group, a cyclooctene group, a cyclononene group, a cyclodecene group, a cycloundecene group, a cyclododecene group, a cyclotriacontene group, a divalent norbornyl group, a divalent adamantyl group, a divalent phenyl group, a divalent naphthyl group, a divalent anthracenyl group, a divalent pyrene group, a divalent biphenyl group, a divalent heptacenyl group, a divalent vinyl group, a divalent allyl group, and a divalent triacontenyl group.

R¹⁶ listed above includes isomers. For example, a butyl group includes a n-butyl group, an isobutyl group, a sec-butyl group, and a tert-butyl group.

As the compound represented by the formula (3), the compounds represented by the following formulas can be further used.

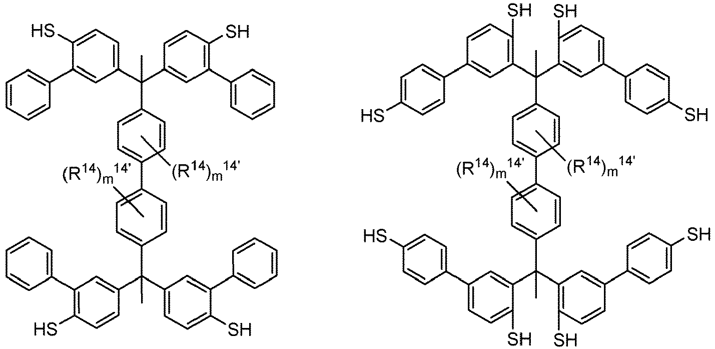

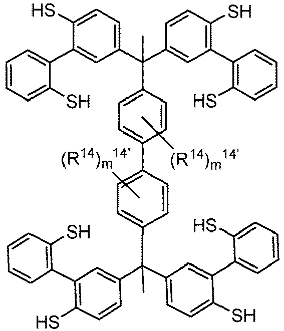

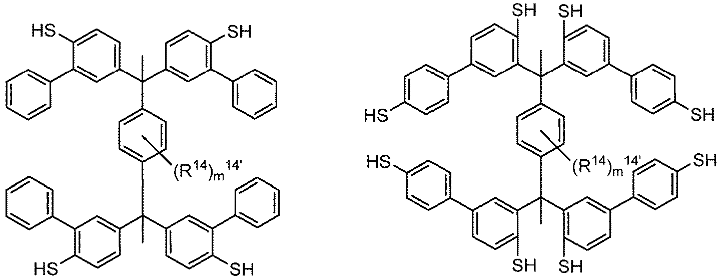

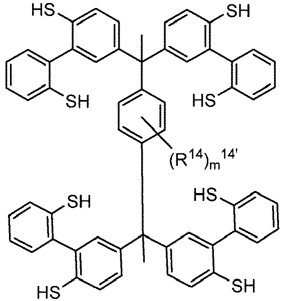

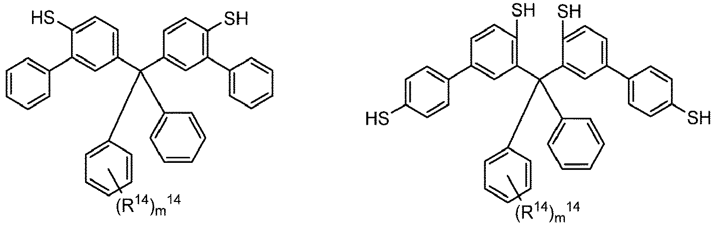

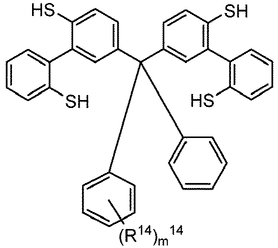

In the above formula compounds, each R¹⁴ is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; m¹⁴ is an integer of 0 to 5; and m^{14'} is an integer of 0 to 4.

Examples of R¹⁴ include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracenyl group, a pyrenyl group, a biphenyl group, a heptacenyl group, a vinyl group, an allyl group, a triacontenyl group, a methoxy group, an ethoxy group, a triacontyloxy group, a fluorine atom, a chlorine atom, a bromine atom, an iodine atom, and a thiol group.

R¹⁴ listed above includes isomers. For example, a butyl group includes a n-butyl group, an isobutyl group, a sec-butyl group, and a tert-butyl group.

As the compound represented by the formula (3), the compounds represented by the following formulas can be further used.

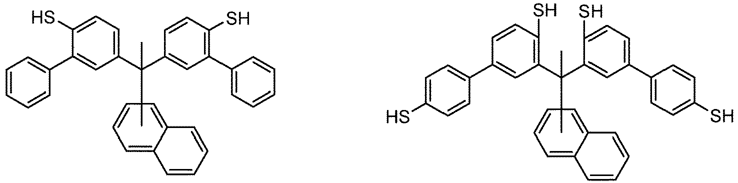

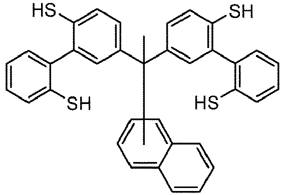

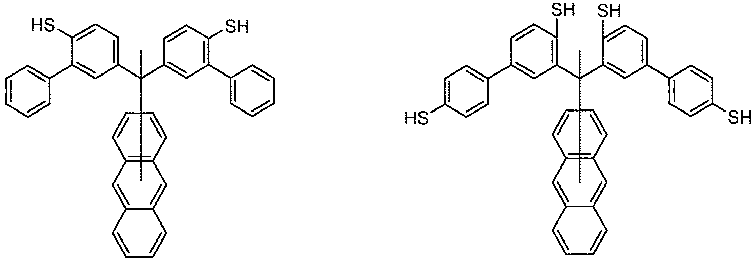

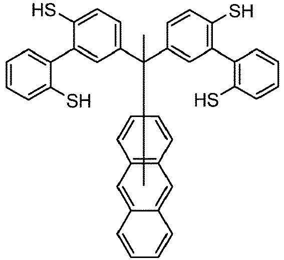

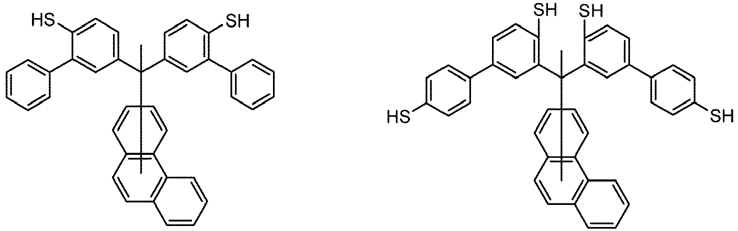

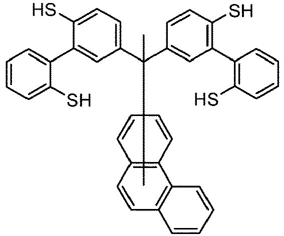

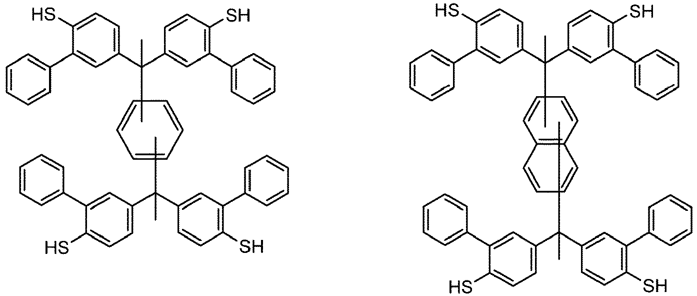

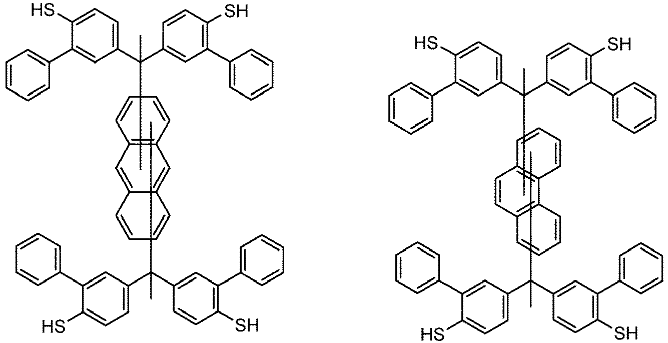

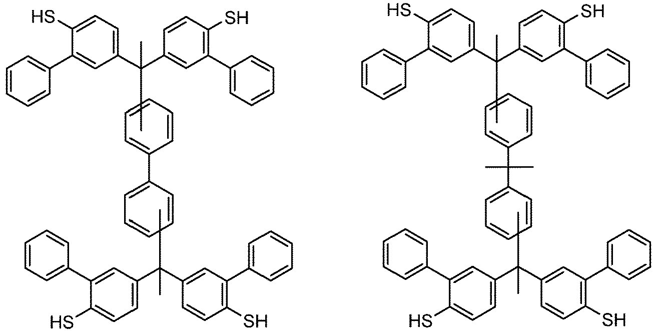

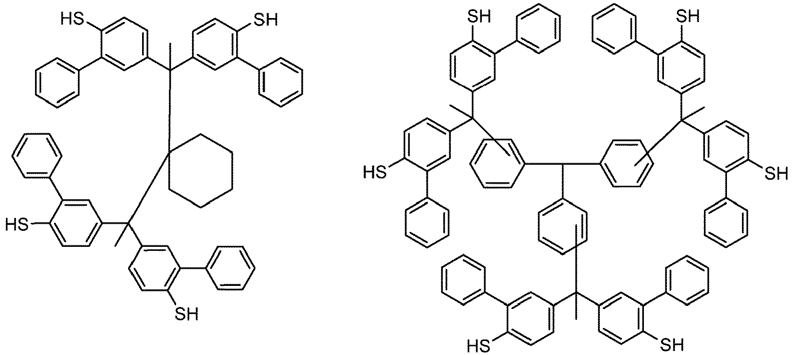

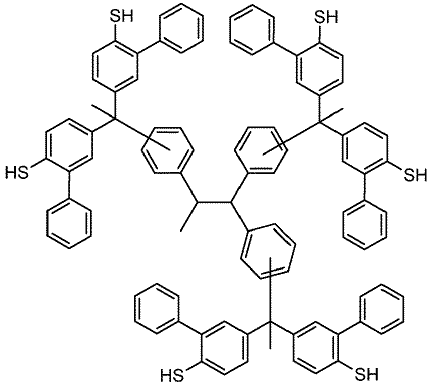

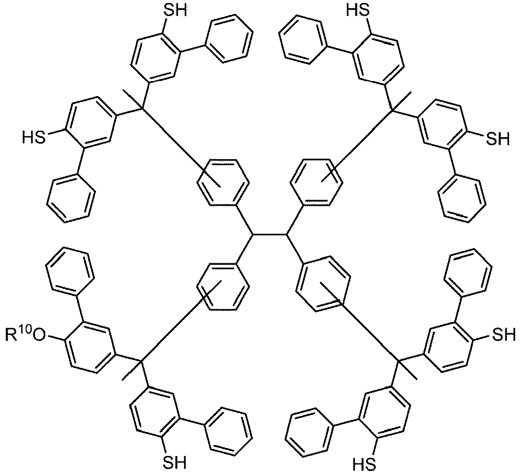

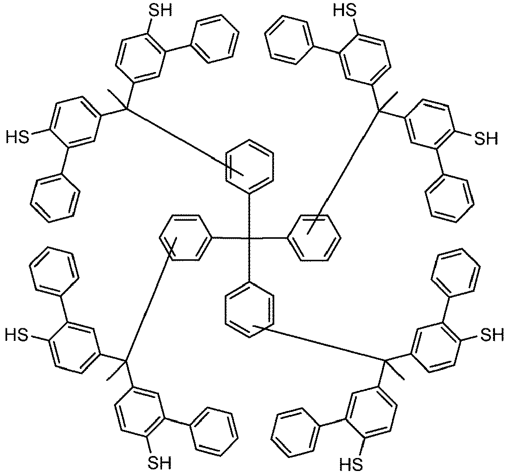

As the compound represented by the formula (3), the compounds represented by the following formulas can be further used.

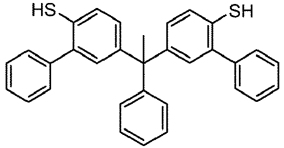

From the viewpoint of the availability of raw materials, the compounds represented below are further preferable.

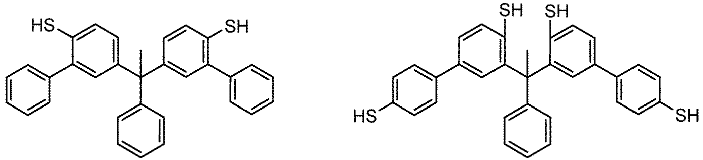

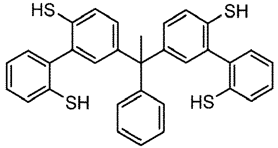

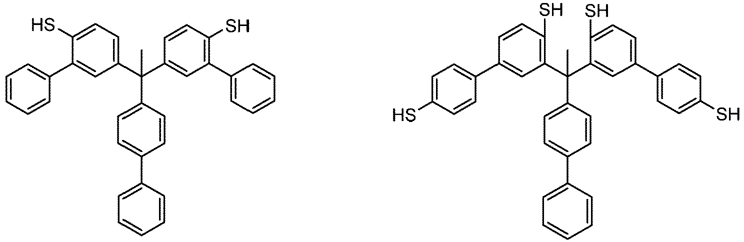

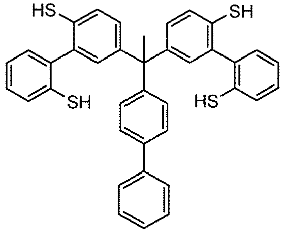

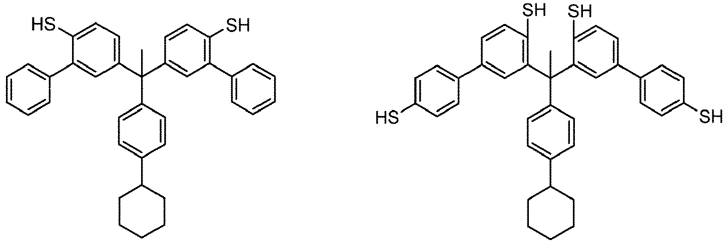

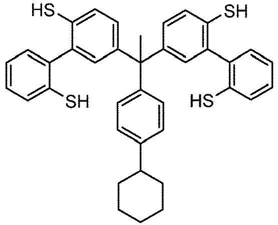

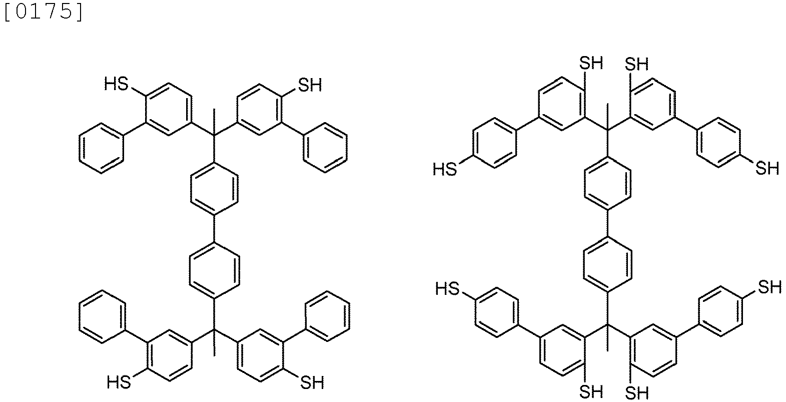

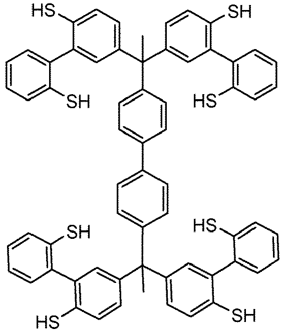

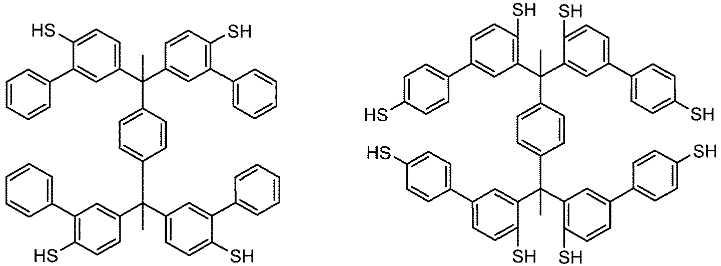

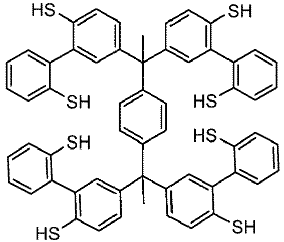

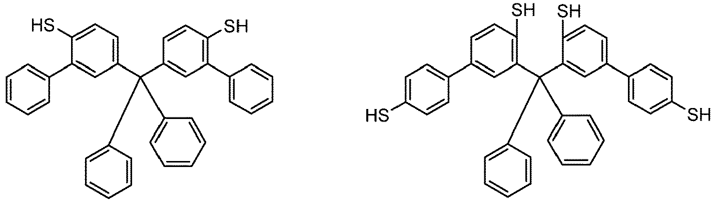

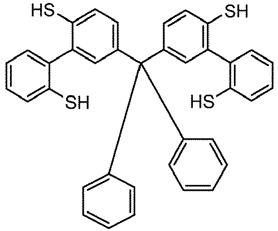

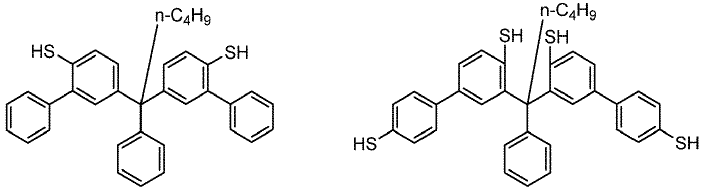

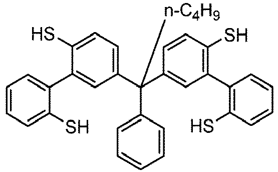

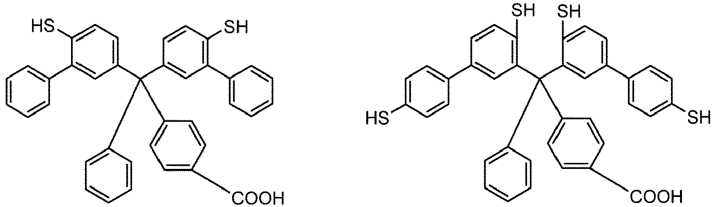

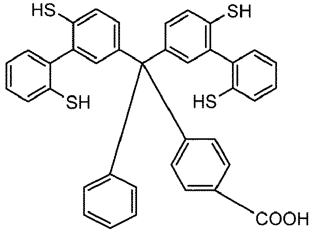

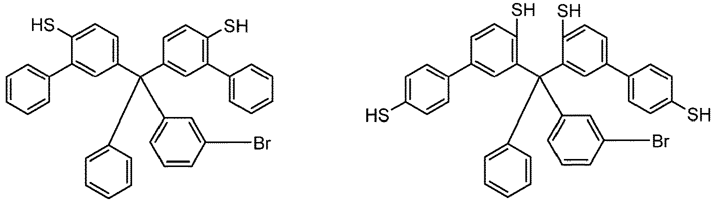

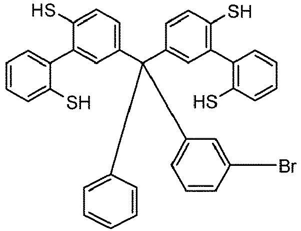

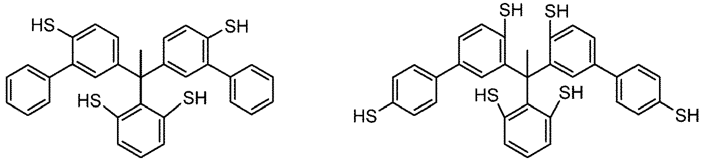

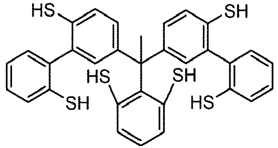

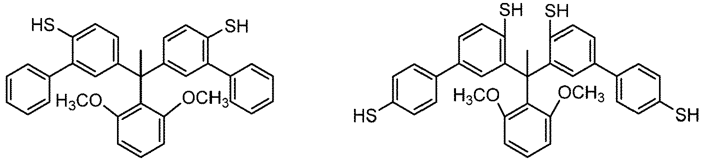

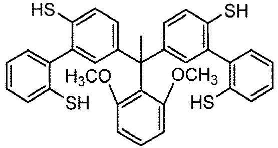

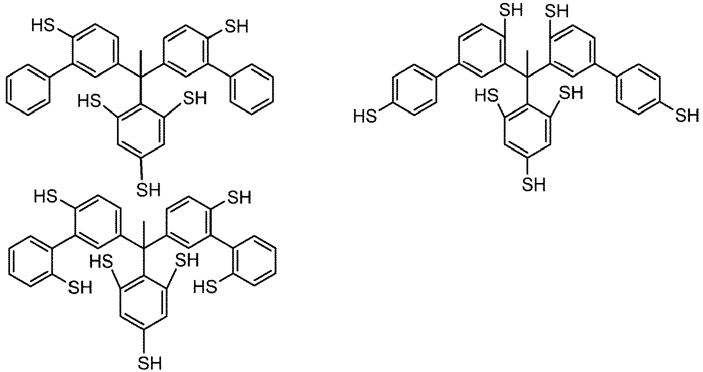

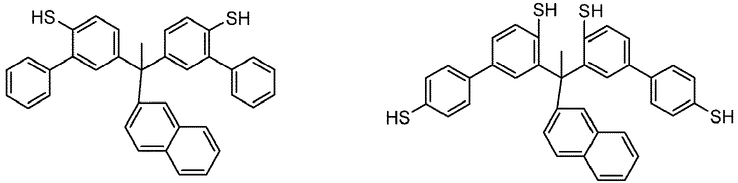

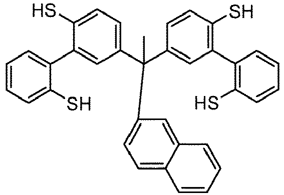

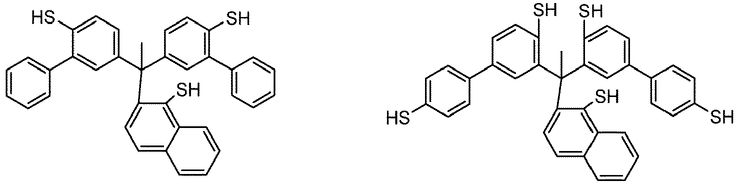

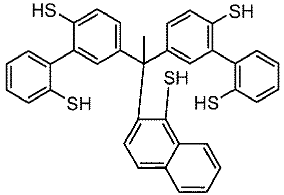

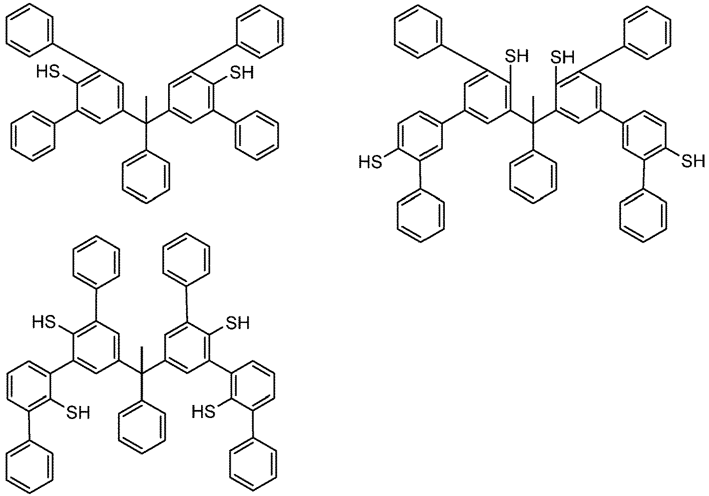

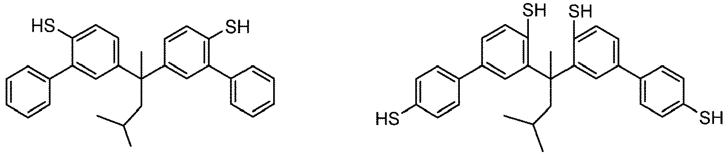

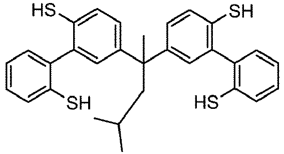

### [Compound represented by formula (4)]

The compound of the present embodiment is preferably represented by the following formula (4). When the compound of the present embodiment has the following structure, the heat resistance and the solvent solubility are further increased.

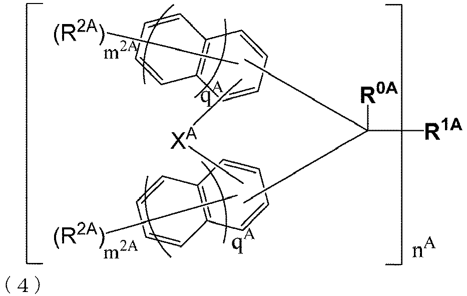

In the formula (4), R^{0A} is as defined in the above R^{Y}, and is a hydrogen atom, an alkyl group having 1 to 30 carbon atoms and optionally having a substituent, or an aryl group having 6 to 30 carbon atoms and optionally having a substituent.

R^{1A} is an n^{A}-valent group having 1 to 60 carbon atoms or a single bond;
each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group; and
in the formula (4), at least one R^{2A} is a thiol group.
n^{A} is an integer of 1 to 4, where when n^{A} is an integer of 2 or larger in the formula (4), n^{A} structural formulas within the parentheses [ ] are the same or different.

Each X^{A} is independently an oxygen atom, a sulfur atom or not a crosslink. Herein, X^{A} is preferably an oxygen atom or a sulfur atom and more preferably an oxygen atom, because there is a tendency to exhibit high heat resistance. Preferably, X^{A} is not a crosslink from the viewpoint of solubility.

Each m^{2A} is independently an integer of 0 to 6. However, at least one m^{2A} is an integer of 1 to 6.

Each q^{A} is independently 0 or 1. When q^{A} is 0, the site represented by the naphthalene structure in the formula (4) represents a benzene structure, and when q^{A} is 1, the site represents a naphthalene structure.

The above n^{A}-valent group refers to an alkyl group having 1 to 60 carbon atoms when n^{A} is 1, an alkylene group having 1 to 30 carbon atoms when n^{A} is 2, an alkanetriyl group having 2 to 60 carbon atoms when n^{A} is 3, and an alkanetetrayl group having 3 to 60 carbon atoms when n^{A} is 4. Examples of the above n^{A}-valent group include groups having a linear hydrocarbon group, a branched hydrocarbon group, and an alicyclic hydrocarbon group. Herein, the above alicyclic hydrocarbon group also includes a bridged alicyclic hydrocarbon group. Also, the above n^{A}-valent group may have an aromatic group having 6 to 60 carbon atoms.

In addition, the above n^{A}-valent hydrocarbon group may have an alicyclic hydrocarbon group, a double bond, a heteroatom, or an aromatic group having 6 to 60 or 6 to 30 carbon atoms. Herein, the above alicyclic hydrocarbon group also includes a bridged alicyclic hydrocarbon group.

The compound represented by the above formula (4) has high heat resistance attributed to its rigid structure, in spite of its relatively low molecular weight, and can therefore be used even under high temperature baking conditions. Also, the compound represented by the above formula (4) has quaternary carbon in the molecule, which inhibits crystallinity, and is thus suitably used as a film forming composition for lithography that can be used in film production for lithography.

Furthermore, the compound represented by the above formula (4) has high solubility in a safe solvent and has good heat resistance and etching resistance. Therefore, the resist forming composition for lithography of the present embodiment imparts a good shape to a resist pattern.

Moreover, the compound represented by the above formula (4) has a relatively low molecular weight and a low viscosity and therefore facilitates enhancing film smoothness while uniformly and completely filling even the steps of an uneven substrate (particularly having fine space, hole pattern, etc.). As a result, an underlayer film forming composition for lithography using this compound is capable of relatively advantageously enhancing embedding and smoothing properties. Moreover, the compound has a relatively high carbon concentration and therefore also imparts high etching resistance.

The compound represented by the above formula (4) has high refractive index and is prevented from being stained by heat treatment in a wide range from a low temperature to a high temperature. Therefore, the composition is also useful as various optical component forming compositions. The optical component is used in the form of a film or a sheet and is also useful as a plastic lens (a prism lens, a lenticular lens, a microlens, a Fresnel lens, a viewing angle control lens, a contrast improving lens, etc.), a phase difference film, a film for electromagnetic wave shielding, a prism, an optical fiber, a solder resist for flexible printed wiring, a plating resist, an interlayer insulating film for multilayer printed circuit boards, and a photosensitive optical waveguide.

The compound represented by the above formula (4) is preferably a compound represented by the following formula (4-1) from the viewpoint of easy crosslinking and solubility in an organic solvent.

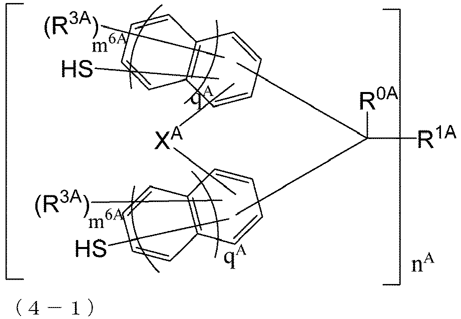

In the formula (4-1), R^{0A}, R^{1A}, n^{A}, q^{A}, and X^{A} are as defined in the description of the above formula (4).

Each R^{3A} is independently a halogen atom, an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, or an alkoxy group having 1 to 30 carbon atoms and optionally having a substituent.

Each m^{6A} is independently an integer of 0 to 5.

In addition, the compound is preferably a compound represented by the following formula (4a) from the viewpoint of the supply of raw materials.

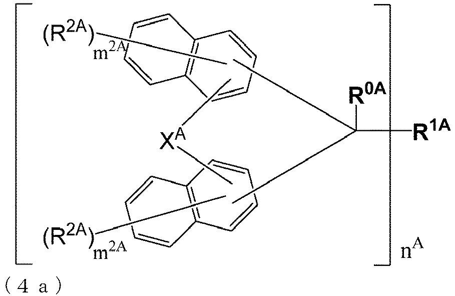

In the above formula (4a), X^{A}, R^{0A} to R^{2A}, m^{2A}, and n^{A} are as defined in the description of the above formula (4) .

In addition, the compound is more preferably a compound represented by the following formula (4b) from the viewpoint of solubility in an organic solvent.

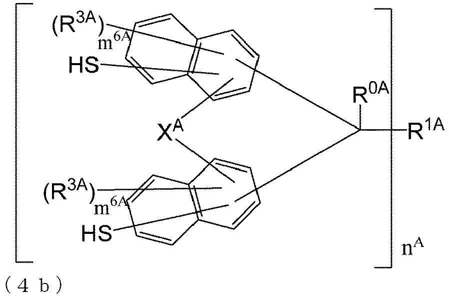

In the above formula (4b), X^{A}, R^{0A}, R^{1A}, and n^{A} are as defined in the description of the above formula (4), and R^{3A} and m^{6A} are as defined in the description of the above formula (4-1).

In addition, the compound is further preferably a compound represented by the following formula (4c) from the viewpoint of solubility in an organic solvent.

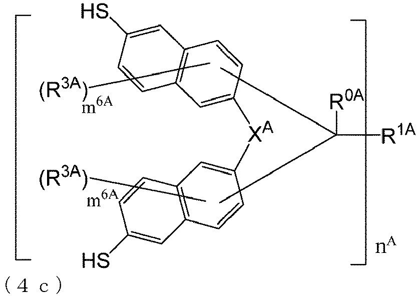

In the above formula (4c), X^{A}, R^{0A}, R^{1A}, and n^{A} are as defined in the description of the above formula (4), and R^{3A} and m^{6A} are as defined in the description of the above formula (4-1).

The compound represented by the above formula (4) is particularly preferably a compound represented by the following formula (4d-1) or (4d-2) from the viewpoint of further solubility in an organic solvent.

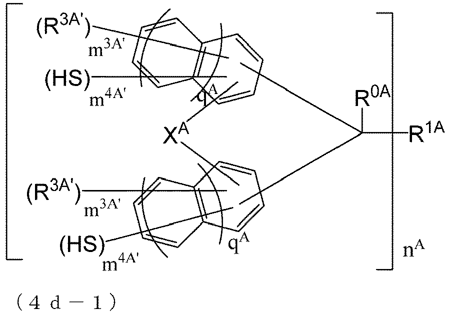

In the above formula (4d-1), R^{0A}, R^{1A}, n^{A}, q^{A}, and X^{A} are as defined in the description of the above formula (4); and each R^{3A'} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, or a halogen atom. m^{3A'} is an integer of 0 to 6; m^{4A'} is an integer of 1 to 7; and each m^{3A'} + m^{4A'} is independently an integer of 1 to 7.

Examples of R^{0A} include a methyl group, an ethyl group, a propyl group, a butyl group, a pentyl group, a hexyl group, a heptyl group, an octyl group, a nonyl group, a decyl group, an undecyl group, a dodecyl group, a triacontyl group, a phenyl group, a naphthyl group, an anthracenyl group, a pyrenyl group, a biphenyl group, and a heptacenyl group.

Examples of R^{3A'} include a methyl group, an ethyl group, a propyl group, a butyl group, a pentyl group, a hexyl group, a heptyl group, an octyl group, a nonyl group, a decyl group, an undecyl group, a dodecyl group,
a triacontyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotriacontyl group, a norbornyl group, an adamantyl group, a phenyl group, a naphthyl group, an anthracenyl group, a pyrenyl group, a biphenyl group, a heptacenyl group, a vinyl group, an allyl group, a triacontenyl group, a methoxy group, an ethoxy group, a triacontyloxy group, a fluorine atom, a chlorine atom, a bromine atom, and an iodine atom.

R^{0A} and R^{3A'} listed above include isomers. For example, a butyl group includes a n-butyl group, an isobutyl group, a sec-butyl group, and a tert-butyl group.

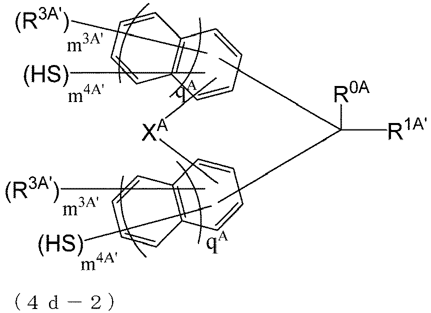

In the above formula (4d-2), R^{0A}, q^{A}, and X^{A} are as defined in the description of the above formula (4); R^{3A'}, m^{3A'}, and m^{4A'} are as defined in the description of the above formula (4d-1); and R^{1A'} is a monovalent group having 1 to 60 carbon atoms.

The compound represented by the above formula (4) preferably has any of the following structures from the viewpoint of the availability of raw materials.

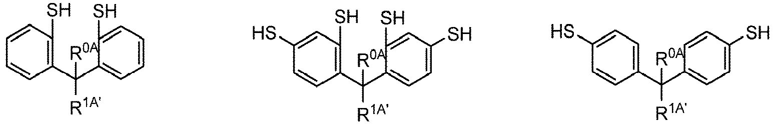

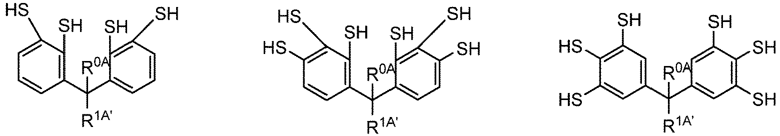

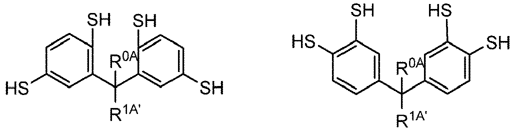

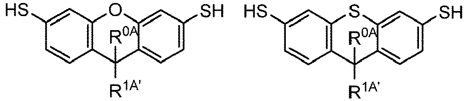

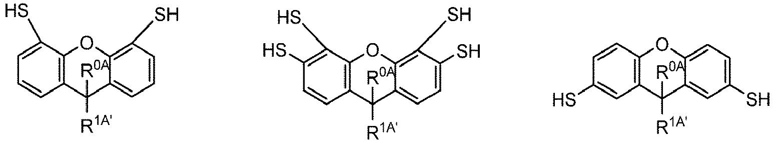

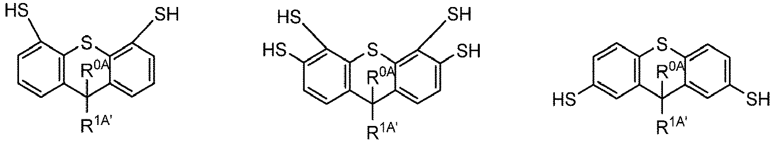

In the above compounds, R^{0A} is as defined in the description of the above formula (4), and R^{1A'} is as defined in the description of the above formula (4d-2).

It is preferable that the above compound have a xanthene skeleton or a thioxanthene skeleton from the viewpoint of heat resistance.

The compound represented by the formula (4) preferably has any of the following structures from the viewpoint of etching resistance.

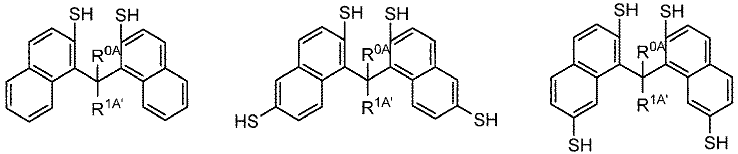

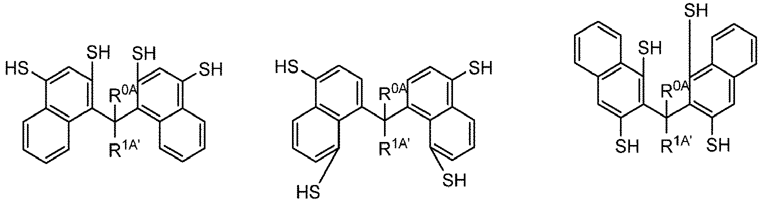

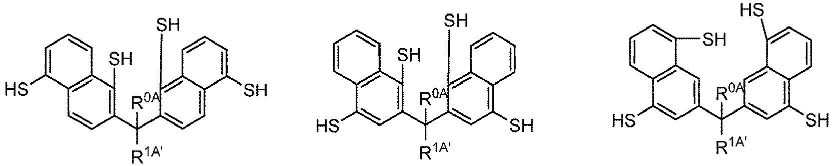

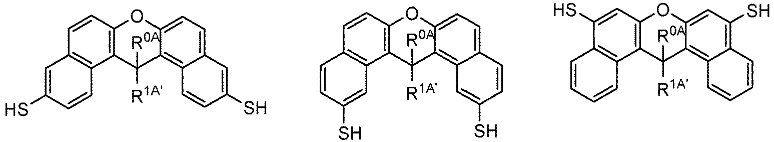

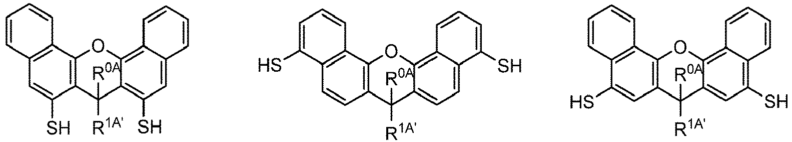

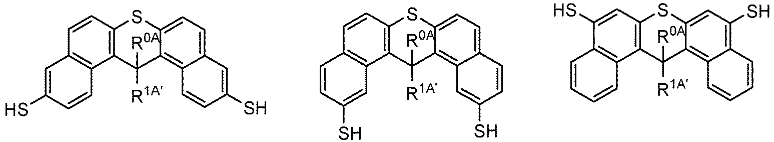

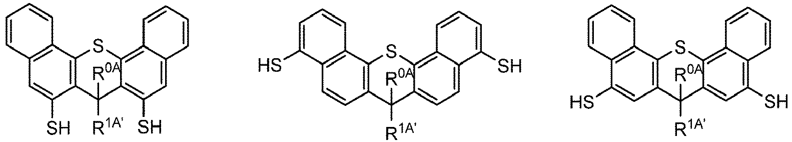

In the above compounds, R^{0A} is as defined in the description of the above formula (4), and R^{1A'} is as defined in the description of the above formula (4d-2).

It is preferable that the above compound have a dibenzoxanthene skeleton from the viewpoint of heat resistance.

Examples of the compound represented by the formula (4) include those having the following structures.

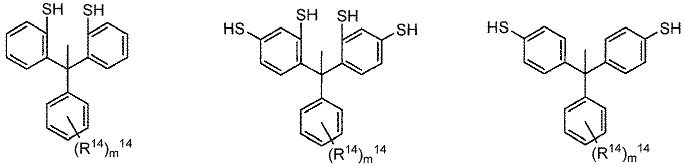

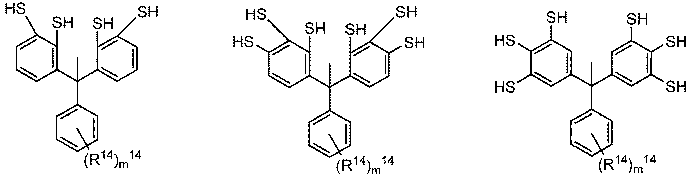

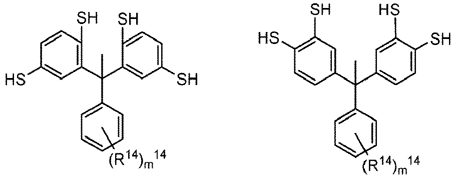

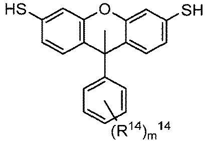

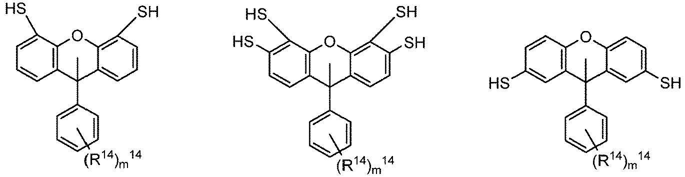

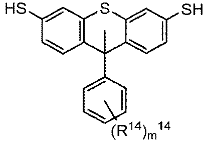

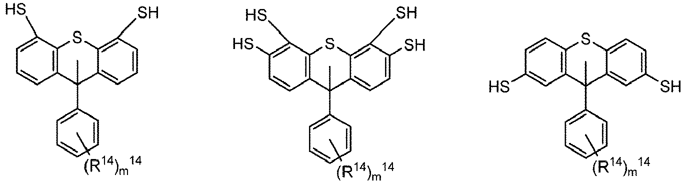

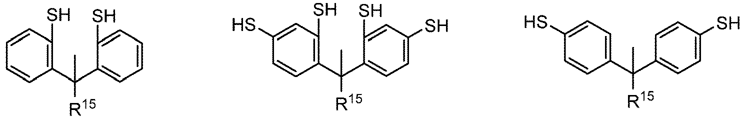

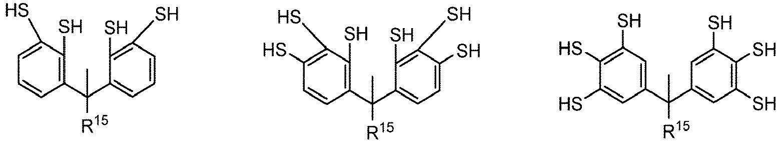

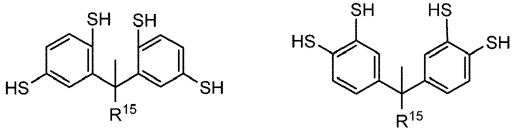

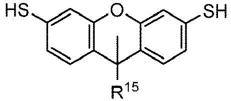

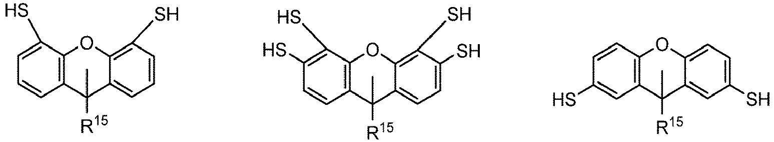

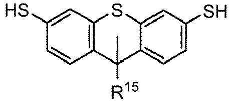

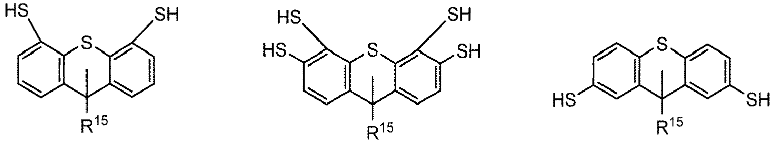

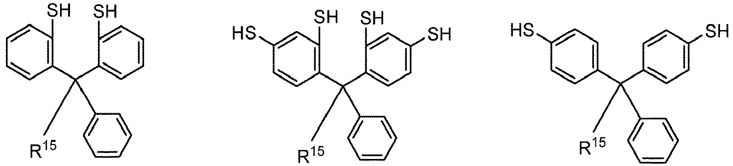

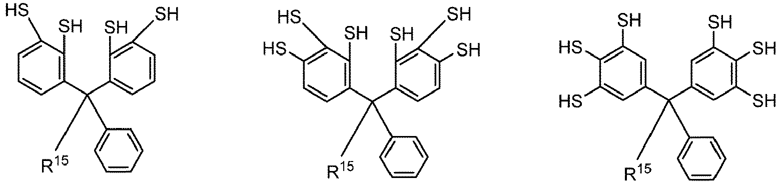

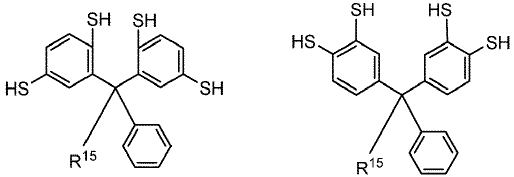

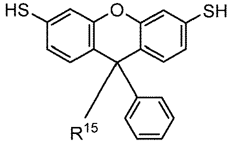

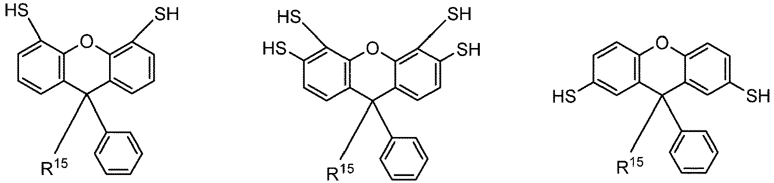

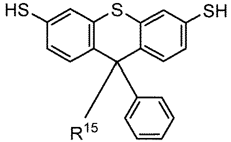

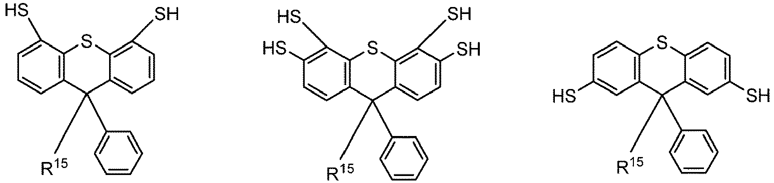

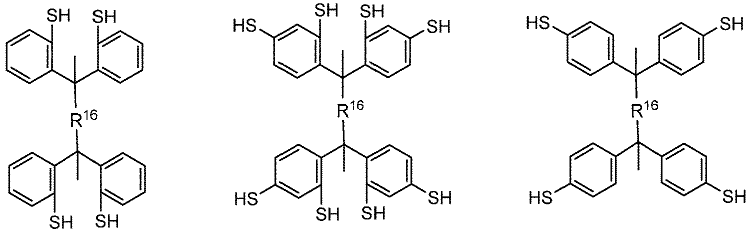

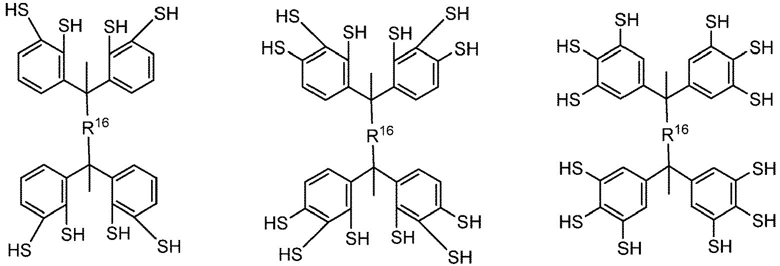

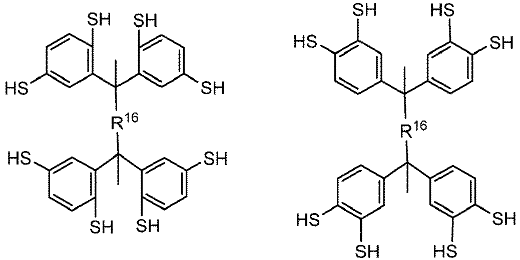

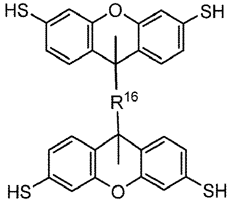

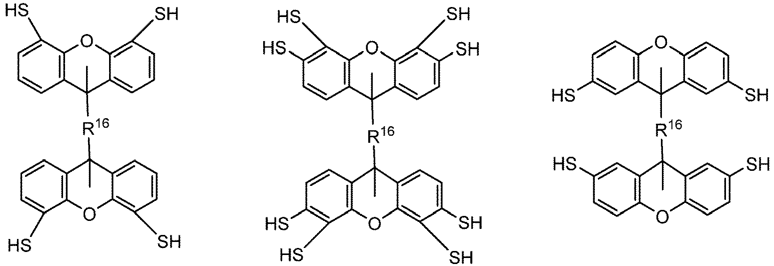

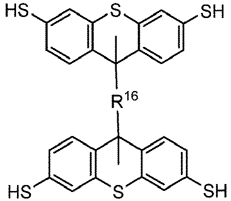

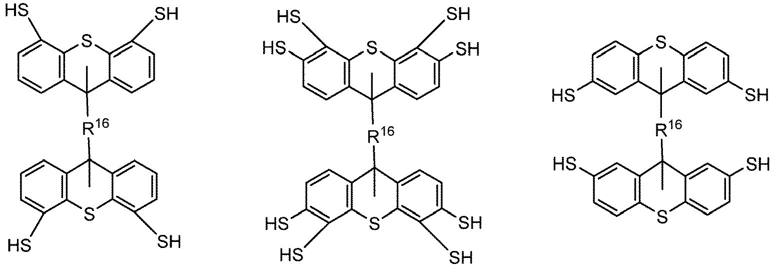

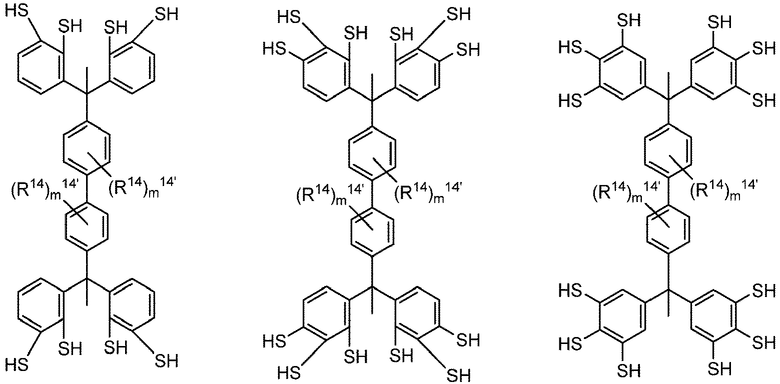

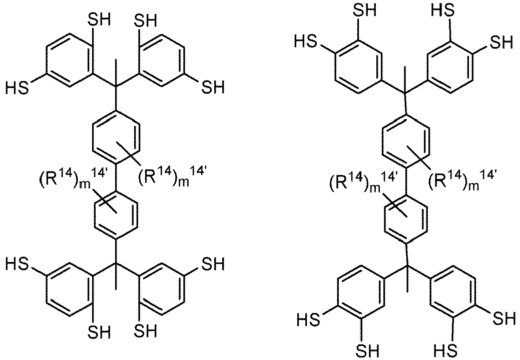

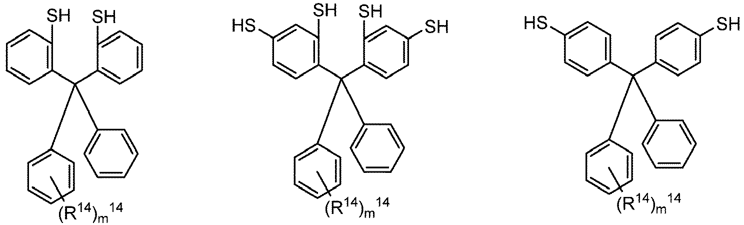

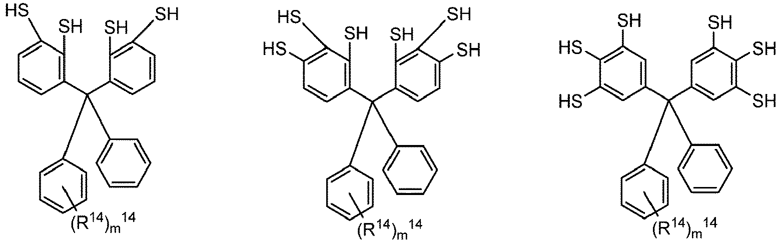

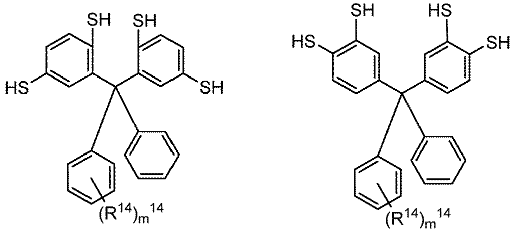

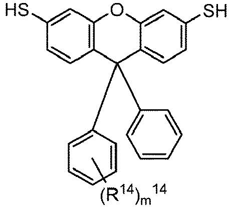

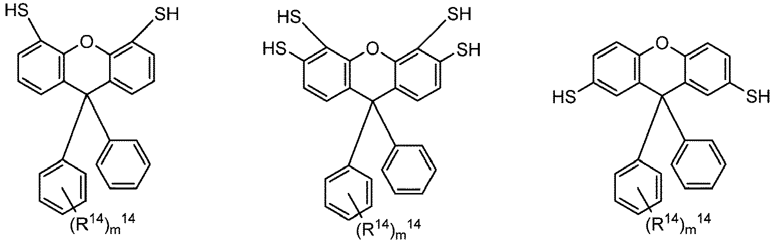

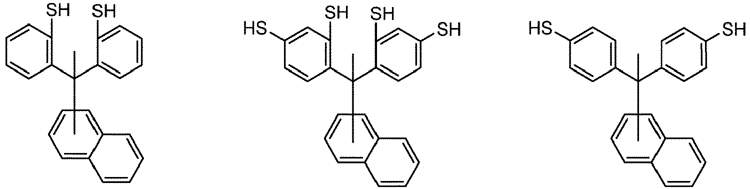

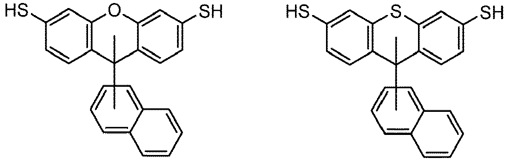

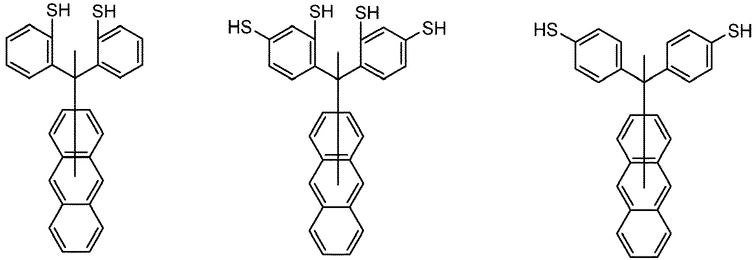

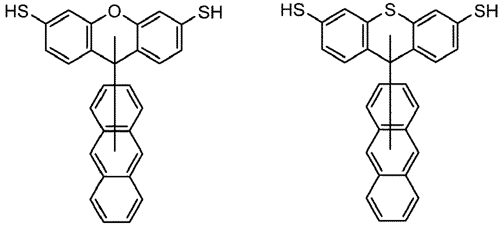

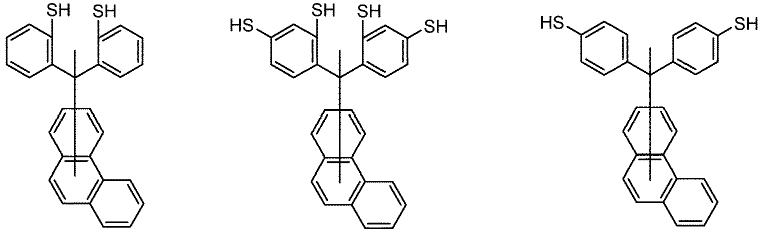

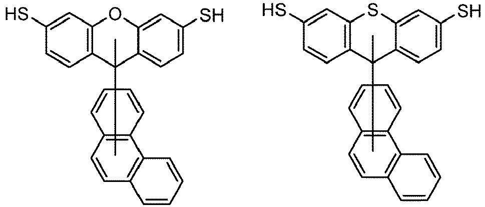

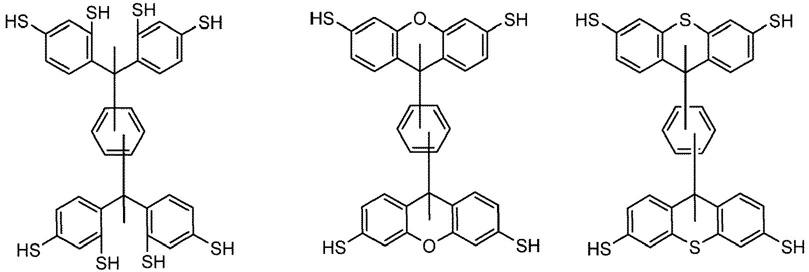

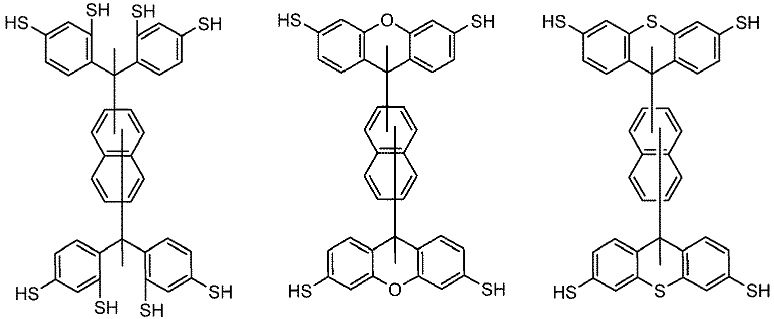

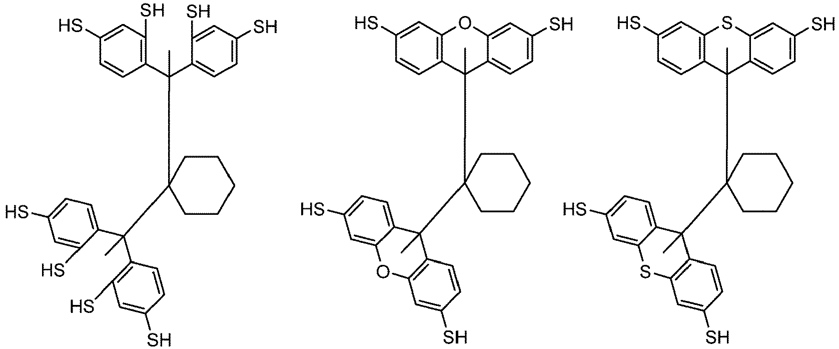

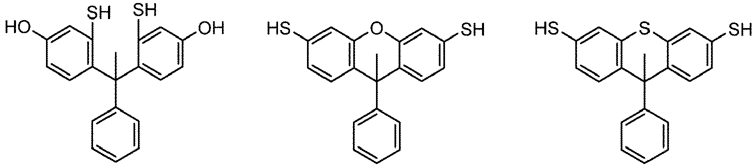

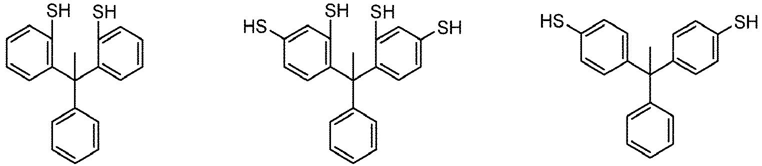

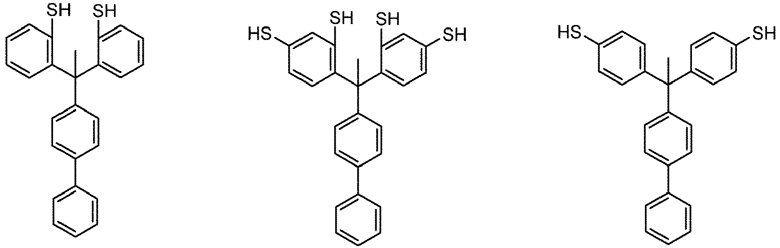

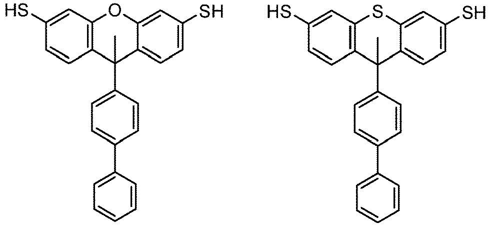

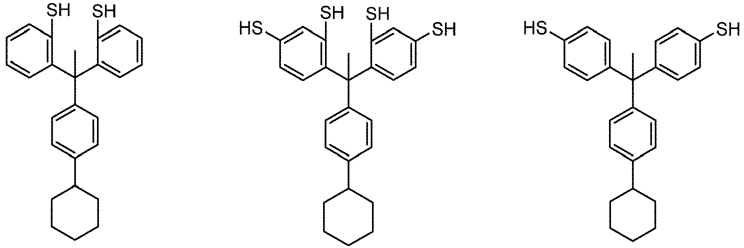

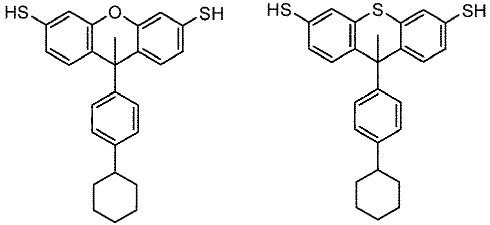

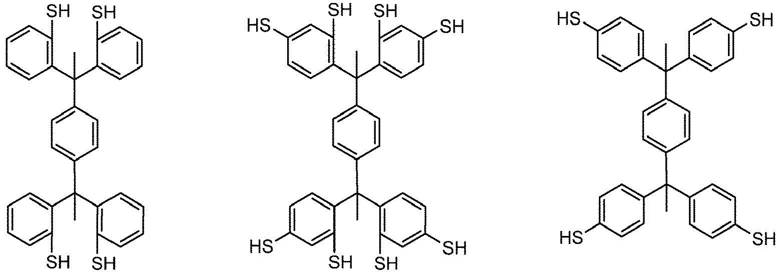

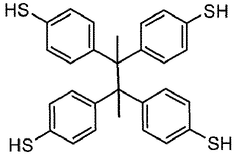

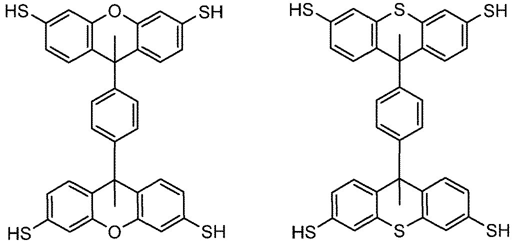

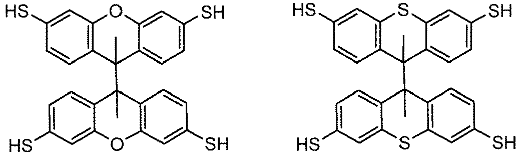

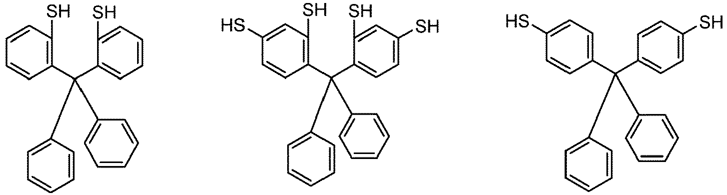

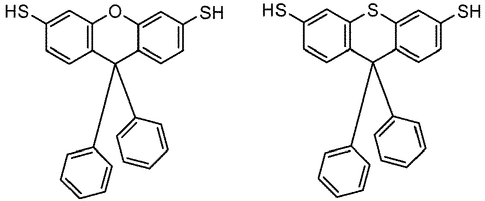

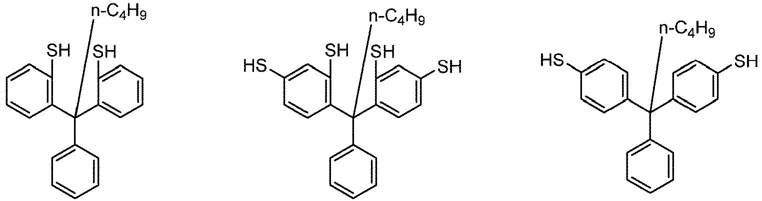

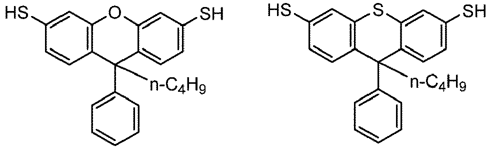

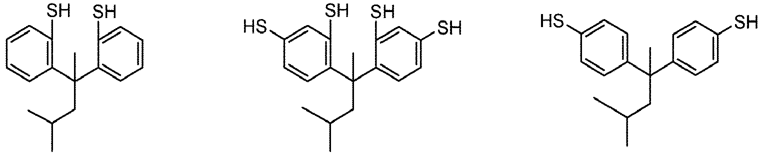

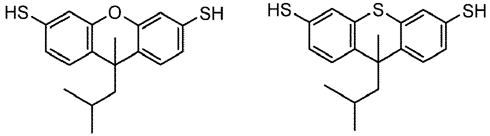

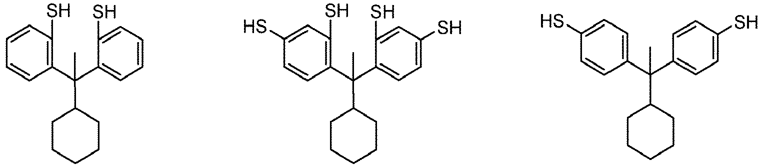

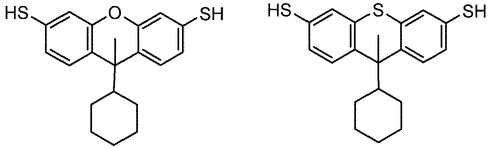

In the above compounds, R¹⁴ is an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; m¹⁴ is an integer of 0 to 5; and m^{14'} is an integer of 0 to 4.

R¹⁵ is an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group.

R¹⁶ is a linear, branched or cyclic alkylene group having 1 to 30 carbon atoms and optionally having a substituent, a divalent aryl group having 6 to 30 carbon atoms and optionally having a substituent, a divalent alkenyl group having 2 to 30 carbon atoms and optionally having a substituent, or an alkynyl group having 2 to 30 carbon atoms and optionally having a substituent.

It is preferable that the above compound have a xanthene skeleton from the viewpoint of heat resistance.

### [Method for producing compound represented by formula (1)]

The compound represented by the formula (1) according to the present embodiment can be arbitrarily synthesized by the application of a publicly known approach, and the synthesis approach is not particularly limited.

For example, the polyphenol compound can be obtained, for example, by subjecting a phenol and a corresponding aldehyde or ketone to polycondensation reaction in the presence of an acid catalyst at normal pressure. It is preferable to use a phenol containing a sulfur atom and/or an aldehyde or ketone containing a sulfur atom. If necessary, this reaction can also be carried out under increased pressure.

Examples of the above phenol include, but not particularly limited to, phenol, cresol, methoxybenzene, catechol, resorcinol, hydroquinone, trimethylhydroquinone, pyrogallol, phenylphenol, biphenol, methylbiphenol, methoxybiphenol, naphthol, methylnaphthol, methoxynaphthol, naphthalenediol, and naphthalenetriol. These phenols can be used alone as one kind or can be used in combination of two or more kinds. Among them, it is preferable to use phenol, cresol, catechol, resorcinol, hydroquinone, phenylphenol, biphenol, naphthol, naphthalenediol, and naphthalenetriol from the viewpoint of the stable supply of raw materials, and it is more preferable to use phenylphenol, biphenol, naphthol, naphthalenediol, and naphthalenetriol from the viewpoint of high heat resistance.

Examples of the aldehyde include, but not particularly limited to, formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, furfural, methylthiobenzaldehyde, thiophene carboxaldehyde, methylthiothiophene carboxaldehyde, formyltetrathiafulvalene, and benzothiophene carboxaldehyde. These aldehydes can be used alone as one kind or can be used in combination of two or more kinds. Among them, it is preferable to use benzaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde, furfural, methylthiobenzaldehyde, thiophene carboxaldehyde, methylthiothiophene carboxaldehyde, formyltetrathiafulvalene, and benzothiophene carboxaldehyde from the viewpoint of providing high heat resistance; it is more preferable to use benzaldehyde, hydroxybenzaldehyde, chlorobenzaldehyde, nitrobenzaldehyde, methylbenzaldehyde, ethylbenzaldehyde, butylbenzaldehyde, cyclohexylbenzaldehyde, biphenylaldehyde, naphthaldehyde, anthracenecarbaldehyde, phenanthrenecarbaldehyde, pyrenecarbaldehyde, furfural, methylthiobenzaldehyde, thiophene carboxaldehyde, methylthiothiophene carboxaldehyde, formyltetrathiafulvalene, and benzothiophene carboxaldehyde from the viewpoint of improving etching resistance; and further preferable are methylthiobenzaldehyde, thiophene carboxaldehyde, methylthiothiophene carboxaldehyde, formyltetrathiafulvalene, and benzothiophene carboxaldehyde, to all of which a sulfur atom is introduced, from the viewpoint of improving the refractive index.

Examples of the ketone include, but not particularly limited to, acetone, methyl ethyl ketone, cyclobutanone, cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, diphenylcarbonylbiphenyl, mercaptobenzonic acid, methylthioacetophenone, acetylthiophene, and acetylbenzothiophene. These ketones can be used alone as one kind or can be used in combination of two or more kinds. Among them, it is preferable to use cyclopentanone, cyclohexanone, norbornanone, tricyclohexanone, tricyclodecanone, adamantanone, fluorenone, benzofluorenone, acenaphthenequinone, acenaphthenone, anthraquinone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, diphenylcarbonylbiphenyl, mercaptobenzonic acid, methylthioacetophenone, acetylthiophene, and acetylbenzothiophene from the viewpoint of providing high heat resistance; it is more preferable to use acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, diphenylcarbonylbiphenyl, mercaptobenzonic acid, methylthioacetophenone, acetylthiophene, and acetylbenzothiophene from the viewpoint of improving etching resistance; and further preferable are mercaptobenzonic acid, methylthioacetophenone, acetylthiophene, and acetylbenzothiophene, to all of which a sulfur atom is introduced, from the viewpoint of improving the refractive index.

As the aldehyde or the ketone, an aldehyde having an aromatic ring or a ketone having aromatics is preferably used from the viewpoint that both high heat resistance and high etching resistance are achieved.

The acid catalyst used in the above reaction can be arbitrarily selected for use from publicly known acid catalysts and is not particularly limited. Inorganic acids, organic acids, Lewis acids, solid acids, and the like are widely known as such acid catalysts, and examples thereof include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is more preferably used from the viewpoint of production such as easy availability and handleability. The acid catalysts can be used alone as one kind or can be used in combination of two or more kinds. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

Upon the above reaction, a reaction solvent may be used. The reaction solvent is not particularly limited as long as the reaction of the aldehyde or the ketone used with the phenol proceeds, and can be arbitrarily selected for use from publicly known solvents. Examples of the reaction solvent include water, methanol, ethanol, propanol, butanol, tetrahydrofuran, dioxane, ethylene glycol dimethyl ether, ethylene glycol diethyl ether, and a mixed solvent thereof. The solvents can be used alone as one kind or can be used in combination of two or more kinds.

Also, the amount of these reaction solvents used can be arbitrarily set according to, for example, the kind of the raw materials used and the catalyst used and moreover the reaction conditions and is not particularly limited, but is preferably in the range of 0 to 2000 parts by mass based on 100 parts by mass of the reaction raw materials. Furthermore, the reaction temperature in the above reaction can be arbitrarily selected according to the reactivity of the reaction raw materials and is not particularly limited, but is usually within the range of 10 to 200°C.

In order to obtain the polyphenol compound, a higher reaction temperature is more preferable. Specifically, the range of 60 to 200°C is preferable. The reaction method can be arbitrarily selected for use from publicly known approaches and is not particularly limited, and examples thereof include a method of charging the phenol, the aldehyde or the ketone, and the catalyst in one portion, and a method of dropping the phenol and the aldehyde or the ketone in the presence of the catalyst. After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, the compound that is the target compound can be isolated.

Examples of the preferable reaction conditions include using 1 mol to an excess of the phenol and 0.001 to 1 mol of the acid catalyst based on 1 mol of the aldehyde or the ketone, and reacting them at 50 to 150°C at normal pressure for about 20 minutes to 100 hours.

The target compound can be isolated by a publicly known method after the reaction terminates. The compound represented by the above formula (1) which is the target compound can be obtained, for example, by concentrating the reaction liquid, precipitating the reaction product by the addition of pure water, cooling the reaction liquid to room temperature, then separating the precipitates by filtration, filtering and drying the obtained solid matter, then separating and purifying the solid matter from by-products by column chromatography, and distilling off the solvent, followed by filtration and drying.

### [Method for producing compound represented by formula (3)]

The compound represented by the formula (3) used in the present embodiment can be arbitrarily synthesized by the application of a publicly known approach, and the synthesis approach is not particularly limited. For example, there are (i) a method of subjecting a biphenol, binaphthol or bianthracenol and a corresponding ketone to polycondensation reaction in the presence of an acid catalyst to obtain a polyphenol, and substituting a hydroxy group of the polyphenol with a thiol group according to a method described in J. Am. Chem. Soc., Vol. 122, No. 28, 2000, and (ii) a method of subjecting a methine site of a triarylmethane obtained by subjecting a biphenol, binaphthol or bianthracenol and a corresponding aldehyde to polycondensation in the presence of an acid catalyst, or a xanthene to substitution reaction to obtain a polyphenol, and substituting a hydroxy group of the polyphenol with a thiol group according to a method described in J. Am. Chem. Soc., Vol. 122, No. 28, 2000.

In addition, examples of (i) the method of subjecting a biphenol, binaphthol or bianthracenol and a corresponding ketone to polycondensation reaction in the presence of an acid catalyst include (a) a method of carrying out the polycondensation reaction in an organic solvent, (b) a method of carrying out the polycondensation reaction in an aqueous solvent, and (c) a method of carrying out the polycondensation reaction with no solvent.

In (i) (a) the method of subjecting a biphenol, binaphthol or bianthracenol and a corresponding ketone to polycondensation reaction in the presence of an acid catalyst in an organic solvent, the compound represented by the above formula (3) can be obtained by subjecting a biphenol, binaphthol or bianthracenol and a corresponding ketone to polycondensation reaction in the presence of an acid catalyst at normal pressure. In addition, an acid dissociation group can be introduced into at least one phenolic hydroxy group of that compound according to a publicly known method. If necessary, this reaction can also be carried out under increased pressure.

In (i) (b) or (c) the method of subjecting a biphenol, binaphthol or bianthracenol and a corresponding ketone to polycondensation reaction in the presence of an acid catalyst in an aqueous solvent or with no solvent, the compound represented by the above formula (3) can be obtained by subjecting a biphenol, binaphthol or bianthracenol and a corresponding ketone to polycondensation reaction in the presence of an acid and a mercapto catalyst. In addition, an acid dissociation group can be introduced into at least one phenolic hydroxy group of that compound according to a publicly known method. Also, the present reaction can be carried out under reduced pressure, at normal pressure, or under increased pressure.

Examples of the above biphenol include, but not particularly limited to, biphenol, methylbiphenol, and methoxybiphenol. These biphenols can be used alone as one kind or can be used in combination of two or more kinds. Among them, biphenol is more preferably used from the viewpoint of the stable supply of raw materials.

Examples of the above binaphthol include, but not particularly limited to, binaphthol, methylbinaphthol, and methoxybinaphthol. These binaphthols can be used alone as one kind or can be used in combination of two or more kinds. Among them, binaphthol is more preferably used from the viewpoint of increasing a carbon atom concentration and improving heat resistance.

Examples of the above bianthracenol include, but not particularly limited to, bianthracenol, methylbianthracenol, and methoxybianthracenol. These bianthracenols can be used alone as one kind or can be used in combination of two or more kinds. Among them, bianthracenol is more preferably used from the viewpoint of increasing a carbon atom concentration and improving heat resistance.

Examples of the above ketone include, but not particularly limited to, acetone, methyl ethyl ketone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl. These ketones can be used alone as one kind or can be used in combination of two or more kinds. Among them, it is preferable to use acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl from the viewpoint of conferring high heat resistance, and it is more preferable to use acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl from the viewpoint of conferring high etching resistance.

As the ketone, a ketone having an aromatic ring is preferably used from the viewpoint that both high heat resistance and high etching resistance are achieved.

The acid catalyst used in the above reaction can be arbitrarily selected for use from publicly known acid catalysts and is not particularly limited. Inorganic acids, organic acids, Lewis acids, and solid acids are widely known as such acid catalysts, and examples thereof include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferably used from the viewpoint of production such as easy availability and handleability. The acid catalysts can be used alone as one kind or can be used in combination of two or more kinds. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

The mercapto catalyst used in the above reaction can be arbitrarily selected for use from publicly known mercapto catalysts and is not particularly limited. As such a catalyst, an alkylthiol and a mercaptocarboxylic acid are widely known. Examples of the alkylthiol include an alkyl mercaptan having 1 to 12 carbon atoms, preferably n-octyl mercaptan, n-decyl mercaptan, and n-dodecyl mercaptan, and examples of the mercaptocarboxylic acid include, but not particularly limited to, 2-mercaptopropionic acid and 3-mercaptopropionic acid. Among them, from the viewpoint of production, n-octyl mercaptan, n-decyl mercaptan, and n-dodecyl mercaptan are preferable. The mercapto catalysts can be used alone as one kind or can be used in combination of two or more kinds. Also, the amount of the mercapto catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

Upon the above reaction, a reaction solvent may be used. The reaction solvent is not particularly limited as long as the reaction of the ketone used with the biphenol, the binaphthol, or the bianthracenediol proceeds, and can be arbitrarily selected for use from publicly known solvents. Examples thereof include water, methanol, ethanol, propanol, butanol, tetrahydrofuran, dioxane, ethylene glycol dimethyl ether, ethylene glycol diethyl ether, and a mixed solvent thereof. The solvents can be used alone as one kind or can be used in combination of two or more kinds.

Also, the amount of these solvents used can be arbitrarily set according to, for example, the kind of the raw materials used and the catalyst used and moreover the reaction conditions and is not particularly limited, but is preferably in the range of 0 to 2000 parts by mass based on 100 parts by mass of the reaction raw materials. Furthermore, the reaction temperature in the above reaction can be arbitrarily selected according to the reactivity of the reaction raw materials and is not particularly limited, but is usually within the range of 10 to 200°C.

In order to obtain the compound represented by the formula (3) of the present embodiment, a higher reaction temperature is more preferable. Specifically, the range of 60 to 200°C is preferable. The reaction method can be arbitrarily selected for use from publicly known approaches and is not particularly limited, and there are a method of charging the biphenol, the binaphthol or the bianthracenediol, the ketone, and the catalyst in one portion, and a method of dropping the biphenol, the binaphthol or the bianthracenediol, and the ketone, in the presence of the catalyst. After the polycondensation reaction terminates, isolation of the obtained compound can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, the compound that is the target compound can be obtained.

Examples of the preferable reaction conditions include using 1.0 mol to an excess of the biphenol, the binaphthol or the bianthracenediol and 0.001 to 1 mol of the acid catalyst based on 1 mol of the ketone, and reacting them at 50 to 150°C at normal pressure for about 20 minutes to 100 hours.

The target compound can be isolated by a publicly known method after the reaction terminates. The compound represented by the above formula (3) which is the target compound can be obtained, for example, by concentrating the reaction liquid, precipitating the reaction product by the addition of pure water, cooling the reaction liquid to room temperature, then separating the precipitates by filtration, filtering and drying the obtained solid matter, then separating and purifying the solid matter from by-products by column chromatography, and distilling off the solvent, followed by filtration and drying.

In (ii) the method of subjecting a methine site of a triarylmethane obtained by subjecting a biphenol, binaphthol or bianthracenol and a corresponding aldehyde to polycondensation in the presence of an acid catalyst, or a xanthene to substitution, a compound (A) formed by replacing R^{Y} of the compound represented by the above formula (3) with a hydrogen atom is obtained by subjecting a biphenol, binaphthol or bianthracenol and a corresponding aldehyde to polycondensation reaction in the presence of an acid catalyst. Using a protective group introducing agent, a compound (B) is obtained in which a hydroxy group of the compound (A) is substituted with a protective group. Then, by allowing the hydrogen atom corresponding to the R^{Y} moiety of the compound represented by the above formula (3) to react with an alkylating agent in the presence of a basic catalyst, an alkyl group corresponding to the R^{Y} moiety of the compound represented by the above formula (3) is introduced. Further later, the protective group that has substituted the hydroxy group in the above compound (B) is deprotected, thereby obtaining the above formula (3). In addition, an acid dissociation group can be introduced into at least one phenolic hydroxy group of that compound according to a publicly known method. If necessary, this reaction can also be carried out under increased pressure. The above alkylating agent can be arbitrarily selected for use from publicly known alkylating agents and is not particularly limited. Examples thereof include alkyl chloride, alkyl bromide, and alkyl iodide.

In the above production method, for the method of introducing an alkyl group corresponding to the R^{Y} moiety of the compound represented by the above formula (3) into the hydrogen atom of the compound (B) corresponding to the R^{Y} moiety of the compound represented by the above formula (3), instead of allowing the hydrogen atom to react with an alkylating agent in the presence of a basic catalyst as in the above production method, the compound (B) may also be allowed to react with a halogenating agent to substitute the hydrogen atom corresponding to the R^{Y} moiety of the compound represented by the above formula (3) with a halogen atom, and then allowed to react with an alkylating agent, thereby obtaining the above formula (3). The alkylating agent can be arbitrarily selected for use from publicly known alkylating agents and is not particularly limited. Examples thereof include a Grignard reagent and an alkyllithium.

Examples of the above biphenol include, but not particularly limited to, biphenol, methylbiphenol, and methoxybiphenol. These biphenols can be used alone as one kind or can be used in combination of two or more kinds. Among them, biphenol is more preferably used from the viewpoint of the stable supply of raw materials.

Examples of the above binaphthol include, but not particularly limited to, binaphthol, methylbinaphthol, and methoxybinaphthol. These binaphthols can be used alone as one kind or can be used in combination of two or more kinds. Among them, binaphthol is more preferably used from the viewpoint of increasing a carbon atom concentration and improving heat resistance.

Examples of the above bianthracenol include, but not particularly limited to, bianthracenol, methylbianthracenol, and methoxybianthracenol. These bianthracenols can be used alone as one kind or can be used in combination of two or more kinds. Among them, bianthracenol is more preferably used from the viewpoint of increasing a carbon atom concentration and improving heat resistance.

Examples of the above aldehyde include, but not particularly limited to, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, and furfural.

The method for introducing an acid dissociation group to at least one phenolic hydroxy group of the polyphenol compound is publicly known. For example, an acid dissociation group can be introduced to at least one phenolic hydroxy group of the above compound, as follows. A compound for introducing an acid dissociation group can be synthesized by a publicly known method or easily obtained. Examples thereof include, but not particularly limited to, acid chlorides, acid anhydrides, active carboxylic acid derivative compounds such as dicarbonates, alkyl halides, vinyl alkyl ethers, dihydropyran, and halocarboxylic acid alkyl esters.

For example, the above compound is dissolved or suspended in an aprotic solvent such as acetone, tetrahydrofuran (THF), or propylene glycol monomethyl ether acetate. Subsequently, a vinyl alkyl ether such as ethyl vinyl ether, or dihydropyran is added to the solution or the suspension, and the mixture is reacted at 20 to 60°C at normal pressure for 6 to 72 hours in the presence of an acid catalyst such as pyridinium p-toluenesulfonate. The reaction liquid is neutralized with an alkali compound and added to distilled water to precipitate a white solid. Then, the separated white solid can be washed with distilled water and dried to obtain a compound in which a hydrogen atom of a hydroxy group is replaced with an acid dissociation group.

Alternatively, for example, the above compound having a hydroxy group is dissolved or suspended in an aprotic solvent such as acetone, THF, or propylene glycol monomethyl ether acetate. Subsequently, an alkyl halide such as ethyl chloromethyl ether or a halocarboxylic acid alkyl ester such as methyladamantyl bromoacetate is added to the solution or the suspension, and the mixture is reacted at 20 to 110°C at normal pressure for 6 to 72 hours in the presence of an alkali catalyst such as potassium carbonate. The reaction liquid is neutralized with an acid such as hydrochloric acid and added to distilled water to precipitate a white solid. Then, the separated white solid can be washed with distilled water and dried to obtain a compound in which a hydrogen atom of a hydroxy group is replaced with an acid dissociation group.

The timing of introducing an acid dissociation group may be not only after condensation reaction of the binaphthol with the ketone but at a stage previous to the condensation reaction. Alternatively, the introduction may be carried out after production of a resin, which will be mentioned later.

In the present embodiment, the acid dissociation group refers to a characteristic group that is cleaved in the presence of an acid to generate a functional group that alters solubility, such as an alkali soluble group. Examples of the alkali soluble group include a phenolic hydroxy group, a carboxyl group, a sulfonic acid group, and a hexafluoroisopropanol group. A phenolic hydroxy group and a carboxyl group are preferable, and a phenolic hydroxy group is particularly preferable. The above acid dissociation group preferably has the property of causing chained cleavage reaction in the presence of an acid, for achieving pattern formation with much higher sensitivity and higher resolution.

### [Method for producing compound represented by formula (4)]

The compound represented by the formula (4) used in the present embodiment can be arbitrarily synthesized by the application of a publicly known approach, and the synthesis approach is not particularly limited. For example, there are (i) a method of subjecting a phenol, naphthol or anthracenol and a corresponding ketone to polycondensation reaction in the presence of an acid catalyst to obtain a polyphenol, and substituting a phenolic hydroxy group of the polyphenol with a thiol group according to a method described in J. Am. Chem. Soc., Vol. 122, No. 28, 2000, and (ii) a method of subjecting a methine site of a triarylmethane obtained by subjecting a phenol, naphthol or anthracenol and a corresponding aldehyde to polycondensation in the presence of an acid catalyst, or a xanthene to substitution to obtain a polyphenol, and substituting a phenolic hydroxy group of the polyphenol with a thiol group according to a method described in J. Am. Chem. Soc., Vol. 122, No. 28, 2000.

In addition, examples of (i) the method of subjecting a phenol, naphthol or anthracenol and a corresponding ketone to polycondensation reaction in the presence of an acid catalyst include (a) a method of carrying out the polycondensation reaction in an organic solvent, (b) a method of carrying out the polycondensation reaction in an aqueous solvent, and (c) a method of carrying out the polycondensation reaction with no solvent.

In (i) (a) the method of subjecting a phenol, naphthol or anthracenol and a corresponding ketone to polycondensation reaction in the presence of an acid catalyst in an organic solvent, the compound represented by the above formula (4) can be obtained by subjecting a phenol, naphthol or anthracenol and a corresponding ketone to polycondensation reaction in the presence of an acid catalyst at normal pressure. If necessary, this reaction can also be carried out under increased pressure. In addition, an acid dissociation group can be introduced into at least one phenolic hydroxy group of that compound according to a publicly known method.

In (i) (b) or (c) the method of subjecting a phenol, naphthol or anthracenol and a corresponding ketone to polycondensation reaction in the presence of an acid catalyst in an aqueous solvent or with no solvent, the compound represented by the above formula (4) can be obtained by subjecting a phenol, naphthol or anthracenol and a corresponding ketone to polycondensation reaction in the presence of an acid and a mercapto catalyst. In addition, an acid dissociation group can be introduced into at least one phenolic hydroxy group of that compound according to a publicly known method. Also, the present reaction can be carried out under reduced pressure, at normal pressure, or under increased pressure.

Examples of the above naphthol include, but not particularly limited to, naphthol, methylnaphthol, methoxynaphthol, and naphthalenediol. Naphthalenediol is more preferably used from the viewpoint that a xanthene structure can be easily formed.

Examples of the phenol include, but not particularly limited to, phenol, methylphenol, methoxybenzene, catechol, resorcinol, hydroquinone, and trimethylhydroquinone.

Examples of the above anthracenol include, but not particularly limited to, anthracenol, methylanthracenol, and methoxyanthracenol. These anthracenols can be used alone as one kind or can be used in combination of two or more kinds. Among them, anthracenol is more preferably used from the viewpoint of increasing a carbon atom concentration and improving heat resistance.

Examples of the above ketone include, but not particularly limited to, acetone, methyl ethyl ketone, acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl. These ketones can be used alone as one kind or can be used in combination of two or more kinds. Among them, it is preferable to use acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl from the viewpoint of conferring high heat resistance, and it is more preferable to use acetophenone, diacetylbenzene, triacetylbenzene, acetonaphthone, diphenylcarbonylnaphthalene, phenylcarbonylbiphenyl, diphenylcarbonylbiphenyl, benzophenone, diphenylcarbonylbenzene, triphenylcarbonylbenzene, benzonaphthone, diphenylcarbonylnaphthalene, phenylcarbonylbiphenyl, and diphenylcarbonylbiphenyl from the viewpoint of conferring high etching resistance.

As the ketone, a ketone having an aromatic ring is preferably used from the viewpoint that both high heat resistance and high etching resistance are achieved.

The above acid catalyst is not particularly limited and can be arbitrarily selected from well known inorganic acids, organic acids, Lewis acids, and solid acids. Examples thereof include inorganic acids such as hydrochloric acid, sulfuric acid, phosphoric acid, hydrobromic acid, and hydrofluoric acid; organic acids such as oxalic acid, formic acid, p-toluenesulfonic acid, methanesulfonic acid, trifluoroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Hydrochloric acid or sulfuric acid is preferably used from the viewpoint of production such as easy availability and handleability. The acid catalyst can be used as one kind or two or more kinds. Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

The mercapto catalyst used in the above reaction can be arbitrarily selected for use from publicly known mercapto catalysts and is not particularly limited. As such a catalyst, an alkylthiol and a mercaptocarboxylic acid are widely known. Examples of the alkylthiol include an alkyl mercaptan having 1 to 12 carbon atoms, preferably n-octyl mercaptan, n-decyl mercaptan, and n-dodecyl mercaptan, and examples of the mercaptocarboxylic acid include, but not particularly limited to, 2-mercaptopropionic acid and 3-mercaptopropionic acid. Among them, from the viewpoint of production, n-octyl mercaptan, n-decyl mercaptan, and n-dodecyl mercaptan are preferable. The mercapto catalysts can be used alone as one kind or can be used in combination of two or more kinds. Also, the amount of the mercapto catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials.

Upon producing the compound represented by the above formula (4), a reaction solvent may be used. The reaction solvent is not particularly limited as long as the reaction of the ketone used with the naphthol or the like proceeds. For example, water, methanol, ethanol, propanol, butanol, tetrahydrofuran, dioxane, or a mixed solvent thereof can be used. The amount of the above solvent is not particularly limited and is, for example, in the range of 0 to 2000 parts by mass based on 100 parts by mass of the reaction raw materials.

Upon producing the above polyphenol compound, the reaction temperature is not particularly limited and can be arbitrarily selected according to the reactivity of the reaction raw materials, but is preferably within the range of 10 to 200°C. In order to synthesize the compound represented by the formula (4) of the present embodiment with good selectivity, a lower temperature is more effective, and the range of 10 to 60°C is more preferable.

The method for producing the compound represented by the above formula (4) is not particularly limited, but there are a method of charging the naphthol or the like, the ketone, and the catalyst in one portion, and a method of dropping the naphthol and the ketone, in the presence of the catalyst. After the polycondensation reaction terminates, the temperature of the reaction vessel is elevated to 130 to 230°C in order to remove unreacted raw materials, catalyst, etc. present in the system, and volatile portions can be removed at about 1 to 50 mmHg.

The amounts of the raw materials upon producing the compound represented by the above formula (4) are not particularly limited, but the reaction proceeds, for example, by using 2 mol to an excess of the naphthol or the like and 0.001 to 1 mol of the acid catalyst based on 1 mol of the ketone, and reacting them at 20 to 60°C at normal pressure for about 20 minutes to 100 hours.

Upon producing the compound represented by the above formula (4), the target compound is isolated by a publicly known method after the above reaction terminates. Examples of the method for isolating the target compound include, but not particularly limited to, a method of concentrating the reaction liquid, precipitating the reaction product by the addition of pure water, cooling the reaction liquid to room temperature, then separating the precipitates by filtration, filtering and drying the obtained solid matter, then separating and purifying the solid matter from by-products by column chromatography, and distilling off the solvent, followed by filtration and drying to obtain the target compound.

In (ii) the method of subjecting a methine site of a triarylmethane obtained by subjecting a phenol, naphthol or anthracenol and a corresponding aldehyde to polycondensation in the presence of an acid catalyst, or a xanthene to substitution, a compound (A') formed by replacing R^{Y} of the compound represented by the above formula (4) with a hydrogen atom is obtained by subjecting a phenol, naphthol or anthracenol and a corresponding aldehyde to polycondensation reaction in the presence of an acid catalyst. Using a protective group introducing agent, a compound (B') is obtained in which a hydroxy group of the compound (A') is substituted with a protective group. Then, by allowing the hydrogen atom corresponding to the R^{Y} moiety of the compound represented by the above formula (4) to react with an alkylating agent in the presence of a basic catalyst, an alkyl group corresponding to the R^{Y} moiety of the compound represented by the above formula (4) is introduced. Further later, the protective group that has substituted the hydroxy group in the above compound (B') is deprotected, thereby obtaining the above formula (4). In addition, an acid dissociation group can be introduced into at least one phenolic hydroxy group of that compound according to a publicly known method. If necessary, this reaction can also be carried out under increased pressure. The above alkylating agent can be arbitrarily selected for use from publicly known alkylating agents and is not particularly limited. Examples thereof include alkyl chloride, alkyl bromide, and alkyl iodide.

In the above production method, for the method of introducing an alkyl group corresponding to the R^{Y} moiety of the compound represented by the above formula (4) into the hydrogen atom of the compound (B') corresponding to the R^{Y} moiety of the compound represented by the above formula (4), instead of allowing the hydrogen atom to react with an alkylating agent in the presence of a basic catalyst as in the above production method, the compound (B') may also be allowed to react with a halogenating agent to substitute the hydrogen atom corresponding to the R^{Y} moiety of the compound represented by the above formula (4) with a halogen atom, and then allowed to react with an alkylating agent, thereby obtaining the above formula (4). The alkylating agent can be arbitrarily selected for use from publicly known alkylating agents and is not particularly limited. Examples thereof include a Grignard reagent and an alkyllithium.

Examples of the above phenol include, but not particularly limited to, phenol, methylphenol, and methoxyphenol. These phenols can be used alone as one kind or can be used in combination of two or more kinds. Among them, phenol is more preferably used from the viewpoint of the stable supply of raw materials.

Examples of the above naphthol include, but not particularly limited to, naphthol, methylnaphthol, and methoxynaphthol. These naphthols can be used alone as one kind or can be used in combination of two or more kinds. Among them, naphthol is more preferably used from the viewpoint of increasing a carbon atom concentration and improving heat resistance.

Examples of the above anthracenol include, but not particularly limited to, anthracenol, methylanthracenol, and methoxyanthracenol. These anthracenols can be used alone as one kind or can be used in combination of two or more kinds. Among them, anthracenol is more preferably used from the viewpoint of increasing a carbon atom concentration and improving heat resistance.

Examples of the above aldehyde include, but not particularly limited to, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, and furfural.

The method for introducing an acid dissociation group to at least one phenolic hydroxy group of the polyphenol compound is publicly known. For example, an acid dissociation group can be introduced to at least one phenolic hydroxy group of the above compound, as follows. A compound for introducing an acid dissociation group can be synthesized by a publicly known method or easily obtained. Examples thereof include, but not particularly limited to, acid chlorides, acid anhydrides, active carboxylic acid derivative compounds such as dicarbonates, alkyl halides, vinyl alkyl ethers, dihydropyran, and halocarboxylic acid alkyl esters.

For example, the above compound is dissolved or suspended in an aprotic solvent such as acetone, tetrahydrofuran (THF), or propylene glycol monomethyl ether acetate. Subsequently, a vinyl alkyl ether such as ethyl vinyl ether, or dihydropyran is added to the solution or the suspension, and the mixture is reacted at 20 to 60°C at normal pressure for 6 to 72 hours in the presence of an acid catalyst such as pyridinium p-toluenesulfonate. The reaction liquid is neutralized with an alkali compound and added to distilled water to precipitate a white solid. Then, the separated white solid can be washed with distilled water and dried to obtain a compound in which a hydrogen atom of a hydroxy group is replaced with an acid dissociation group.

Alternatively, for example, the above compound having a hydroxy group is dissolved or suspended in an aprotic solvent such as acetone, THF, or propylene glycol monomethyl ether acetate. Subsequently, an alkyl halide such as ethyl chloromethyl ether or a halocarboxylic acid alkyl ester such as methyladamantyl bromoacetate is added to the solution or the suspension, and the mixture is reacted at 20 to 110°C at normal pressure for 6 to 72 hours in the presence of an alkali catalyst such as potassium carbonate. The reaction liquid is neutralized with an acid such as hydrochloric acid and added to distilled water to precipitate a white solid. Then, the separated white solid can be washed with distilled water and dried to obtain a compound in which a hydrogen atom of a hydroxy group is replaced with an acid dissociation group.

The timing of introducing an acid dissociation group may be not only after condensation reaction of the naphthol with the ketone but at a stage previous to the condensation reaction. Alternatively, the introduction may be carried out after production of a resin, which will be mentioned later.

In the present embodiment, the acid dissociation group refers to a characteristic group that is cleaved in the presence of an acid to generate a functional group that alters solubility, such as an alkali soluble group. Examples of the alkali soluble group include a phenolic hydroxy group, a carboxyl group, a sulfonic acid group, and a hexafluoroisopropanol group. A phenolic hydroxy group and a carboxyl group are preferable, and a phenolic hydroxy group is particularly preferable. The above acid dissociation group preferably has the property of causing chained cleavage reaction in the presence of an acid, for achieving pattern formation with much higher sensitivity and higher resolution.

### [Resin comprising compound represented by formula (1) as constituent unit]

The compound represented by the above formula (1) can be used as is in a composition for use in film formation for lithography or optical component formation (hereinafter, also simply referred to as a "composition"). Also, a resin comprising the compound represented by the above formula (1) as a constituent unit can be used in the composition. The resin is obtained, for example, by reacting the compound represented by the above formula (1) with a crosslinking compound.

Examples of the resin comprising the compound represented by the above formula (1) as a constituent unit include a resin having a structure represented by the following formula (5). That is, the composition of the present embodiment may contain a resin having a structure represented by the following formula (5). 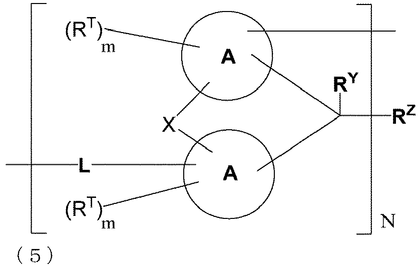 (In the formula (5),
R^{Y}, R^{Z}, A, R^{T}, X, and N are as defined in the formula (1) ;
each m is independently an integer of 0 to 8; and
L is an alkylene group having 1 to 30 carbon atoms and optionally having a substituent and/or a heteroatom, an arylene group having 6 to 30 carbon atoms and optionally having a substituent and/or a heteroatom, an alkoxylene group having 1 to 30 carbon atoms and optionally having a substituent and/or a heteroatom, or a single bond, wherein the alkylene group, the arylene group and the alkoxylene group each optionally contain an ether bond, a thioether bond, a ketone bond or an ester bond;
wherein, at least one selected from A, R^{Y}, R^{Z}, R^{T}, X, and L contains a sulfur atom; and
at least one R^{T} contains a thiol group, a hydroxy group, or a group in which a hydrogen atom of a thiol group or a hydroxy group is replaced with an acid crosslinking group or an acid dissociation group.

Examples of the substituent for the alkylene group, the arylene group, and the alkoxylene group can include, as described above, those exemplified as the substituent for the alkyl group, the aryl group, and the alkoxy group, respectively.

### [Resin comprising compound represented by formula (3) as constituent unit]

The compound represented by the above formula (3) can be used as is in a film forming composition for lithography. Also, a resin comprising the compound represented by the above formula (3) as a constituent unit can be used in the composition. For example, a resin obtained by reacting the compound represented by the above formula (3) with a crosslinking compound can also be used.

Examples of the resin comprising the compound represented by the above formula (3) as a constituent unit include a resin having a structure represented by the following formula (6). That is, the film forming composition for lithography of the present embodiment may contain a resin having a structure represented by the following formula (6).

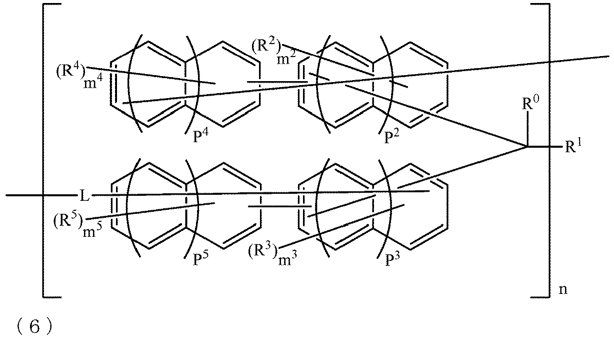

In the formula (6), L is a linear or branched alkylene group having 1 to 30 carbon atoms and optionally having a substituent, or a single bond. R⁰, R¹, R² to R⁵, m² to m⁵, p² to p⁵, and n are as defined in the above formula (3), provided that m² to m⁵ are not 0 at the same time and at least one of R² to R⁵ is a thiol group.

### [Resin comprising compound represented by formula (4) as constituent unit]

The compound represented by the above formula (4) can be used as is in a film forming composition for lithography. Also, a resin comprising the compound represented by the above formula (4) as a constituent unit can be used in the composition. For example, a resin obtained by reacting the compound represented by the above formula (4) with a crosslinking compound can also be used.

Examples of the resin comprising the compound represented by the above formula (4) as a constituent unit include a resin having a structure represented by the following formula (7). That is, the film forming composition for lithography of the present embodiment may contain a resin having a structure represented by the following formula (7).

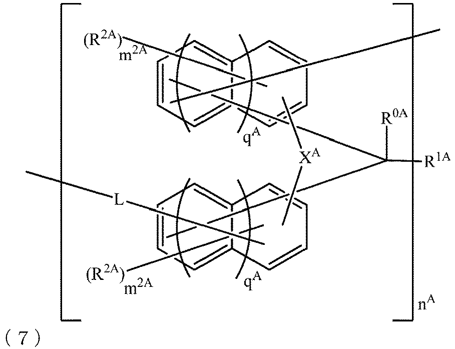

In the formula (7), L is a linear or branched alkylene group having 1 to 30 carbon atoms and optionally having a substituent, or a single bond.

R^{0A}, R^{1A}, R^{2A}, m^{2A}, n^{A}, q^{A}, and X^{A} are as defined in the above formula (4).

When n^{A} is an integer of 2 or larger, n^{A} structural formulas within the parentheses [ ] are the same or different.

However, at least one R^{2A} is a thiol group.

### [Method for producing resin comprising compound of present embodiment as constituent unit]

The resin according to the present embodiment is obtained by reacting the compound of the present embodiment with a crosslinking compound. As the crosslinking compound, those that are publicly known can be used without particular limitations as long as they can oligomerize or polymerize the compound of the present embodiment. Specific examples thereof include, but not particularly limited to, aldehydes, ketones, carboxylic acids, carboxylic acid halides, halogen-containing compounds, amino compounds, imino compounds, isocyanates, and unsaturated hydrocarbon group-containing compounds.

Specific examples of the resin of the present embodiment include a resin that is made novolac by, for example, a condensation reaction between the compound represented by the above formula of the present embodiment and an aldehyde and/or ketone that is a crosslinking compound.

Herein, examples of the aldehyde used upon making the compound of the present embodiment novolac include, but not particularly limited to, formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, furfural, methylthiobenzaldehyde, thiophene carboxaldehyde, methylthiothiophene carboxaldehyde, formyltetrathiafulvalene, and benzothiophene carboxaldehyde. From the viewpoint of improving the refractive index, methylthiobenzaldehyde, thiophene carboxaldehyde, methylthiothiophene carboxaldehyde, formyltetrathiafulvalene, and benzothiophene carboxaldehyde, to all of which a sulfur atom is introduced, are preferable. Examples of the ketone include the ketones listed above. From the viewpoint of improving the refractive index, mercaptobenzonic acid, methylthioacetophenone, acetylthiophene, and acetylbenzothiophene, to all of which a sulfur atom is introduced, are further preferable. Among them, formaldehyde is preferable from the viewpoint of productivity.

These aldehydes and/or ketones can be used alone as one kind or may be used in combination of two or more kinds. In addition, the amount of the above aldehyde and/or ketone used is not particularly limited, but is preferably 0.2 to 5 mol and more preferably 0.5 to 2 mol based on 1 mol of the compound of the present embodiment.

An acid catalyst can also be used in the condensation reaction between the compound of the present embodiment and the aldehyde and/or ketone. The acid catalyst used herein can be arbitrarily selected for use from publicly known acid catalysts and is not particularly limited. Inorganic acids, organic acids, Lewis acids, solid acids, and the like are widely known as such acid catalysts, and examples thereof include, but not particularly limited to, inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, organic acids and solid acids are preferable from the viewpoint of production, and hydrochloric acid or sulfuric acid is preferable from the viewpoint of production such as easy availability and handleability. The acid catalysts can be used alone as one kind or can be used in combination of two or more kinds.

Also, the amount of the acid catalyst used can be arbitrarily set according to, for example, the kind of the raw materials used and the catalyst used and moreover the reaction conditions and is not particularly limited, but is preferably 0.01 to 100 parts by mass based on 100 parts by mass of the reaction raw materials. However, the aldehyde is not necessarily needed in the case of a copolymerization reaction with a compound having a nonconjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborn-2-ene, α-pinene, β-pinene, and limonene.

A reaction solvent can also be used in the condensation reaction between the compound of the present embodiment and the aldehyde and/or ketone. The reaction solvent in this polycondensation can be arbitrarily selected for use from publicly known solvents and is not particularly limited, and examples thereof include water, methanol, ethanol, propanol, butanol, tetrahydrofuran, dioxane, and a mixed solvent thereof. The solvents can be used alone as one kind or can be used in combination of two or more kinds.

Also, the amount of these solvents used can be arbitrarily set according to, for example, the kind of the raw materials used and the catalyst used and moreover the reaction conditions and is not particularly limited, but is preferably in the range of 0 to 2000 parts by mass based on 100 parts by mass of the reaction raw materials. Furthermore, the reaction temperature can be arbitrarily selected according to the reactivity of the reaction raw materials and is not particularly limited, but is usually within the range of 10 to 200°C. The reaction method can be arbitrarily selected for use from publicly known approaches and is not particularly limited, and examples thereof include a method of charging the compound of the present embodiment, the aldehyde and/or ketone, and the catalyst in one portion, and a method of dropping the compound of the present embodiment and the aldehyde and/or ketone in the presence of the catalyst.

After the polycondensation reaction terminates, isolation of the obtained resin can be carried out according to a conventional method, and is not particularly limited. For example, by adopting a commonly used approach in which the temperature of the reaction vessel is elevated to 130 to 230°C in order to remove unreacted raw materials, catalyst, etc. present in the system, and volatile portions are removed at about 1 to 50 mmHg, a novolac resin that is the target compound can be isolated.

Herein, the resin of the present embodiment may be a homopolymer of the compound of the present embodiment, or may be a copolymer with a further phenol. Herein, examples of the copolymerizable phenol include, but not particularly limited to, phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol, thymol, and biphenol.

The resin of the present embodiment may be a copolymer with a polymerizable monomer other than the above-described further phenols. Examples of such a copolymerization monomer include, but not particularly limited to, naphthol, methylnaphthol, methoxynaphthol, dihydroxynaphthalene, naphthalenetriol, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornene, pinene, and limonene. The resin of the present embodiment may be a copolymer of two or more components (for example, a binary to quaternary system) composed of the compound of the present embodiment and the above-described phenol, may be a copolymer of two or more components (for example, a binary to quaternary system) composed of the compound of the present embodiment and the above-described copolymerization monomer, or may be a copolymer of three or more components (for example, a tertiary to quaternary system) composed of the compound of the present embodiment, the above-described phenol, and the above-described copolymerization monomer.

The molecular weight of the resin of the present embodiment is not particularly limited, and the weight average molecular weight (Mw) in terms of polystyrene is preferably 500 to 30,000 and more preferably 750 to 20,000. The resin of the present embodiment preferably has dispersibility (weight average molecular weight Mw/number average molecular weight Mn) within the range of 1 to 7 from the viewpoint of enhancing crosslinking efficiency while suppressing volatile components during baking. The above Mw and Mn can be determined by a method described in Examples mentioned later.

The resin of the present embodiment preferably has high solubility in a solvent from the viewpoint of easier application to a wet process, etc. More specifically, in the case of using 1-methoxy-2-propanol (PGME) and/or propylene glycol monomethyl ether acetate (PGMEA) as a solvent, the resin preferably has a solubility of 10% by mass or more in the solvent. Herein, the solubility in PGME and/or PGMEA is defined as "mass of the resin / (mass of the resin + mass of the solvent) × 100 (% by mass)". For example, when 10 g of the resin is dissolved in 90 g of PGMEA, the solubility of the resin in PGMEA is "10% by mass or more"; and when 10 g of the resin is not dissolved in 90 g of PGMEA, the solubility is "less than 10% by mass".

The resin represented by the above formula (5) is preferably a compound represented by the following formula (5-1) from the viewpoint of solubility in an organic solvent.

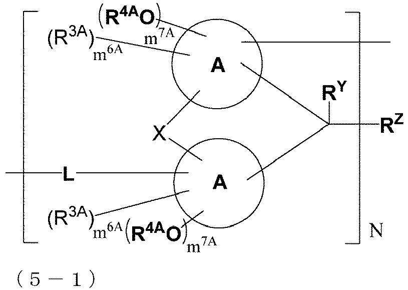

In the formula (5-1),
R^{Y}, R^{Z}, A, X, and N are as defined in the formula (1) ;
R^{3A} and R^{4A} are as defined in the formula (1-1) ; and
L is as defined in the formula (5);
wherein, at least one selected from A, R^{Y}, R^{Z}, R^{3A}, R^{4A}, X, and L contains a sulfur atom;
each m^{6A} is independently an integer of 0 to 5; and
each m^{7A} is independently an integer of 0 to 5.

### [Method for purifying compound and/or resin]

The method for purifying the compound and/or the resin according to the present embodiment comprises the steps of: obtaining a solution (S) by dissolving the compound and/or the resin of the present embodiment in a solvent; and extracting impurities in the compound and/or the resin by bringing the obtained solution (S) into contact with an acidic aqueous solution (a first extraction step), wherein the solvent used in the step of obtaining the solution (S) contains a solvent that does not inadvertently mix with water.

In the first extraction step, the above resin is preferably a resin obtained by a reaction between the compound of the present embodiment and a crosslinking compound. According to the purification method of the present embodiment, the contents of various metals that may be contained as impurities in the compound or the resin having a specific structure described above can be reduced.

More specifically, in the purification method of the present embodiment, the compound and/or the resin is dissolved in an organic solvent that does not inadvertently mix with water to obtain the solution (S), and further, extraction treatment can be carried out by bringing the solution (S) into contact with an acidic aqueous solution. Thereby, metals contained in the solution (S) are transferred to the aqueous phase, then the organic phase and the aqueous phase are separated, and thus the compound and/or the resin having a reduced metal content can be obtained.

The compound and/or the resin used in the purification method of the present embodiment may be alone, or may be a mixture of two or more kinds. Also, the compound and/or the resin may contain various surfactants, various acid crosslinking agents, various acid generating agents, various stabilizers, and the like.

The solvent that does not inadvertently mix with water used in the purification method of the present embodiment is not particularly limited, but is preferably an organic solvent that is safely applicable to semiconductor manufacturing processes, and specifically it is an organic solvent having a solubility in water at room temperature of less than 30%, more preferably less than 20%, and still more preferably less than 10%. The amount of the organic solvent used is preferably 1 to 100 times the total mass of the compound and/or the resin to be used.

Specific examples of the solvent that does not inadvertently mix with water include those described in International Publication No. WO 2015/080240. Among these, toluene, 2-heptanone, cyclohexanone, cyclopentanone, methyl isobutyl ketone, propylene glycol monomethyl ether acetate, and ethyl acetate are preferable, methyl isobutyl ketone, ethyl acetate, cyclohexanone, and propylene glycol monomethyl ether acetate are more preferable, and methyl isobutyl ketone and ethyl acetate are still more preferable. Methyl isobutyl ketone, ethyl acetate, and the like have relatively high saturation solubility for the above compound and the resin comprising the compound as a constituent and a relatively low boiling point, and it is thus possible to reduce the load in the case of industrially distilling off the solvent and in the step of removing the solvent by drying. These solvents can be each used alone, and can be used as a mixture of two or more kinds.

The acidic aqueous solution used in the purification method of the present embodiment is arbitrarily selected from among aqueous solutions in which organic compounds or inorganic compounds are dissolved in water, generally known as acidic aqueous solutions. For example, examples thereof include those described in International Publication No. WO 2015/080240. These acidic aqueous solutions can be each used alone, and can be also used as a combination of two or more kinds. Among these acidic aqueous solutions, aqueous solutions of one or more mineral acids selected from the group consisting of hydrochloric acid, sulfuric acid, nitric acid, and phosphoric acid, or aqueous solutions of one or more organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are preferable, aqueous solutions of sulfuric acid, nitric acid, and carboxylic acids such as acetic acid, oxalic acid, tartaric acid, and citric acid are more preferable, aqueous solutions of sulfuric acid, oxalic acid, tartaric acid, and citric acid are still more preferable, and an aqueous solution of oxalic acid is further preferable. It is considered that polyvalent carboxylic acids such as oxalic acid, tartaric acid, and citric acid coordinate with metal ions and provide a chelating effect, and thus tend to be capable of more effectively removing metals. As for water used herein, preferable is water, the metal content of which is small, such as ion exchanged water, according to the purpose of the purification method of the present embodiment.

The pH of the acidic aqueous solution used in the purification method of the present embodiment is not particularly limited, but it is preferable to regulate the pH in consideration of an influence on the above compound or resin. Normally, the pH of the acidic aqueous solution is about 0 to 5, and is preferably about pH 0 to 3.

The amount of the acidic aqueous solution used in the purification method of the present embodiment is not particularly limited, but it is preferable to regulate the amount from the viewpoint of reducing the number of extraction operations for removing metals and from the viewpoint of ensuring operability in consideration of the overall amount of fluid. From the above viewpoints, the amount of the acidic aqueous solution used is preferably 10 to 200% by mass, more preferably 20 to 100% by mass, based on 100% by mass of the solution (S).

In the purification method of the present embodiment, by bringing the acidic aqueous solution into contact with the solution (S), metals can be extracted from the compound or the resin in the solution (S).

In the purification method of the present embodiment, it is preferable that the solution (S) further contain an organic solvent that inadvertently mixes with water. When the solution (S) contains an organic solvent that inadvertently mixes with water, there is a tendency that the amount of the compound and/or the resin charged can be increased, also the fluid separability is improved, and purification can be carried out at a high reaction vessel efficiency. The method for adding the organic solvent that inadvertently mixes with water is not particularly limited and may be, for example, any of a method involving adding it to the organic solvent-containing solution in advance, a method involving adding it to water or the acidic aqueous solution in advance, and a method involving adding it after bringing the organic solvent-containing solution into contact with water or the acidic aqueous solution. Among these, the method involving adding it to the organic solvent-containing solution in advance is preferable from the viewpoint of the workability of operations and the ease of managing the amount.

The organic solvent that inadvertently mixes with water used in the purification method of the present embodiment is not particularly limited, but is preferably an organic solvent that is safely applicable to semiconductor manufacturing processes. The amount of the organic solvent used that inadvertently mixes with water is not particularly limited as long as the solution phase and the aqueous phase separate, but is preferably 0.1 to 100 times, more preferably 0.1 to 50 times, and further preferably 0.1 to 20 times the total mass of the compound and the resin to be used.

Specific examples of the organic solvent used in the purification method of the present embodiment that inadvertently mixes with water include those described in International Publication No. WO 2015/080240. Among these, N-methylpyrrolidone, propylene glycol monomethyl ether, and the like are preferable, and N-methylpyrrolidone and propylene glycol monomethyl ether are more preferable. These solvents can be each used alone, and can be used as a mixture of two or more kinds.

The temperature when extraction treatment is carried out is generally in the range of 20 to 90°C, and preferably 30 to 80°C. The extraction operation is carried out, for example, by thoroughly mixing the solution (S) and the acidic aqueous solution by stirring or the like and then leaving the obtained mixed solution to stand still. Thereby, metals contained in the solution (S) are transferred to the aqueous phase. Also, by this operation, the acidity of the solution is lowered, and the degradation of the compound and/or the resin can be suppressed.

By being left to stand still, the mixed solution is separated into an aqueous phase and a solution phase containing the compound and/or the resin and the organic solvents, and thus the solution phase is recovered by decantation. The time to stand still is not particularly limited, but it is preferable to regulate the time to stand still from the viewpoint of attaining good separation of the solution phase containing the organic solvents and the aqueous phase. Normally, the time to stand still is 1 minute or longer, preferably 10 minutes or longer, and more preferably 30 minutes or longer. While the extraction treatment may be carried out once, it is effective to repeat mixing, leaving-to-stand-still, and separating operations multiple times.

It is preferable that the purification method of the present embodiment include the step of extracting impurities in the compound or the resin by further bringing the solution phase containing the compound or the resin into contact with water after the first extraction step (the second extraction step). Specifically, for example, it is preferable that after the above first extraction step is carried out using an acidic aqueous solution, the recovered solution phase that contains the compound and/or the resin and the organic solvents be further subjected to extraction treatment with water. The extraction treatment with water is not particularly limited, and can be carried out, for example, by thoroughly mixing the solution phase and water by stirring or the like and then leaving the obtained mixed solution to stand still. The mixed solution after being left to stand still is separated into an aqueous phase and a solution phase containing the compound and/or the resin and the organic solvents, and thus the solution phase can be recovered by decantation.

As for water used herein, preferable is water, the metal content of which is small, such as ion exchanged water, according to the purpose of the present embodiment. While the extraction treatment may be carried out once, it is effective to repeat mixing, leaving-to-stand-still, and separating operations multiple times. The proportions of both used in the extraction treatment and temperature, time, and other conditions are not particularly limited, and may be the same as those of the previous contact treatment with the acidic aqueous solution.

Water that is possibly present in the thus-obtained solution containing the compound and/or the resin can be easily removed by performing vacuum distillation or a like operation. Also, if required, the concentration of the compound and/or the resin can be regulated to be any concentration by adding a solvent to the solution.

The method for isolating the compound and/or the resin from the obtained solution containing the compound and/or the resin and the solvents is not particularly limited, and publicly known methods can be carried out, such as reduced-pressure removal, separation by reprecipitation, and a combination thereof. Publicly known treatments such as concentration operation, filtration operation, centrifugation operation, and drying operation can be carried out if required.

### [Film forming composition for lithography]

The film forming composition for lithography according to the present embodiment contains the compound and/or the resin of the present embodiment.

### [Film forming composition for lithography for chemical amplification type resist purposes]

The film forming composition for lithography for chemical amplification type resist purposes according to the present embodiment (hereinafter, also referred to as a "resist composition") contains the compound and/or the resin of the present embodiment as a resist base material.

It is preferable that the resist composition of the present embodiment should contain a solvent. Examples of the solvent can include, but not particularly limited to, ethylene glycol monoalkyl ether acetates such as ethylene glycol monomethyl ether acetate, ethylene glycol monoethyl ether acetate, ethylene glycol mono-n-propyl ether acetate, and ethylene glycol mono-n-butyl ether acetate; ethylene glycol monoalkyl ethers such as ethylene glycol monomethyl ether and ethylene glycol monoethyl ether; propylene glycol monoalkyl ether acetates such as propylene glycol monomethyl ether acetate (PGMEA), propylene glycol monoethyl ether acetate, propylene glycol mono-n-propyl ether acetate, and propylene glycol mono-n-butyl ether acetate; propylene glycol monoalkyl ethers such as propylene glycol monomethyl ether (PGME) and propylene glycol monoethyl ether; ester lactates such as methyl lactate, ethyl lactate, n-propyl lactate, n-butyl lactate, and n-amyl lactate; aliphatic carboxylic acid esters such as methyl acetate, ethyl acetate, n-propyl acetate, n-butyl acetate, n-amyl acetate, n-hexyl acetate, methyl propionate, and ethyl propionate; other esters such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutylacetate, 3-methyl-3-methoxybutylacetate, butyl 3-methoxy-3-methylpropionate, butyl 3-methoxy-3-methylbutyrate, methyl acetoacetate, methyl pyruvate, and ethyl pyruvate; aromatic hydrocarbons such as toluene and xylene; ketones such as 2-heptanone, 3-heptanone, 4-heptanone, cyclopentanone (CPN), and cyclohexanone (CHN); amides such as N,N-dimethylformamide, N-methylacetamide, N,N-dimethylacetamide, and N-methylpyrrolidone; and lactones such as γ-lactone. These solvents may be used alone or in combination of two or more kinds.

The solvent used in the present embodiment is preferably a safe solvent, more preferably at least one selected from PGMEA, PGME, CHN, CPN, 2-heptanone, anisole, butyl acetate, ethyl propionate, and ethyl lactate, and still more preferably at least one selected from PGMEA, PGME, and CHN.

In the present embodiment, the amounts of the solid component and the solvent are not particularly limited, but preferably the solid component is 1 to 80% by mass and the solvent is 20 to 99% by mass, more preferably the solid component is 1 to 50% by mass and the solvent is 50 to 99% by mass, still more preferably the solid component is 2 to 40% by mass and the solvent is 60 to 98% by mass, and particularly preferably the solid component is 2 to 10% by mass and the solvent is 90 to 98% by mass, based on 100% by mass of the total mass of the solid component and the solvent.

The resist composition of the present embodiment may contain at least one selected from the group consisting of an acid generating agent (C), an acid crosslinking agent (G), an acid diffusion controlling agent (E), and a further component (F), as other solid components. In the present specification, "the solid components" refer to components except for the solvent.

Herein, as the acid generating agent (C), the acid crosslinking agent (G), the acid diffusion controlling agent (E), and the further component (F), publicly known agents can be used, and they are not particularly limited, but those described in International Publication No. WO 2013/024778 are preferable.

In addition, as the acid crosslinking agent (G), it is also preferable to contain a compound that reacts with a thiol group, and examples of the compound that reacts with a thiol group can include, but not particularly limited to, an epoxy group containing compound, an episulfide group containing compound, and an isocyanate group containing compound. These compounds can be used alone or in mixture of two or more kinds.

As the epoxy group containing compound, without limitations, a bisphenol A-based epoxy resin, a bisphenol F-based epoxy resin, a bisphenol S-based epoxy resin and an alicyclic epoxy resin, a phenol novolac-based epoxy resin, a heterocycle containing epoxy resin, a hydrogenated bisphenol A-based epoxy resin, a hydrogenated bisphenol F-based epoxy resin, an aliphatic epoxy resin and a spiro ring containing epoxy resin, and the like can be used.

The episulfide group containing compound is formed by replacing the oxygen atom of an epoxy compound with a sulfur atom, and can be obtained, for example, by allowing the epoxy compound described above to react with a thiocyanate or a thiourea, which are sulfurizing agents.

As the isocyanate group containing compound, a compound that is generally used for polyurethane, polyisocyanurate, or the like can be used. Examples of such a polyisocyanate include, but not limited to, an aromatic polyisocyanate, an aliphatic polyisocyanate, an alicyclic polyisocyanate, and a modified product thereof, and an isocyanate group-capped prepolymer.

### [Content ratio of each component]

In the resist composition of the present embodiment, the content of the compound and/or the resin used as a resist base material is not particularly limited, but is preferably 1 to 100% of the total mass of the solid components (summation of solid components including the resist base material, and optionally used components such as acid generating agent (C), acid crosslinking agent (G), acid diffusion controlling agent (E), and further component (F), hereinafter the same), more preferably 50 to 99.4% by mass, further preferably 55 to 90% by mass, still more preferably 60 to 80% by mass, and particularly preferably 60 to 70% by mass. When the content of the compound and/or the resin used as a resist base material falls within the above range, there is a tendency that resolution is further improved, and line edge roughness (LER) is further decreased.

When both of the compound and the resin are contained as a resist base material, the above content refers to the total amount of these components.

### [Further component (F)]

To the resist composition of the present embodiment, if required, as a component other than the resist base material, the acid generating agent (C), the acid crosslinking agent (G) and the acid diffusion controlling agent (E), one kind or two kinds or more of various additive agents such as a dissolution promoting agent, dissolution controlling agent, sensitizing agent, surfactant, organic carboxylic acid or oxo acid of phosphor or derivative thereof, thermal and/or light curing catalyst, polymerization inhibitor, flame retardant, filler, coupling agent, thermosetting resin, light curable resin, dye, pigment, thickener, lubricant, antifoaming agent, leveling agent, ultraviolet absorber, surfactant, colorant, and nonionic surfactant can be added within the range not inhibiting the objects of the present invention. In the present specification, the further component (F) is also referred to as an optional component (F).

In the resist composition of the present embodiment, the contents of the resist base material (hereinafter, also referred to as a "component (A)"), the acid generating agent (C), the acid crosslinking agent (G), the acid diffusion controlling agent (E), and the optional component (F) (the component (A)/the acid generating agent (C)/the acid crosslinking agent (G)/the acid diffusion controlling agent (E)/the optional component (F)) are, in % by mass based on the solid content,
preferably 1 to 100/0 to 49/0 to 49/0 to 49/0 to 99,
more preferably 50 to 99.4/0.001 to 49/0.5 to 49/0.001 to 49/0 to 49,
further preferably 55 to 90/1 to 40/0.5 to 40/0.01 to 10/0 to 5,
further preferably 60 to 80/3 to 30/1 to 30/0.01 to 5/0 to 1, and
particularly preferably 60 to 70/10 to 25/2 to 20/0.01 to 3/0.

The content ratio of each component is selected from each range so that the summation thereof is 100% by mass. When the content ratio of each component falls within the above range, performance such as sensitivity, resolution, and developability tends to be excellent.

The resist composition of the present embodiment is generally prepared by dissolving each component in a solvent upon use into a homogeneous solution, and then if required, filtering through a filter or the like with a pore diameter of about 0.2 µm, for example.

The resist composition of the present embodiment can contain an additional resin other than the resin of the present embodiment, within the range not inhibiting the objects of the present invention. Examples of the additional resin include, but not particularly limited to, a novolac resin, a polyvinyl phenol, a polyacrylic acid, an epoxy resin, a polyvinyl alcohol, a styrene-maleic anhydride resin, and a polymer containing an acrylic acid, vinyl alcohol, vinylphenol or maleimide compound as a monomeric unit, and a derivative thereof. The content of the additional resin is not particularly limited and is arbitrarily adjusted according to the kind of the component (A) to be used, and is preferably 30 parts by mass or less per 100 parts by mass of the component (A), more preferably 10 parts by mass or less, still more preferably 5 parts by mass or less, and particularly preferably 0 part by mass.

### [Physical properties and the like of resist composition]

The resist composition of the present embodiment can be used to form an amorphous film by spin coating. Also, the resist composition of the present embodiment can be applied to a general semiconductor production process. Any of positive type and negative type resist patterns can be individually prepared depending on the type of the compound and/or the resin of the present embodiment, and/or the kind of a developing solution to be used.

In the case of a positive type resist pattern, the dissolution rate of the amorphous film formed by spin coating with the resist composition of the present embodiment in a developing solution at 23°C is preferably 5 angstrom/sec or less, more preferably 0.05 to 5 angstrom/sec, and further preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound and/or the resin of the present embodiment, contrast at the interface between the exposed portion being dissolved in a developing solution and the unexposed portion not being dissolved in a developing solution is increased. Also, effects of reducing LER and defects are seen.

In the case of a negative type resist pattern, the dissolution rate of the amorphous film formed by spin coating with the resist composition of the present embodiment in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound and/or the resin of the present embodiment dissolves, and LER is reduced. Also, effects of reducing defects are seen.

The above dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound and/or the resin of the present embodiment dissolves, and LER is reduced. Also, effects of reducing defects are seen.

In the case of a negative type resist pattern, the dissolution rate of the portion exposed by radiation such as KrF excimer laser, extreme ultraviolet, electron beam or X-ray, of the amorphous film formed by spin coating with the resist composition of the present embodiment, in a developing solution at 23°C is preferably 5 angstrom/sec or less, more preferably 0.05 to 5 angstrom/sec, and further preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound and/or the resin of the present embodiment, contrast at the interface between the unexposed portion being dissolved in a developing solution and the exposed portion not being dissolved in a developing solution is increased. Also, effects of reducing LER and defects are seen.

### [Film forming composition for lithography for non-chemical amplification type resist purposes]

The component (A) to be contained in the film forming composition for lithography for non-chemical amplification type resist purposes (hereinafter, also referred to as a "radiation-sensitive composition") of the present embodiment is used in combination with the optically active diazonaphthoquinone compound (B) mentioned later and is useful as a base material for positive type resists that becomes a compound easily soluble in a developing solution by irradiation with g-ray, h-ray, i-ray, KrF excimer laser, ArF excimer laser, extreme ultraviolet, electron beam, or X-ray. Although the properties of the component (A) are not largely altered by g-ray, h-ray, i-ray, KrF excimer laser, ArF excimer laser, extreme ultraviolet, electron beam, or X-ray, the optically active diazonaphthoquinone compound (B) poorly soluble in a developing solution is converted to an easily soluble compound, and a resist pattern can therefore be formed in a development step.

Since the component (A) to be contained in the radiation-sensitive composition of the present embodiment is a relatively low molecular weight compound, the obtained resist pattern has very small roughness. In addition, in the above formula (1), it is preferable that at least one selected from the group consisting of R^{T}, A, R^{Y}, and R^{Z} be a group containing an iodine atom. When the radiation-sensitive composition of the present embodiment contains the component (A) containing an iodine atom, the ability to absorb radiation such as electron beam, extreme ultraviolet (EUV), or X-ray is increased, and as a result, the sensitivity improves.

The glass transition temperature of the component (A) to be contained in the radiation-sensitive composition of the present embodiment is preferably 100°C or higher, more preferably 120°C or higher, still more preferably 140°C or higher, and particularly preferably 150°C or higher. The upper limit of the glass transition temperature of the component (A) is not particularly limited and is, for example, 400°C. When the glass transition temperature of the component (A) falls within the above range, there is a tendency that the resulting radiation-sensitive composition has heat resistance capable of maintaining a pattern shape in a semiconductor lithography process, and improves performance such as high resolution.

The heat of crystallization determined by the differential scanning calorimetry of the glass transition temperature of the component (A) to be contained in the radiation-sensitive composition of the present embodiment is preferably less than 20 J/g. (Crystallization temperature) - (Glass transition temperature) is preferably 70°C or more, more preferably 80°C or more, still more preferably 100°C or more, and particularly preferably 130°C or more. When the heat of crystallization is less than 20 J/g or (Crystallization temperature) - (Glass transition temperature) falls within the above range, there is a tendency that the radiation-sensitive composition easily forms an amorphous film by spin coating, can maintain film formability necessary for a resist over a long period, and can improve resolution.

In the present embodiment, the above heat of crystallization, crystallization temperature, and glass transition temperature can be determined by differential scanning calorimetry using "DSC/TA-50WS" manufactured by Shimadzu Corp. For example, about 10 mg of a sample is placed in an unsealed container made of aluminum, and the temperature is raised to the melting point or more at a temperature increase rate of 20°C/min in a nitrogen gas stream (50 mL/min). After quenching, again the temperature is raised to the melting point or more at a temperature increase rate of 20°C/min in a nitrogen gas stream (30 mL/min). After further quenching, again the temperature is raised to 400°C at a temperature increase rate of 20°C/min in a nitrogen gas stream (30 mL/min). The temperature at the middle point (where the specific heat is changed into the half) of steps in the baseline shifted in a step-like pattern is defined as the glass transition temperature (Tg). The temperature of the subsequently appearing exothermic peak is defined as the crystallization temperature. The heat is determined from the area of a region surrounded by the exothermic peak and the baseline and defined as the heat of crystallization.

The component (A) to be contained in the radiation-sensitive composition of the present embodiment is preferably low sublimable at 100°C or lower, preferably 120°C or lower, more preferably 130°C or lower, still more preferably 140°C or lower, and particularly preferably 150°C or lower at normal pressure. The low sublimability means that in thermogravimetry, weight reduction when the resist base material is kept at a predetermined temperature for 10 minutes is 10% or less, preferably 5% or less, more preferably 3% or less, still more preferably 1% or less, and particularly preferably 0.1% or less. The low sublimability can prevent an exposure apparatus from being contaminated by outgassing upon exposure. In addition, a good pattern shape with low roughness can be obtained.

The component (A) to be contained in the radiation-sensitive composition of the present embodiment dissolves at preferably 1% by mass or more, more preferably 5% by mass or more, and still more preferably 10% by mass or more at 23°C in a solvent that is selected from the group consisting of propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate and exhibits the highest ability to dissolve the component (A). Particularly preferably, the component (A) dissolves at 20% by mass or more at 23°C in a solvent that is selected from the group consisting of PGMEA, PGME, and CHN and exhibits the highest ability to dissolve the component (A). Particularly preferably, the component (A) dissolves at 20% by mass or more at 23°C in PGMEA. When the above conditions are met, the radiation-sensitive composition is easily used in a semiconductor production process at a full production scale.

### [Optically active diazonaphthoquinone compound (B)]

The optically active diazonaphthoquinone compound (B) to be contained in the radiation-sensitive composition of the present embodiment is a diazonaphthoquinone substance including a polymer or non-polymer optically active diazonaphthoquinone compound and is not particularly limited as long as it is generally used as a photosensitive component (sensitizing agent) in positive type resist compositions. One kind or two or more kinds can be optionally selected for use.

The component (B) is preferably a compound obtained by reacting naphthoquinonediazide sulfonic acid chloride, benzoquinonediazide sulfonic acid chloride, or the like with a low molecular weight compound or a high molecular weight compound having a functional group condensable with these acid chlorides. Herein, examples of the above functional group condensable with the acid chlorides include, but not particularly limited to, a hydroxyl group and an amino group. Particularly, a hydroxyl group is preferable. Examples of the compound containing a hydroxyl group condensable with the acid chlorides can include, but not particularly limited to, hydroquinone; resorcin; hydroxybenzophenones such as 2,4-dihydroxybenzophenone, 2,3,4-trihydroxybenzophenone, 2,4,6-trihydroxybenzophenone, 2,4,4'-trihydroxybenzophenone, 2,3,4,4'-tetrahydroxybenzophenone, 2,2',4,4'-tetrahydroxybenzophenone, and 2,2',3,4,6'-pentahydroxybenzophenone; hydroxyphenylalkanes such as bis(2,4-dihydroxyphenyl)methane, bis(2,3,4-trihydroxyphenyl)methane, and bis(2,4-dihydroxyphenyl)propane; and hydroxytriphenylmethanes such as 4,4',3",4"-tetrahydroxy-3,5,3',5'-tetramethyltriphenylmethane and 4,4',2",3",4"-pentahydroxy-3,5,3',5'-tetramethyltriphenylmethane.

Preferable examples of the acid chloride such as naphthoquinonediazide sulfonic acid chloride or benzoquinonediazide sulfonic acid chloride include 1,2-naphthoquinonediazide-5-sulfonyl chloride and 1,2-naphthoquinonediazide-4-sulfonyl chloride.

The radiation-sensitive composition of the present embodiment is preferably prepared by, for example, dissolving each component in a solvent upon use into a homogeneous solution, and then if required, filtering through a filter or the like with a pore diameter of about 0.2 µm, for example.

### [Properties of radiation-sensitive composition]

The radiation-sensitive composition of the present embodiment can be used to form an amorphous film by spin coating. Also, the radiation-sensitive composition of the present embodiment can be applied to a general semiconductor production process. Any of positive type and negative type resist patterns can be individually prepared depending on the kind of a developing solution to be used.

In the case of a positive type resist pattern, the dissolution rate of the amorphous film formed by spin coating with the radiation-sensitive composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound and/or the resin of the present embodiment, contrast at the interface between the exposed portion being dissolved in a developing solution and the unexposed portion not being dissolved in a developing solution is increased. Also, effects of reducing LER and defects are seen.

In the case of a negative type resist pattern, the dissolution rate of the amorphous film formed by spin coating with the radiation-sensitive composition of the present embodiment in a developing solution at 23°C is preferably 10 angstrom/sec or more. When the dissolution rate is 10 angstrom/sec or more, the amorphous film more easily dissolves in a developing solution, and is suitable for a resist. When the dissolution rate is 10 angstrom/sec or more, the resolution may improve. It is presumed that this is because the micro surface portion of the compound and/or the resin of the present embodiment dissolves, and LER is reduced. Also, effects of reducing defects are seen.

The above dissolution rate can be determined by immersing the amorphous film in a developing solution for a predetermined period of time at 23°C and then measuring the film thickness before and after immersion by a publicly known method such as visual, ellipsometric, or QCM method.

In the case of a positive type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 10 angstrom/sec or more, more preferably 10 to 10000 angstrom/sec, and still more preferably 100 to 1000 angstrom/sec. When the dissolution rate is 10 angstrom/sec or more, the amorphous film more easily dissolves in a developing solution, and is suitable for a resist. When the dissolution rate is 10000 angstrom/sec or less, the resolution may improve. It is presumed that this is because the micro surface portion of the compound and/or the resin of the present embodiment dissolves, and LER is reduced. Also, effects of reducing defects are seen.

In the case of a negative type resist pattern, the dissolution rate of the exposed portion after irradiation with radiation such as KrF excimer laser, extreme ultraviolet, electron beam or X-ray, or after heating at 20 to 500°C, of the amorphous film formed by spin coating with the radiation-sensitive composition of the present embodiment, in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, there is a tendency that the above portion is insoluble in a developing solution, and thus the amorphous film easily forms a resist. When the dissolution rate is 0.0005 angstrom/sec or more, the resolution may improve. It is presumed that this is because due to the change in the solubility before and after exposure of the compound and/or the resin of the present embodiment, contrast at the interface between the unexposed portion being dissolved in a developing solution and the exposed portion not being dissolved in a developing solution is increased. Also, effects of reducing LER and defects are seen.

### [Content ratio of each component]

In the radiation-sensitive composition of the present embodiment, the content of the component (A) is preferably 1 to 100% by mass of the total weight of the solid components (summation of the component (A), the optically active diazonaphthoquinone compound (B), and optionally used solid components such as further component (D), hereinafter the same), more preferably 1 to 99% by mass, further preferably 5 to 95% by mass, still more preferably 10 to 90% by mass, and particularly preferably 25 to 75% by mass. When the content of the component (A) falls within the above range, there is a tendency that the radiation-sensitive composition of the present embodiment can produce a pattern with high sensitivity and low roughness.

In the radiation-sensitive composition of the present embodiment, the content of the optically active diazonaphthoquinone compound (B) is preferably 1 to 99% by mass, more preferably 5 to 95% by mass, still more preferably 10 to 90% by mass, and particularly preferably 25 to 75% by mass, based on the total weight of the solid components. When the content of the optically active diazonaphthoquinone compound (B) falls within the above range, there is a tendency that the radiation-sensitive composition of the present embodiment can produce a pattern with high sensitivity and low roughness.

### [Further component (D)]

To the radiation-sensitive composition of the present embodiment, if required, as a component other than the component (A) and the optically active diazonaphthoquinone compound (B), one kind or two kinds or more of various additive agents such as an acid generating agent, acid crosslinking agent, acid diffusion controlling agent, dissolution promoting agent, dissolution controlling agent, sensitizing agent, surfactant, organic carboxylic acid or oxo acid of phosphor or derivative thereof, thermal and/or light curing catalyst, polymerization inhibitor, flame retardant, filler, coupling agent, thermosetting resin, light curable resin, dye, pigment, thickener, lubricant, antifoaming agent, leveling agent, ultraviolet absorber, surfactant, colorant, and nonionic surfactant can be added within the range not inhibiting the objects of the present invention. In the present specification, the further component (D) is also referred to as an optional component (D).

In the radiation-sensitive composition of the present embodiment, the content ratio of each component (the component (A)/the optically active diazonaphthoquinone compound (B)/the optional component (D)) is, in % by mass based on the solid components, preferably 1 to 99/99 to 1/0 to 98,
more preferably 5 to 95/95 to 5/0 to 49,
still more preferably 10 to 90/90 to 10/0 to 10, further preferably 20 to 80/80 to 20/0 to 5, and particularly preferably 25 to 75/75 to 25/0.

The content ratio of each component is selected from each range so that the summation thereof is 100% by mass. When the content ratio of each component falls within the above range, the radiation-sensitive composition of the present embodiment tends to be excellent in performance such as sensitivity and resolution, in addition to roughness.

The radiation-sensitive composition of the present embodiment may contain an additional resin other than the resin of the present embodiment within the range not inhibiting the objects of the present invention. Examples of such an additional resin include a novolac resin, a polyvinyl phenol, a polyacrylic acid, a polyvinyl alcohol, a styrene-maleic anhydride resin, and a polymer containing an acrylic acid, vinyl alcohol or vinylphenol as a monomeric unit, and a derivative thereof. The content of these resins, which is arbitrarily adjusted according to the kind of the component (A) to be used, is preferably 30 parts by mass or less per 100 parts by mass of the component (A), more preferably 10 parts by mass or less, still more preferably 5 parts by mass or less, and particularly preferably 0 part by mass.

In addition, the radiation-sensitive composition of the present embodiment may use the acid crosslinking agent, crosslinking promoting agent, radical polymerization initiator, acid generating agent, and basic compound listed in the "Film forming composition for lithography for underlayer film purposes", which will be mentioned later, within the range not inhibiting the objects of the present invention.

### [Method for forming resist pattern or insulating film]

The resist pattern formation method of the present embodiment includes the steps of: forming a photoresist layer on a substrate using the above resist composition or radiation-sensitive composition of the present embodiment; and then irradiating a predetermined region of the photoresist layer with radiation for development. An insulating film can be formed by the same method as the above resist pattern formation method.

In more detail, the resist pattern formation method of the present embodiment includes the steps of: forming a resist film on a substrate using the above resist composition or radiation-sensitive composition of the present embodiment; exposing the formed resist film; and developing the resist film, thereby forming a resist pattern. The resist pattern according to the present embodiment can also be formed as an upper layer resist in a multilayer process.

Examples of the resist pattern formation method include, but not particularly limited to, the following methods. A resist film is formed by coating a conventionally publicly known substrate with the resist composition or radiation-sensitive composition using a coating means such as spin coating, flow casting coating, and roll coating. Examples of the conventionally publicly known substrate include, but not particularly limited to, a substrate for electronic components, and the one having a predetermined wiring pattern formed thereon. More specific examples include a substrate made of a metal such as a silicon wafer, copper, chromium, iron and aluminum, and a glass substrate. Examples of a wiring pattern material include copper, aluminum, nickel, and gold. Also if required, the substrate may be a substrate having an inorganic and/or organic film provided thereon. Examples of the inorganic film include an inorganic antireflection film (inorganic BARC). Examples of the organic film include an organic antireflection film (organic BARC). The substrate may be subjected to surface treatment with hexamethylene disilazane or the like on the substrate.

Next, the substrate coated with the resist composition or the radiation-sensitive composition is heated if required. The heating conditions vary according to the compounding composition of the resist composition or radiation-sensitive composition, or the like, but are preferably 20 to 250°C, and more preferably 20 to 150°C. By heating, the adhesiveness of a resist to a substrate tends to improve, which is preferable. Then, the resist film is exposed to a desired pattern by any radiation selected from the group consisting of visible light, ultraviolet, excimer laser, electron beam, extreme ultraviolet (EUV), X-ray, and ion beam. The exposure conditions or the like are arbitrarily selected according to the compounding composition of the resist composition or radiation-sensitive composition, or the like. In the present embodiment, in order to stably form a fine pattern with a high degree of accuracy in exposure, the resist film is preferably heated after radiation irradiation. The heating conditions vary according to the compounding composition of the resist composition or radiation-sensitive composition, or the like, but are preferably 20 to 250°C, and more preferably 20 to 150°C.

Next, by developing the exposed resist film in a developing solution, a predetermined resist pattern is formed. As the developing solution, it is preferable to select a solvent having a solubility parameter (SP value) close to that of the compound or the resin of the present embodiment. For example, a polar solvent such as a ketone-based solvent, an ester-based solvent, an alcohol-based solvent, an amide-based solvent, and an ether-based solvent described in International Publication No. WO 2013/024778; and a hydrocarbon-based solvent, or an alkaline aqueous solution can be used.

A plurality of above solvents may be mixed, or the solvent may be used by mixing the solvent with a solvent other than those described above or water within the range having performance. From the viewpoint of sufficiently exhibiting the effect of the present invention, the water content ratio as the whole developing solution is preferably less than 70% by mass, more preferably less than 50% by mass, still more preferably less than 30% by mass, and further preferably less than 10% by mass. Particularly preferably, the developing solution is substantially moisture free. That is, the content of the organic solvent in the developing solution is preferably 30% by mass or more and 100% by mass or less based on the total amount of the developing solution, more preferably 50% by mass or more and 100% by mass or less, still more preferably 70% by mass or more and 100% by mass or less, further preferably 90% by mass or more and 100% by mass or less, and particularly preferably 95% by mass or more and 100% by mass or less.

Particularly, the developing solution is preferably a developing solution containing at least one kind of solvent selected from a ketone-based solvent, an ester-based solvent, an alcohol-based solvent, an amide-based solvent, and an ether-based solvent from the viewpoint of improving resist performance such as resolution and roughness of the resist pattern.

The vapor pressure of the developing solution is preferably 5 kPa or less at 20°C, more preferably 3 kPa or less, and still more preferably 2 kPa or less. When the vapor pressure of the developing solution is 5 kPa or less, there is a tendency that the evaporation of the developing solution on the substrate or in a developing cup is inhibited to improve temperature uniformity within a wafer surface, thereby resulting in improvement in size uniformity within the wafer surface.

Specific examples of the developing solution having a vapor pressure of 5 kPa or less at 20°C include those described in International Publication No. WO 2013/024778.

Specific examples of the developing solution having a vapor pressure of 2 kPa or less at 20°C include those described in International Publication No. WO 2013/024778.

To the developing solution, a surfactant can be added in an appropriate amount, if required. The surfactant is not particularly limited but, for example, an ionic or nonionic fluorine-based and/or silicon-based surfactant can be used. Examples of the fluorine-based and/or silicon-based surfactant can include the surfactants described in Japanese Patent Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but a fluorine-based surfactant or a silicon-based surfactant is preferable.

The amount of the surfactant used is usually 0.001 to 5% by mass based on the total amount of the developing solution, preferably 0.005 to 2% by mass, and further preferably 0.01 to 0.5% by mass.

As the development method, for example, a method for dipping a substrate in a bath filled with a developing solution for a fixed time (dipping method), a method for raising a developing solution on a substrate surface by the effect of a surface tension and keeping it still for a fixed time (puddle method), a method for spraying a developing solution on a substrate surface (spraying method), and a method for continuously ejecting a developing solution on a substrate rotating at a constant speed while scanning a developing solution ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the resist pattern cured by crosslinking. A solution containing a general organic solvent or water may be used as the rinsing solution. As the foregoing rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still more preferably, after development, a step of rinsing the film by using a rinsing solution containing an alcohol-based solvent or an ester-based solvent is conducted. Still more preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, examples of the monohydric alcohol used in the rinsing step after development include a linear, branched or cyclic monohydric alcohol, and for example, those described in International Publication No. WO 2013/024778 can be used. Particularly preferable examples of the monohydric alcohol having 5 or more carbon atoms include 1-hexanol, 2-hexanol, 4-methyl-2-pentanol, 1-pentanol, and 3-methyl-1-butanol.

A plurality of these components may be mixed, or the component may be used by mixing the component with an organic solvent other than those described above.

The water content ratio in the rinsing solution is preferably 10% by mass or less, more preferably 5% by mass or less, and still more preferably 3% by mass or less. By setting the water content ratio in the rinsing solution to 10% by mass or less, there is a tendency that better development characteristics can be obtained.

The vapor pressure at 20°C of the rinsing solution used after development is preferably 0.05 kPa or more and 5 kPa or less, more preferably 0.1 kPa or more and 5 kPa or less, and still more preferably 0.12 kPa or more and 3 kPa or less. When the vapor pressure of the rinsing solution is 0.05 kPa or more and 5 kPa or less, there is a tendency that the temperature uniformity in the wafer surface is enhanced and moreover, swelling due to permeation of the rinsing solution is further inhibited; and as a result, the dimensional uniformity in the wafer surface is further improved.

The rinsing solution may also be used after adding an appropriate amount of a surfactant to the rinsing solution.

In the rinsing step, the wafer after development is rinsed using the above organic solvent-containing rinsing solution. The method for rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to conduct the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the plating method include copper plating, solder plating, nickel plating, and gold plating.

The remaining resist pattern after etching can be peeled by an organic solvent. Examples of the organic solvent include PGMEA (propylene glycol monomethyl ether acetate), PGME (propylene glycol monomethyl ether), and EL (ethyl lactate). Examples of the peeling method include a dipping method and a spraying method. A wiring substrate having a resist pattern formed thereon may be a multilayer wiring substrate, and may have a small diameter through hole.

The wiring substrate of the present embodiment can also be formed by a method for forming a resist pattern, then depositing a metal in vacuum, and subsequently dissolving the resist pattern in a solution, i.e., a liftoff method.

### [Film forming composition for lithography for underlayer film purposes]

The film forming composition for lithography for underlayer film purposes according to the present embodiment (hereinafter, also referred to as an "underlayer film forming material") contains the compound and/or the resin of the present embodiment. In the present embodiment, the content of the above substance in the solid components of the underlayer film forming material is preferably 1 to 100% by mass, more preferably 10 to 100% by mass, further preferably 50 to 100% by mass, still more preferably 80 to 100% by mass, and particularly preferably 100% by mass, from the viewpoint of coatability and quality stability.

The film forming composition for lithography for underlayer film purposes according to the present embodiment can also be used as an optical component forming composition. For example, by coating a substrate with the optical component forming composition or casting the optical component forming composition, an optical component forming film can be formed.

The underlayer film forming material of the present embodiment is applicable to a wet process and is excellent in heat resistance and etching resistance. Furthermore, the underlayer film forming material of the present embodiment employs the above substances and can therefore form an underlayer film that is prevented from deteriorating during high temperature baking and is also excellent in etching resistance against oxygen plasma etching or the like. Moreover, the underlayer film forming material of the present embodiment is also excellent in adhesiveness to a resist layer and can therefore produce an excellent resist pattern. The underlayer film forming material of the present embodiment may contain an already known underlayer film forming material for lithography or the like, within the range not deteriorating the effect of the present invention.

### [Solvent]

The underlayer film forming material of the present embodiment may contain a solvent. The solvent used for the underlayer film forming material is not particularly limited, and a publicly known solvent can be arbitrarily used as long as at least the above substances dissolve.

Specific examples of the solvent include, but not particularly limited to: ketone-based solvents such as acetone, methyl ethyl ketone, methyl isobutyl ketone, and cyclohexanone; cellosolve-based solvents such as propylene glycol monomethyl ether and propylene glycol monomethyl ether acetate; ester-based solvents such as ethyl lactate, methyl acetate, ethyl acetate, butyl acetate, isoamyl acetate, ethyl lactate, methyl methoxypropionate, and methyl hydroxyisobutyrate; alcohol-based solvents such as methanol, ethanol, isopropanol, and 1-ethoxy-2-propanol; and aromatic hydrocarbons such as toluene, xylene, and anisole. These solvents can be used alone as one kind or used in combination of two or more kinds.

Among the above solvents, cyclohexanone, propylene glycol monomethyl ether, propylene glycol monomethyl ether acetate, ethyl lactate, methyl hydroxyisobutyrate, or anisole is particularly preferable from the viewpoint of safety.

The content of the solvent is not particularly limited and is preferably 100 to 10,000 parts by mass based on 100 parts by mass of the compound and/or the resin of the present embodiment, more preferably 200 to 5,000 parts by mass, and still more preferably 200 to 1,000 parts by mass, from the viewpoint of solubility and film formation.

### [Acid crosslinking agent]

The underlayer film forming material according to the present embodiment may contain an acid crosslinking agent, if required, from the viewpoint of, for example, suppressing intermixing. The acid crosslinking agent is not particularly limited, but those described in, for example, International Publication No. WO 2013/024779 can be used.

Specific examples of the acid crosslinking agent that may be used in the present embodiment include, but not particularly limited to, phenol compounds, epoxy compounds, cyanate compounds, amino compounds, benzoxazine compounds, acrylate compounds, melamine compounds, guanamine compounds, glycoluril compounds, urea compounds, isocyanate compounds, and azide compounds. These acid crosslinking agents can be used alone as one kind or can be used in combination of two or more kinds. Among them, a benzoxazine compound, an epoxy compound or a cyanate compound is preferable, and a benzoxazine compound is more preferable from the viewpoint of improvement in etching resistance.

As the above phenol compound, a publicly known compound can be used. Examples of the phenol include phenol as well as alkylphenols such as cresols and xylenols, polyhydric phenols such as hydroquinone, polycyclic phenols such as naphthols and naphthalenediols, bisphenols such as bisphenol A and bisphenol F, and polyfunctional phenol compounds such as phenol novolac and phenol aralkyl resins. Among them, an aralkyl-based phenol resin is preferred from the viewpoint of heat resistance and solubility.

As the above epoxy compound, a publicly known compound can be used and is selected from among compounds having two or more epoxy groups in one molecule. Examples thereof include epoxidation products of dihydric phenols such as bisphenol A, bisphenol F, 3,3',5,5'-tetramethyl-bisphenol F, bisphenol S, fluorene bisphenol, 2,2'-biphenol, 3,3',5,5'-tetramethyl-4,4'-dihydroxybiphenol, resorcin, and naphthalenediols, epoxidation products of trihydric or higher phenols such as tris-(4-hydroxyphenyl)methane, 1,1,2,2-tetrakis(4-hydroxyphenyl)ethane, tris(2,3-epoxypropyl) isocyanurate, trimethylolmethane triglycidyl ether, trimethylolpropane triglycidyl ether, triethylolethane triglycidyl ether, phenol novolac, and o-cresol novolac, epoxidation products of co-condensed resins of dicyclopentadiene and phenols, epoxidation products of phenol aralkyl resins synthesized from phenols and paraxylylene dichloride or the like, epoxidation products of biphenyl aralkyl-based phenolic resins synthesized from phenols and bischloromethylbiphenyl or the like, and epoxidation products of naphthol aralkyl resins synthesized from naphthols and paraxylylene dichloride or the like. These epoxy resins may be used alone or in combination of two or more kinds. Above all, an epoxy resin that is in a solid state at normal temperature, such as an epoxy resin obtained from a phenol aralkyl resin or a biphenyl aralkyl resin, is preferable from the viewpoint of heat resistance and solubility.

The above cyanate compound is not particularly limited as long as the compound has two or more cyanate groups in one molecule, and a publicly known compound can be used. In the present embodiment, preferable examples of the cyanate compound include cyanate compounds having a structure where hydroxy groups of a compound having two or more hydroxy groups in one molecule are replaced with cyanate groups. Also, the cyanate compound preferably has an aromatic group, and a structure where a cyanate group is directly bonded to an aromatic group can be preferably used. Examples of such a cyanate compound include cyanate compounds having a structure where hydroxy groups of bisphenol A, bisphenol F, bisphenol M, bisphenol P, bisphenol E, a phenol novolac resin, a cresol novolac resin, a dicyclopentadiene novolac resin, tetramethylbisphenol F, a bisphenol A novolac resin, brominated bisphenol A, a brominated phenol novolac resin, trifunctional phenol, tetrafunctional phenol, naphthalene-based phenol, biphenyl-based phenol, a phenol aralkyl resin, a biphenyl aralkyl resin, a naphthol aralkyl resin, a dicyclopentadiene aralkyl resin, alicyclic phenol, phosphorus-containing phenol, or the like are replaced with cyanate groups. These cyanate compounds may be used alone or in arbitrary combination of two or more kinds. Also, the above cyanate compound may be in any form of a monomer, an oligomer and a resin.

Examples of the above amino compound include m-phenylenediamine, p-phenylenediamine, 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,4'-diaminodiphenylsulfone, 3,3'-diaminodiphenylsulfone, 4,4'-diaminodiphenyl sulfide, 3,4'-diaminodiphenyl sulfide, 3,3'-diaminodiphenyl sulfide, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, bis[4-(3-aminophenoxy)phenyl] ether, 9,9-bis(4-aminophenyl)fluorene, 9,9-bis(4-amino-3-chlorophenyl)fluorene, 9,9-bis(4-amino-3-fluorophenyl)fluorene, O-tolidine, m-tolidine, 4,4'-diaminobenzanilide, 2,2'-bis(trifluoromethyl)-4,4'-diaminobiphenyl, 4-aminophenyl-4-aminobenzoate, and 2-(4-aminophenyl)-6-aminobenzoxazole. Further examples thereof include aromatic amines such as 4,4'-diaminodiphenylmethane, 4,4'-diaminodiphenylpropane, 4,4'-diaminodiphenyl ether, 3,4'-diaminodiphenyl ether, 3,3'-diaminodiphenyl ether, 4,4'-diaminodiphenylsulfone, 3,3'-diaminodiphenylsulfone, 1,4-bis(4-aminophenoxy)benzene, 1,3-bis(4-aminophenoxy)benzene, 1,4-bis(3-aminophenoxy)benzene, 1,3-bis(3-aminophenoxy)benzene, bis[4-(4-aminophenoxy)phenyl]sulfone, 2,2-bis[4-(4-aminophenoxy)phenyl]propane, 2,2-bis[4-(3-aminophenoxy)phenyl]propane, 4,4'-bis(4-aminophenoxy)biphenyl, 4,4'-bis(3-aminophenoxy)biphenyl, bis[4-(4-aminophenoxy)phenyl] ether, and bis[4-(3-aminophenoxy)phenyl] ether, alicyclic amines such as diaminocyclohexane, diaminodicyclohexylmethane, dimethyl-diaminodicyclohexylmethane, tetramethyl-diaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bisaminomethylcyclohexane, and isophoronediamine, and aliphatic amines such as ethylenediamine, hexamethylenediamine, octamethylenediamine, decamethylenediamine, diethylenetriamine, and triethylenetetramine.

Examples of the above benzoxazine compound include P-d-based benzoxazines obtained from difunctional diamines and monofunctional phenols, and F-a-based benzoxazines obtained from monofunctional diamines and difunctional phenols.

Specific examples of the above melamine compound include hexamethylolmelamine, hexamethoxymethylmelamine, a compound in which 1 to 6 methylol groups of hexamethylolmelamine are methoxymethylated or a mixture thereof, hexamethoxyethylmelamine, hexaacyloxymethylmelamine, and a compound in which 1 to 6 methylol groups of hexamethylolmelamine are acyloxymethylated or a mixture thereof.

Specific examples of the above guanamine compound include tetramethylolguanamine, tetramethoxymethylguanamine, a compound in which 1 to 4 methylol groups of tetramethylolguanamine are methoxymethylated or a mixture thereof, tetramethoxyethylguanamine, tetraacyloxyguanamine, and a compound in which 1 to 4 methylol groups of tetramethylolguanamine are acyloxymethylated or a mixture thereof.

Specific examples of the above glycoluril compound include tetramethylolglycoluril, tetramethoxyglycoluril, tetramethoxymethylglycoluril, a compound in which 1 to 4 methylol groups of tetramethylolglycoluril are methoxymethylated or a mixture thereof, and a compound in which 1 to 4 methylol groups of tetramethylolglycoluril are acyloxymethylated or a mixture thereof.

Specific examples of the above urea compound include tetramethylolurea, tetramethoxymethylurea, a compound in which 1 to 4 methylol groups of tetramethylolurea are methoxymethylated or a mixture thereof, and tetramethoxyethylurea.

In the present embodiment, an acid crosslinking agent having at least one allyl group may also be used from the viewpoint of improvement in crosslinkability. Specific examples of the acid crosslinking agent having at least one allyl group include, but not limited to, allylphenols such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide, and bis(3-allyl-4-hydroxyphenyl) ether, allyl cyanates such as 2,2-bis(3-allyl-4-cyanatophenyl)propane, 1,1,1,3,3,3-hexafluoro-2,2-bis(3-allyl-4-cyanatophenyl)propane, bis(3-allyl-4-cyanatophenyl)sulfone, bis(3-allyl-4-cyanatophenyl) sulfide, and bis(3-allyl-4-cyanatophenyl) ether, diallyl phthalate, diallyl isophthalate, diallyl terephthalate, triallyl isocyanurate, trimethylolpropane diallyl ether, and pentaerythritol allyl ether. These crosslinking agents may be alone, or may be a mixture of two or more kinds. Among them, an allylphenol such as 2,2-bis(3-allyl-4-hydroxyphenyl)propane, 1,1,1,3,3,3-hexafluoro-2,2-bis(3-allyl-4-hydroxyphenyl)propane, bis(3-allyl-4-hydroxyphenyl)sulfone, bis(3-allyl-4-hydroxyphenyl) sulfide and bis(3-allyl-4-hydroxyphenyl) ether is preferable from the viewpoint of excellent compatibility with a bismaleimide compound and/or an addition polymerization maleimide resin.

The content of the acid crosslinking agent in the underlayer film forming material is not particularly limited and is preferably 0.1 to 100 parts by mass based on 100 parts by mass of the compound and/or the resin of the present embodiment, more preferably 5 to 50 parts by mass, and further preferably 10 to 40 parts by mass. By setting the content of the acid crosslinking agent to the above range, a mixing event with a resist layer tends to be prevented. Also, an antireflection effect is enhanced, and film formability after crosslinking tends to be enhanced.

### [Crosslinking promoting agent]

In the underlayer film forming material of the present embodiment, if required, a crosslinking promoting agent for accelerating crosslinking and curing reaction can be used.

The above crosslinking promoting agent is not particularly limited as long as it accelerates crosslinking or curing reaction, and examples thereof include amines, imidazoles, organic phosphines, and Lewis acids. These crosslinking promoting agents can be used alone as one kind or can be used in combination of two or more kinds. Among them, an imidazole or an organic phosphine is preferable, and an imidazole is more preferable from the viewpoint of decrease in crosslinking temperature.

Examples of the above crosslinking promoting agent include, but not limited to, tertiary amines such as 1,8-diazabicyclo(5,4,0)undecene-7, triethylenediamine, benzyldimethylamine, triethanolamine, dimethylaminoethanol, and tris(dimethylaminomethyl)phenol; imidazoles such as 2-methylimidazole, 2-phenylimidazole, 2-ethyl-4-methylimidazole, 2-phenyl-4-methylimidazole, 2-heptadecylimidazole, and 2,4,5-triphenylimidazole; organic phosphines such as tributylphosphine, methyldiphenylphosphine, triphenylphosphine, diphenylphosphine, and phenylphosphine; tetra substituted phosphonium-tetra substituted borates such as tetraphenylphosphonium-tetraphenyl borate, tetraphenylphosphonium-ethyltriphenyl borate, and tetrabutylphosphonium-tetrabutyl borate; and tetraphenylboron salts such as 2-ethyl-4-methylimidazole-tetraphenyl borate and N-methylmorpholine-tetraphenyl borate.

The content of the crosslinking promoting agent is usually preferably 0.1 to 10 parts by mass based on 100 parts by mass of the compound and/or the resin of the present embodiment, and is more preferably 0.1 to 5 parts by mass, and still more preferably 0.1 to 3 parts by mass, from the viewpoint of easy control and cost efficiency.

### [Radical polymerization initiator]

The underlayer film forming material of the present embodiment can contain, if required, a radical polymerization initiator. The radical polymerization initiator may be a photopolymerization initiator that initiates radical polymerization by light, or may be a thermal polymerization initiator that initiates radical polymerization by heat.

Such a radical polymerization initiator is not particularly limited, and a radical polymerization initiator conventionally used can be arbitrarily adopted. Examples thereof include ketone-based photopolymerization initiators such as 1-hydroxy cyclohexyl phenyl ketone, benzyl dimethyl ketal, 2-hydroxy-2-methyl-1-phenylpropan-1-one, 1-[4-(2-hydroxyethoxy)-phenyl]-2-hydroxy-2-methyl-1-propan-1-one, 2-hydroxy-1-{4-[4-(2-hydroxy-2-methylpropionyl)-benzyl]phenyl}-2-methylpropan-1-one, 2,4,6-trimethylbenzoyl-diphenyl-phosphine oxide, and bis(2,4,6-trimethylbenzoyl)-phenylphosphine oxide, and organic peroxide-based polymerization initiators such as methyl ethyl ketone peroxide, cyclohexanone peroxide, methylcyclohexanone peroxide, methyl acetoacetate peroxide, acetyl acetate peroxide, 1,1-bis(t-hexylperoxy)-3,3,5-trimethylcyclohexane, 1,1-bis(t-hexylperoxy)-cyclohexane, 1,1-bis(t-butylperoxy)-3,3,5-trimethylcyclohexane, 1,1-bis(t-butylperoxy)-2-methylcyclohexane, 1,1-bis(t-butylperoxy)-cyclohexane, 1,1-bis(t-butylperoxy)cyclododecane, 1,1-bis(t-butylperoxy)butane, 2,2-bis(4,4-di-t-butylperoxycyclohexyl)propane, p-menthane hydroperoxide, diisopropylbenzene hydroperoxide, 1,1,3,3-tetramethylbutyl hydroperoxide, cumene hydroperoxide, t-hexyl hydroperoxide, t-butyl hydroperoxide, α,α'-bis(t-butylperoxy)diisopropylbenzene, dicumyl peroxide, 2,5-dimethyl-2,5-bis(t-butylperoxy)hexane, t-butylcumyl peroxide, di-t-butyl peroxide, 2,5-dimethyl-2,5-bis(t-butylperoxy)hexyne-3, isobutyryl peroxide, 3,5,5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexanoate, 1-cyclohexyl-1-methylethyl peroxy-2-ethylhexanoate, t-hexyl peroxy-2-ethylhexanoate, t-butyl peroxy-2-ethylhexanoate, t-hexyl peroxyisopropylmonocarbonate, t-butyl peroxyisobutyrate, t-butyl peroxymalate, t-butyl peroxy-3,5,5-trimethylhexanoate, t-butyl peroxylaurate, t-butyl peroxyisopropylmonocarbonate, t-butyl peroxy-2-ethylhexylmonocarbonate, t-butyl peroxyacetate, t-butyl peroxy-m-toluylbenzoate, t-butyl peroxybenzoate, bis(t-butylperoxy) isophthalate, 2,5-dimethyl-2,5-bis(m-toluylperoxy)hexane, t-hexyl peroxybenzoate, 2,5-dimethyl-2,5-bis(benzoylperoxy)hexane, t-butyl peroxyallylmonocarbonate, t-butyltrimethylsilyl peroxide, 3,3',4,4'-tetra(t-butylperoxycarbonyl)benzophenone, and 2,3-dimethyl-2,3-diphenylbutane.

Further examples thereof include azo-based polymerization initiators such as 2-phenylazo-4-methoxy-2,4-dimethylvaleronitrile, 1-[(1-cyano-1-methylethyl)azo]formamide, 1,1'-azobis(cyclohexane-1-carbonitrile), 2,2'-azobis(2-methylbutyronitrile), 2,2'-azobisisobutyronitrile, 2,2'-azobis(2,4-dimethylvaleronitrile), 2,2'-azobis(2-methylpropionamidine) dihydrochloride, 2,2'-azobis(2-methyl-N-phenylpropionamidine) dihydrochloride, 2,2'-azobis[N-(4-chlorophenyl)-2-methylpropionamidine]dihydride chloride, 2,2'-azobis[N-(4-hydrophenyl)-2-methylpropionamidine] dihydrochloride, 2,2'-azobis[2-methyl-N-(phenylmethyl)propionamidine] dihydrochloride, 2,2'-azobis[2-methyl-N-(2-propenyl)propionamidine] dihydrochloride, 2,2'-azobis[N-(2-hydroxyethyl)-2-methylpropionamidine] dihydrochloride, 2,2'-azobis[2-(5-methyl-2-imidazolin-2-yl)propane] dihydrochloride, 2,2'-azobis[2-(2-imidazolin-2-yl)propane] dihydrochloride, 2,2'-azobis[2-(4,5,6,7-tetrahydro-1H-1,3-diazepin-2-yl)propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl)propane] dihydrochloride, 2,2'-azobis[2-[1-(2-hydroxyethyl)-2-imidazolin-2-yl]propane] dihydrochloride, 2,2'-azobis[2-(2-imidazolin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)ethyl]propionamide], 2,2'-azobis[2-methyl-N-(2-hydroxyethyl)propionamide], 2,2'-azobis(2-methylpropionamide), 2,2'-azobis(2,4,4-trimethylpentane), 2,2'-azobis(2-methylpropane), dimethyl-2,2-azobis(2-methylpropionate), 4,4'-azobis(4-cyanopentanoic acid), and 2,2'-azobis[2-(hydroxymethyl)propionitrile]. As the radical polymerization initiator according to the present embodiment, one kind thereof may be used alone, or two or more kinds may be used in combination. Alternatively, the radical polymerization initiator according to the present embodiment may be used in further combination with an additional publicly known polymerization initiator.

The content of the above radical polymerization initiator can be a stoichiometrically necessary amount and is preferably 0.05 to 25 parts by mass, and more preferably 0.1 to 10 parts by mass, based on 100 parts by mass of the compound and/or the resin of the present embodiment. When the content of the radical polymerization initiator is 0.05 parts by mass or more, there is a tendency that curing can be prevented from being insufficient. On the other hand, when the content of the radical polymerization initiator is 25 parts by mass or less, there is a tendency that the long term storage stability of the underlayer film forming material at room temperature can be prevented from being impaired.

### [Acid generating agent]

The underlayer film forming material of the present embodiment may contain an acid generating agent, if required, from the viewpoint of, for example, further accelerating crosslinking reaction by heat. An acid generating agent that generates an acid by thermal decomposition, an acid generating agent that generates an acid by light irradiation, and the like are known, any of which can be used. As the acid generating agent, for example, those described in International Publication No. WO 2013/024779 can be used.

The content of the acid generating agent in the underlayer film forming material is not particularly limited and is preferably 0.1 to 50 parts by mass, and more preferably 0.5 to 40 parts by mass, based on 100 parts by mass of the compound and/or the resin of the present embodiment. By setting the content of the acid generating agent to the above range, crosslinking reaction tends to be enhanced by an increased amount of an acid generated. Also, a mixing event with a resist layer tends to be prevented.

### [Basic compound]

The underlayer film forming material according to the present embodiment may contain a basic compound from the viewpoint of, for example, improving storage stability.

The basic compound plays a role as a quencher against acids in order to prevent crosslinking reaction from proceeding due to a trace amount of an acid generated from the acid generating agent. Examples of such a basic compound include, but not particularly limited to, those described in International Publication No. WO 2013/024779.

The content of the basic compound in the underlayer film forming material is not particularly limited and is preferably 0.001 to 2 parts by mass, and more preferably 0.01 to 1 part by mass, based on 100 parts by mass of the compound and/or the resin of the present embodiment. By setting the content of the basic compound to the above range, storage stability tends to be enhanced without excessively deteriorating crosslinking reaction.

### [Further additive agent]

The underlayer film forming material according to the present embodiment may also contain an additional resin and/or compound for the purpose of conferring thermosetting or light curing properties or controlling absorbance. Examples of such an additional resin and/or compound include, but not particularly limited to, a naphthol resin, a xylene resin, a naphthol-modified resin, a phenol-modified resin of a naphthalene resin; a polyhydroxystyrene, a dicyclopentadiene resin, a resin containing (meth)acrylate, dimethacrylate, trimethacrylate, tetramethacrylate, a naphthalene ring such as vinylnaphthalene or polyacenaphthylene, a biphenyl ring such as phenanthrenequinone or fluorene, or a heterocyclic ring having a heteroatom such as thiophene or indene, and a resin not containing an aromatic ring; and a resin or compound containing an alicyclic structure, such as a rosin-based resin, a cyclodextrin, an adamantine(poly)ol, a tricyclodecane(poly)ol, and a derivative thereof. The underlayer film forming material according to the present embodiment may further contain a publicly known additive agent. Examples of the publicly known additive agent include, but not limited to, a thermal and/or light curing catalyst, a polymerization inhibitor, a flame retardant, a filler, a coupling agent, a thermosetting resin, a light curable resin, a dye, a pigment, a thickener, a lubricant, an antifoaming agent, a leveling agent, an ultraviolet absorber, a surfactant, a colorant, and a nonionic surfactant.

### [Underlayer film for lithography and multilayer resist pattern formation method]

The underlayer film for lithography according to the present embodiment is formed from the underlayer film forming material described above.

The resist pattern formation method of the present embodiment includes the steps of: forming an underlayer film on a substrate using the above composition; forming at least one photoresist layer on the underlayer film; and then irradiating a predetermined region of the photoresist layer with radiation for development. In more detail, the resist pattern formation method of the present embodiment has the steps of: forming an underlayer film on a substrate using the underlayer film forming material of the present embodiment (step (A-1)); forming at least one photoresist layer on the underlayer film (step (A-2)); and irradiating a predetermined region of the photoresist layer with radiation for development after the step (A-2) (step (A-3)).

Furthermore, the circuit pattern formation method of the present embodiment includes the steps of: forming an underlayer film on a substrate using the above composition, forming an intermediate layer film on the underlayer film using a resist intermediate layer film material, and forming at least one photoresist layer on the intermediate layer film;
irradiating a predetermined region of the photoresist layer with radiation for development, thereby forming a resist pattern; and
etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate.

In more detail, the circuit pattern formation method of the present embodiment includes the steps of: forming an underlayer film on a substrate using the underlayer film forming material of the present embodiment (step (B-1)); forming an intermediate layer film on the underlayer film using a resist intermediate layer film material containing a silicon atom (step (B-2)); forming at least one photoresist layer on the intermediate layer film (step (B-3)); after the step (B-3), irradiating a predetermined region of the photoresist layer with radiation for development, thereby forming a resist pattern (step (B-4)); and after the step (B-4), etching the intermediate layer film with the resist pattern as a mask, etching the underlayer film with the obtained intermediate layer film pattern as an etching mask, and etching the substrate with the obtained underlayer film pattern as an etching mask, thereby forming a pattern on the substrate (step (B-5)).

The underlayer film for lithography according to the present embodiment is not particularly limited by its formation method as long as it is formed from the underlayer film forming material of the present embodiment. A publicly known approach can be applied thereto. The underlayer film for lithography of the present embodiment can be formed by, for example, applying the underlayer film forming material of the present embodiment onto a substrate by a publicly known coating method or printing method such as spin coating or screen printing, and then removing an organic solvent by volatilization or the like, followed by crosslinking and curing by a publicly known method. Examples of the crosslinking method include approaches such as thermosetting and light curing. By removing the organic solvent by volatilization or the like, the underlayer film can be formed.

It is preferable to perform baking in the formation of the underlayer film, for preventing a mixing event with an upper layer resist while accelerating crosslinking reaction. In this case, the baking temperature is not particularly limited and is preferably in the range of 80 to 450°C, and more preferably 200 to 400°C. The baking time is also not particularly limited and is preferably in the range of 10 to 300 seconds. The thickness of the underlayer film can be arbitrarily selected according to required performance and is not particularly limited, but is usually preferably about 30 to 20,000 nm, and more preferably 50 to 15,000 nm.

After preparing the underlayer film on the substrate, in the case of a two-layer process, a silicon-containing resist layer or a usual single-layer resist made of hydrocarbon can be prepared on the underlayer film. In the case of a three-layer process, a silicon-containing intermediate layer can be prepared on the underlayer film, and a silicon-free single-layer resist layer can be further prepared on the silicon-containing intermediate layer. In these cases, the photoresist material for forming the resist layer can be arbitrarily selected for use from those that are publicly known, and is not particularly limited.

For the silicon-containing resist material for a two-layer process, a silicon atom-containing polymer such as a polysilsesquioxane derivative or a vinylsilane derivative is used as a base polymer, and a positive type photoresist material further containing an organic solvent, an acid generating agent, and if required, a basic compound or the like is preferably used, from the viewpoint of oxygen gas etching resistance. Herein, a publicly known polymer that is used in this kind of resist material can be used as the silicon atom-containing polymer.

A polysilsesquioxane-based intermediate layer is preferably used as the silicon-containing intermediate layer for a three-layer process. By imparting effects as an antireflection film to the intermediate layer, there is a tendency that reflection can be effectively suppressed. For example, use of a material containing a large amount of an aromatic group and having high substrate etching resistance as the underlayer film in a process for exposure at 193 nm tends to increase a k value and enhance substrate reflection. However, the intermediate layer suppresses the reflection so that the substrate reflection can be 0.5% or less. The intermediate layer having such an antireflection effect is not limited, and polysilsesquioxane that crosslinks by an acid or heat in which a light absorbing group having a phenyl group or a silicon-silicon bond is introduced is preferably used for exposure at 193 nm.

Alternatively, an intermediate layer formed by chemical vapour deposition (CVD) may be used. The intermediate layer highly effective as an antireflection film prepared by CVD is not limited, and, for example, a SiON film is known. In general, the formation of an intermediate layer by a wet process such as spin coating or screen printing is more convenient and more advantageous in cost than CVD. The upper layer resist for a three-layer process may be positive type or negative type, and the same as a single-layer resist generally used can be used.

The underlayer film according to the present embodiment can also be used as an antireflection film for usual single-layer resists or an underlying material for suppression of pattern collapse. The underlayer film of the present embodiment is excellent in etching resistance for an underlying process and can be expected to also function as a hard mask for an underlying process.

In the case of forming a resist layer from the above photoresist material, a wet process such as spin coating or screen printing is preferably used, as in the case of forming the above underlayer film. After coating with the resist material by spin coating or the like, prebaking is generally performed. This prebaking is preferably performed at 80 to 180°C in the range of 10 to 300 seconds. Then, exposure, post-exposure baking (PEB), and development can be performed according to a conventional method to obtain a resist pattern. The thickness of the resist film is not particularly limited and is generally preferably 30 to 500 nm, and more preferably 50 to 400 nm.

The exposure light can be arbitrarily selected for use according to the photoresist material to be used. General examples of the exposure light can include a high energy ray having a wavelength of 300 nm or less, specifically, excimer laser of 248 nm, 193 nm, or 157 nm, soft x-ray of 3 to 20 nm, electron beam, and X-ray.

In a resist pattern formed by the above method, pattern collapse is suppressed by the underlayer film. Therefore, use of the underlayer film according to the present embodiment can produce a finer pattern and can reduce an exposure amount necessary for obtaining the resist pattern.

Next, etching is performed with the obtained resist pattern as a mask. Gas etching is preferably used as the etching of the underlayer film in a two-layer process. The gas etching is preferably etching using oxygen gas. In addition to oxygen gas, an inert gas such as He or Ar, or CO, CO₂, NH₃, SO₂, N₂, NO₂, or H₂ gas may be added. Alternatively, the gas etching may be performed with CO, CO₂, NH₃, N₂, NO₂, or H₂ gas without the use of oxygen gas. Particularly, the latter gas is preferably used for side wall protection in order to prevent the undercut of pattern side walls.

On the other hand, gas etching is also preferably used as the etching of the intermediate layer in a three-layer process. The same gas etching as described in the above two-layer process is applicable. Particularly, it is preferable to process the intermediate layer in a three-layer process by using chlorofluorocarbon-based gas and using the resist pattern as a mask. Then, as mentioned above, for example, the underlayer film can be processed by oxygen gas etching with the intermediate layer pattern as a mask.

Herein, in the case of forming an inorganic hard mask intermediate layer film as the intermediate layer, a silicon oxide film, a silicon nitride film, or a silicon oxynitride film (SiON film) is formed by CVD, ALD, or the like. A method for forming the nitride film is not limited, and, for example, a method described in Japanese Patent Laid-Open No. 2002-334869 or WO 2004/066377 can be used. Although a photoresist film can be formed directly on such an intermediate layer film, an organic antireflection film (BARC) may be formed on the intermediate layer film by spin coating and a photoresist film may be formed thereon.

A polysilsesquioxane-based intermediate layer is preferably used as the intermediate layer. By imparting effects as an antireflection film to the resist intermediate layer film, there is a tendency that reflection can be effectively suppressed. A specific material for the polysilsesquioxane-based intermediate layer is not limited, and, for example, a material described in Japanese Patent Laid-Open No. 2007-226170 or Japanese Patent Laid-Open No. 2007-226204 can be used.

The subsequent etching of the substrate can also be performed by a conventional method. For example, the substrate made of SiO₂ or SiN can be etched mainly using chlorofluorocarbon-based gas, and the substrate made of p-Si, Al, or W can be etched mainly using chlorine- or bromine-based gas. In the case of etching the substrate with chlorofluorocarbon-based gas, the silicon-containing resist of the two-layer resist process or the silicon-containing intermediate layer of the three-layer process is peeled at the same time with substrate processing. On the other hand, in the case of etching the substrate with chlorine- or bromine-based gas, the silicon-containing resist layer or the silicon-containing intermediate layer is separately peeled and in general, peeled by dry etching using chlorofluorocarbon-based gas after substrate processing.

A feature of the underlayer film of the present embodiment is that it is excellent in etching resistance of the substrates. The substrate can be arbitrarily selected from publicly known ones and used and is not particularly limited. Examples thereof include Si, α-Si, p-Si, SiO₂, SiN, SiON, W, TiN, and Al. The substrate may be a laminate having a film to be processed (substrate to be processed) on a base material (support). Examples of such a film to be processed include various low-k films such as Si, SiO₂, SiON, SiN, p-Si, α-Si, W, W-Si, Al, Cu, and Al-Si, and stopper films thereof. A material different from that for the base material (support) is generally used. The thickness of the substrate to be processed or the film to be processed is not particularly limited and is generally preferably about 50 to 10,000 nm, and more preferably 75 to 5,000 nm.

The resist permanent film prepared by coating with the composition according to the present embodiment is suitable as a permanent film that also remains in a final product, if required, after formation of a resist pattern. Specific examples of the permanent film include, in relation to semiconductor devices, solder resists, package materials, underfill materials, package adhesive layers for circuit elements and the like, and adhesive layers between integrated circuit elements and circuit substrates, and in relation to thin displays, thin film transistor protecting films, liquid crystal color filter protecting films, black matrixes, and spacers. Particularly, the permanent film made of the composition according to the present embodiment is excellent in heat resistance and humidity resistance and furthermore, also has the excellent advantage that contamination by sublimable components is reduced. Particularly, for a display material, a material that achieves all of high sensitivity, high heat resistance, and hygroscopic reliability with reduced deterioration in image quality due to significant contamination can be obtained.

In the case of using the composition according to the present embodiment for resist permanent film purposes, a curing agent as well as, if required, various additive agents such as other resins, a surfactant, a dye, a filler, an acid crosslinking agent, and a dissolution promoting agent can be added and dissolved in an organic solvent to prepare a composition for resist permanent films.

The film forming composition for lithography or composition for resist permanent films according to the present embodiment can be prepared by adding each of the above components and mixing them using a stirrer or the like. When the composition for resist underlayer films or the composition for resist permanent films according to the present embodiment contains a filler or a pigment, it can be prepared by dispersion or mixing using a dispersion apparatus such as a dissolver, a homogenizer, and a three-roll mill.

### Example 1

The present embodiment will be described in more detail with reference to synthesis working examples, synthesis comparative examples, examples, and comparative examples below. However, the present embodiment is not limited to these examples by any means.

### <<Example A>>

### (Molecular weight)

The molecular weight of a compound was measured by LC-MS analysis using Acquity UPLC/MALDI-Synapt HDMS manufactured by Waters Corp.

Also, the weight average molecular weight (Mw), number average molecular weight (Mn), and dispersibility (Mw/Mn) in terms of polystyrene were determined by gel permeation chromatography (GPC) analysis under the following conditions.
Apparatus: Shodex GPC-101 model (manufactured by Showa Denko K.K.)
Column: KF-80M × 3
Eluent: 1 mL/min THF
Temperature: 40°C

### (Solubility)

A compound was dissolved at 3% by mass in propylene glycol monomethyl ether (PGME), cyclohexanone (CHN), ethyl lactate (EL), methyl amyl ketone (MAK) or tetramethylurea (TMU) by stirring at 23°C. Then, results about the solution obtained 1 week later were evaluated according to the following criteria.
Evaluation A: No precipitate was visually confirmed in any of the solvents.
Evaluation C: Precipitates were visually confirmed in all of the solvents.

### [Structure of compound]

The structure of a compound was confirmed by ¹H-NMR measurement using "Advance 600II spectrometer" manufactured by Bruker Corp. under the following conditions.
Frequency: 400 MHz
Solvent: d6-DMSO
Internal standard: TMS
Measurement temperature: 23°C

### <Synthesis Working Example 1A> Synthesis of BiP-1A

In a container (internal capacity: 300 mL) equipped with a stirrer, a condenser tube, and a burette, 19.7 g of 2,7-dihydroxynaphthalene (a reagent manufactured by Sigma-Aldrich), 10.0 g of benzo[b]thiophene-2-carboxaldehyde (a reagent manufactured by Sigma-Aldrich), and 3 g of concentrated sulfuric acid were added to 120 g of 1,4-dioxane, and the contents were reacted by being stirred at 110°C for 2 hours to obtain a reaction liquid. Then, the reaction liquid was added to 1.5 L of pure water, and the crystallized product was filtered off and dissolved in 500 mL of ethyl acetate. Next, the mixture was separated until neutral by the addition of pure water, and then concentrated to obtain a solution. The obtained solution was separated by column chromatography, and 11.0 g of the objective compound was then obtained.

As a result of measuring the molecular weight of the obtained compound by the above method, it was 446.

The following peaks were found by NMR measurement performed on the obtained compound under the above measurement conditions, and the compound was confirmed to have a chemical structure of the following formula (BiP-1A).
δ (ppm): 9.9 (2H, O-H), 7.0-7.8 (15H, Ph-H), 6.7 (1H, C-H)

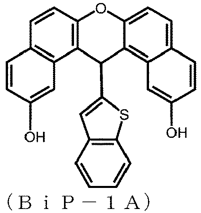

### <Synthesis Working Examples 2A to 9A> Synthesis of BiP-2A to BiP-9A

In the same manner as Synthesis Working Example 1A except that the aldehydes and the phenols listed in the following Table 1 were used instead of benzo[b]thiophene-2-carboxaldehyde and 2,7-dihydroxynaphthalene of Synthesis Working Example 1A, polyphenol compounds represented by the following formulas (BiP-2A) to (BiP-9A) were obtained.

### [Table 1]

**Table 1**

| Synthesis Working Example | Compound | Carbonyl compound | Phenol |
|---|---|---|---|
| 2A | BiP-2A | 4-Biphenylaldehyde | 4,4'-Thiodiphenol |
| 3A | BiP-3A | 4-(Methylthio)benzaldehyde | 2,7-Dihydroxynaphthalene |
| 4A | BiP-4A | Dibenzothiophene-2-carboxaldehyde | 2,7-Dihydroxynaphthalene |
| 5A | BiP-5A | 2-Acetylbenzo[b]thiophene | o-Phenylphenol |
| 6A | BiP-6A | Dibenzothiophene-2-carboxaldehyde | 2,7-Dihydroxynaphthalene |
| 7A | BiP-7A | 2-Thiophenecarboxaldehyde | 2,7-Dihydroxynaphthalene |
| 8A | BiP-8A | 3-Thiophenecarboxaldehyde | 2,7-Dihydroxynaphthalene |
| 9A | BiP-9A | Formyltetrathiafulvalene | 2,7-Dihydroxynaphthalene |

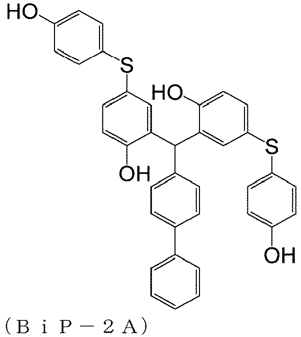

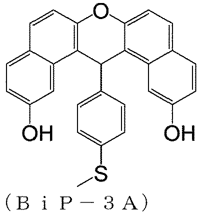

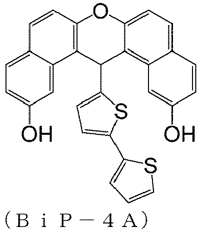

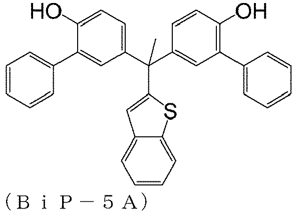

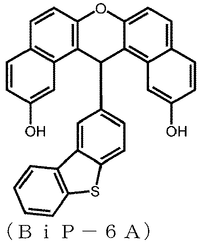

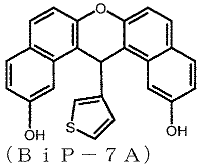

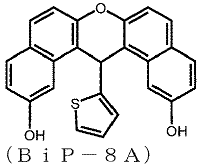

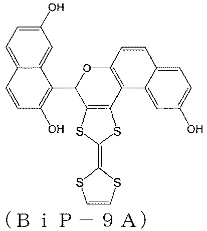

### <Synthesis Working Example 10A> Synthesis of BiP-1A-MeBOC

In a container (internal capacity: 200 mL) equipped with a stirrer, a condenser tube, and a burette, 5.5 g (12.4 mmol) of the compound (BiP-1A) obtained in the above Synthesis Working Example 1A and 5.4 g (27 mmol) of t-butyl bromoacetate (manufactured by Sigma-Aldrich) were fed to 100 mL of acetone, 3.8 g (27 mmol) of potassium carbonate (manufactured by Sigma-Aldrich) and 0.8 g of 18-crown-6 were added, and the contents were reacted by being stirred under reflux for 3 hours to obtain a reaction liquid. Next, the reaction liquid was concentrated, and the reaction product was precipitated by the addition of 100 g of pure water to the concentrate, cooled to room temperature, and then filtered to separate solid matter.

The obtained solid matter was filtered, dried, and then separated and purified by column chromatography, thereby obtaining 2.0 g of the objective compound (BiP-1A-MeBOC) represented by the following formula (BiP-1A-MeBOC).

As a result of measuring the molecular weight of the obtained compound (BiP-1A-MeBOC) by the above method, it was 674.

The following peaks were found by NMR measurement performed on the obtained compound (BiP-1A-MeBOC) under the above measurement conditions, and the compound was confirmed to have a chemical structure of the following formula (BiP-1A-MeBOC).
δ (ppm): 6.8-7.8 (15H, Ph-H), 6.7 (1H, C-H), 5.0 (4H, - CH2-), 1.4 (18H, -CH3)

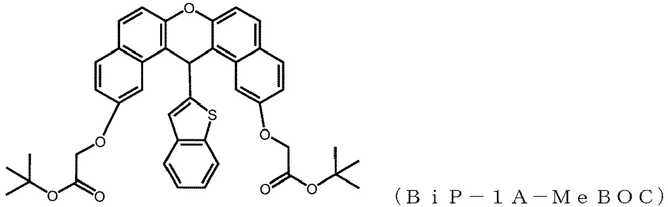

### <Synthesis Working Example 11A> Synthesis of BiP-1A-Prop

In a container (internal capacity: 300 mL) equipped with a stirrer, a condenser tube, and a burette, 8.9 g (19.9 mmol) of the compound (BiP-1A) obtained in the above Synthesis Working Example 1A and 7.9 g (66 mmol) of propargyl bromide were fed to 100 mL of dimethylformamide, and the contents were reacted by being stirred at room temperature for 3 hours to obtain a reaction liquid. Next, the reaction liquid was concentrated, and the reaction product was precipitated by the addition of 300 g of pure water to the concentrate, cooled to room temperature, and then filtered to separate solid matter.

The obtained solid matter was filtered, dried, and then separated and purified by column chromatography, thereby obtaining 6.0 g of the objective compound (BiP-1A-Prop) represented by the following formula (BiP-1A-Prop).

As a result of measuring the molecular weight of the obtained compound (BiP-1A-Prop) by the above method, it was 494.

The following peaks were found by NMR measurement performed on the obtained compound (BiP-1A-Prop) under the above measurement conditions, and the compound was confirmed to have a chemical structure of the following formula (BiP-1A-Prop).
δ (ppm): 6.8-7.8 (15H, Ph-H), 6.7 (1H, C-H), 2.1 (2H, ≡CH)

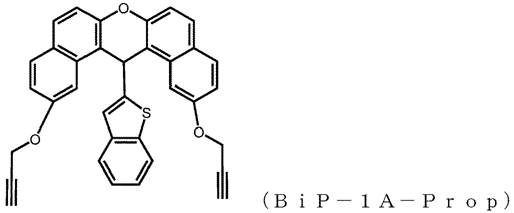

### <Synthesis Working Example 12A> Synthesis of R-BiP-2A

To a container (internal capacity: 300 mL) equipped with a stirrer, a condenser tube, and a burette, 10 g of BiP-2A obtained in Synthesis Working Example 2A, 0.3 g of sulfuric acid, 3.0 g of 4-biphenylaldehyde (a product manufactured by Mitsubishi Gas Chemical Company, Inc.), and 10 g of 1-methoxy-2-propanol were added, and the contents were reacted by being stirred at 90°C for 6 hours to obtain a reaction liquid. The reaction liquid was cooled, the insoluble matter was filtered off, 10 g of 1-methoxy-2-propanol was added, and the reaction product was then crystallized by hexane and collected by filtration. The collected product was dissolved in 100 mL of ethyl acetate (manufactured by Kanto Chemical Co., Inc.), and 50 mL of pure water was added thereto, followed by extraction with ethyl acetate. Next, the mixture was separated until neutral by the addition of pure water, and then dehydrated and concentrated to obtain a solution. The obtained solution was separated by column chromatography to obtain 1.0 g of the objective compound (R-BiP-2A) represented by the following formula (R-BiP-2A). 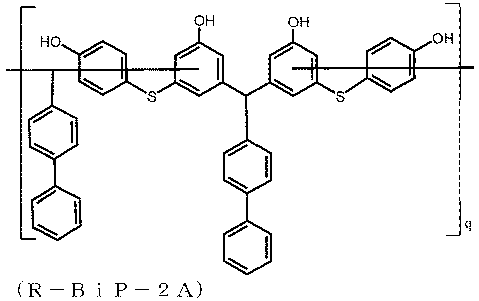 (In the formula (R-BiP-2A), q represents the number of repeat units.)

### <Synthesis Working Example 13A> Synthesis of R-BiP-3A

Through the same reaction as of Synthesis Working Example 12A except that BiP-3 obtained in Synthesis Working Example 3A was used instead of BiP-2A, 0.8 g of the objective resin (R-BiP-3A) represented by the following formula (R-BiP-3A) was obtained. 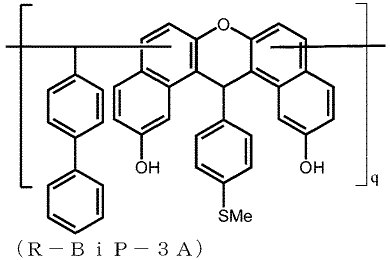 (In the formula (RBiP-3A), q represents the number of repeat units.)

### (Synthesis Working Example 14A) Synthesis of BiP-10A

In a container (internal capacity: 300 mL) equipped with a stirrer, a condenser tube, and a burette, after 12 g (69.0 mmol) of o-phenylphenol (a reagent manufactured by Sigma-Aldrich) was melted at 120°C, 0.27 g of sulfuric acid was added, and 2.7 g (13.8 mmol) of 4-acetylbiphenyl (a reagent manufactured by Sigma-Aldrich) was added, and the contents were reacted by being stirred at 120°C for 6 hours to obtain a reaction liquid. Next, 100 mL of N-methyl-2-pyrrolidone (manufactured by Kanto Chemical Co., Inc.) and 50 mL of pure water were added to the reaction liquid, followed by extraction with ethyl acetate. Next, the mixture was separated until neutral by the addition of pure water, and then concentrated to obtain a solution.

The obtained solution was separated by column chromatography to obtain 5.0 g of the objective compound (BiP-10A) represented by the following formula (BiP-10A).

As a result of measuring the molecular weight of the obtained compound (BiP-10A) by the above method, it was 518. Also, the carbon concentration was 88.0% by mass, and the oxygen concentration was 6.2% by mass.

The following peaks were found by NMR measurement performed on the obtained compound (BiP-10A) using 400 MHz-¹H-NMR with a solvent of DMSO-6, and the compound was confirmed to have a chemical structure of the following formula (BiP-10A).
δ (ppm) 9.48 (2H, O-H), 6.88-7.61 (25H, Ph-H), 3.36 (3H, C-H)

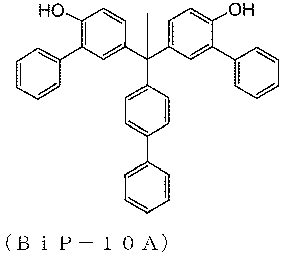

### (Synthesis Working Example 15A) Synthesis of SBiP-10A

In a container (internal capacity: 300 mL) equipped with a stirrer, a condenser tube, and a burette, BiP-10A obtained as described above was subjected to substitution reaction according to the method described in J. Am. Chem. Soc., Vol. 122, No. 28, 2000, thereby replacing a hydroxy group with a thiol group, and after separation by column chromatography, 1.0 g of the objective compound (SBiP-10A) represented by the following formula (SBiP-10A) was obtained.

As a result of measuring the molecular weight of the obtained compound (SBiP-10A) by the above method, it was 550. Also, the carbon concentration was 82.9% by mass.

The following peaks were found by NMR measurement performed on the obtained compound (SBiP-10A) using 400 MHz-¹H-NMR with a solvent of CDCl₃, and the compound was confirmed to have a chemical structure of the following formula (SBiP-10A).
δ (ppm) 3.40 (2H, S-H), 6.88-7.61 (25H, Ph-H), 3.36 (3H, C-H)

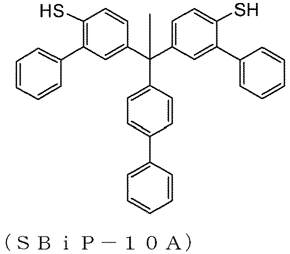

### (Synthesis Comparative Example 1A)

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 ml of 98% by mass of sulfuric acid (manufactured by Kanto Chemical Co., Inc.) were added in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction liquid, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

The molecular weight of the obtained dimethylnaphthalene formaldehyde resin was as follows: Mn: 562, Mw: 1168, and dispersity (Mw/Mn): 2.08.

Subsequently, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin obtained as described above, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was further added thereto, and the temperature was raised to 220°C at which the mixture was reacted for 2 hours. After solvent dilution, neutralization and washing with water were performed, and the solvent was removed under reduced pressure to obtain 126.1 g of a black-brown solid modified resin (CR-1).

The molecular weight of the obtained resin (CR-1) was as follows: Mn: 885, Mw: 2220, and dispersity (Mw/Mn): 4.17.

### <Synthesis Comparative Example 2A> Synthesis of BiP-C2

In the same manner as Synthesis Working Example 1A except that 4-methylbenzaldehyde and 2,6-dihydroxynaphthalene were used instead of benzo[b]thiophene-2-carboxaldehyde and 2,7-dihydroxynaphthalene of Synthesis Working Example 1A, a polyphenol compound represented by the following formula (BiP-C2) was obtained.

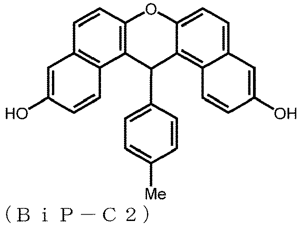

### (Examples 1A to 14A and Comparative Example 1A)

Solubility test was conducted using the compounds described in the above Synthesis Working Examples 1A to 13A and 15A, and CR-1 described in Synthesis Comparative Example 1A. The results are shown in Table 2.

Also, underlayer film forming materials for lithography were each prepared according to the composition shown in Table 2.

Next, a silicon substrate was spin coated with each of these underlayer film forming materials for lithography, and then baked at 240°C for 60 seconds and further at 400°C for 120 seconds to prepare each underlayer film with a film thickness of 200 nm. The following acid generating agent, acid crosslinking agent, and organic solvent were used.
Acid generating agent: di-tertiary butyl diphenyliodonium nonafluoromethanesulfonate (DTDPI) manufactured by Midori Kagaku Co., Ltd.
Acid crosslinking agent: NIKALAC MX270 (NIKALAC) manufactured by Sanwa Chemical Co., Ltd.
Organic solvent: propylene glycol monomethyl ether (PGME)

### (Examples 15A to 26A)

In addition, underlayer film forming materials for lithography were each prepared according to the composition shown in Table 3 below. Next, a silicon substrate was spin coated with each of these underlayer film forming materials for lithography, and then baked at 110°C for 60 seconds. After removal of the solvent from the coating film, the resulting film was cured at an integrated exposure amount of 600 mJ/cm² for an irradiation time of 20 seconds using a high pressure mercury lamp to prepare each underlayer film with a film thickness of 200 nm. The following photo radical polymerization initiator, acid crosslinking agent, and organic solvent were used.
Photo radical polymerization initiator: IRGACURE 184 manufactured by BASF SE
Acid crosslinking agent: NIKALAC MX270 (NIKALAC) manufactured by Sanwa Chemical Co., Ltd.
Organic solvent: propylene glycol monomethyl ether (PGME)

Then, etching test was conducted under conditions shown below to evaluate etching resistance. The evaluation results are shown in Table 2 and Table 3.

### [Etching test]

Etching apparatus: RIE-10NR manufactured by Samco International, Inc.
Output: 50 W
Pressure: 20 Pa
Time: 2 min
Etching gas
Ar gas flow rate:CF₄ gas flow rate:O₂ gas flow rate = 50:5:5 (sccm)

### [Evaluation of etching resistance]

The evaluation of etching resistance was conducted by the following procedures.

First, an underlayer film of novolac was prepared under the same conditions as in Example 1A except that novolac (PSM4357 manufactured by Gunei Chemical Industry Co., Ltd.) was used instead of the compound (Bip-1A). Then, this underlayer film of novolac was subjected to the above etching test, and the etching rate was measured.

Next, underlayer films of Examples and Comparative Example 1A were subjected to the above etching test in the same way as above, and the etching rate was measured.

Then, the etching resistance was evaluated according to the following evaluation criteria on the basis of the etching rate of the underlayer film of novolac.

### [Evaluation criteria]

A: The etching rate was less than -10% as compared with the underlayer film of novolac.
B: The etching rate was -10% to +5% as compared with the underlayer film of novolac.
C: The etching rate was more than +5% as compared with the underlayer film of novolac.

### [Table 2]

**Table 2**

| | No | Compound | Solubility | Composition of underlayer film forming material for lithography | | | | |
|---|---|---|---|---|---|---|---|---|
| | | | | Compound (parts by mass) | Solvent PGME (parts by mass) | Acid generating agent DTDPI (parts by mass) | Crosslinking agent NIKALAC (parts by mass) | Etching resistance |
| Example | 1A | BiP-1A | A | 10 | 180 | 0.5 | 0.5 | A |
| Example | 2A | BiP-2A | A | 10 | 180 | 0.5 | 0.5 | A |
| Example | 3A | BiP-3A | A | 10 | 180 | 0.5 | 0.5 | A |
| Example | 4A | BiP-4A | A | 10 | 180 | 0.5 | 0.5 | A |
| Example | 5A | BiP-5A | A | 10 | 180 | 0.5 | 0.5 | A |
| Example | 6A | BiP-6A | A | 10 | 180 | 0.5 | 0.5 | A |
| Example | 7A | BiP-7A | A | 10 | 180 | 0.5 | 0.5 | A |
| Example | 8A | BiP-8A | A | 10 | 180 | 0.5 | 0.5 | A |
| Example | 9A | BiP-9A | A | 10 | 180 | 0.5 | 0.5 | A |
| Example | 10A | BiP-1A-MeBOC | A | 10 | 180 | 0.5 | 0.5 | A |
| Example | 11A | BiP-1A-Prop | A | 10 | 180 | 0.5 | 0.5 | A |
| Example | 12A | R-BiP-2A | A | 10 | 180 | 0.5 | 0.5 | A |
| Example | 13A | R-BiP-3A | A | 10 | 180 | 0.5 | 0.5 | A |
| Example | 15A | SBiP-10A | A | 10 | 180 | 0.5 | 0.5 | A |
| Comparative Example | 1A | CR-1 | A | 10 | 180 | 0.5 | 0.5 | C |

### [Table 3]

**Table 3**

| | No | Compound (parts by mass) | Solvent (parts by mass) | Photo radical polymerization initiator (parts by mass) | Crosslinking agent (parts by mass) | Etching resistance |
|---|---|---|---|---|---|---|
| Example | 15A | BiP-1A(10) | PGME(180) | IRGACURE184(0.5) | NIKALAC (0.5) | A |
| Example | 16A | BiP-2A(10) | PGME(180) | IRGACURE184(0.5) | NIKALAC (0.5) | A |
| Example | 17A | BiP-3A(10) | PGME(180) | IRGACURE184(0.5) | NIKALAC (0.5) | A |
| Example | 18A | BiP-4A(10) | PGME(180) | IRGACURE184(0.5) | NIKALAC (0.5) | A |
| Example | 19A | BiP-5A(10) | PGME(180) | IRGACURE184(0.5) | NIKALAC (0.5) | A |
| Example | 20A | BiP-6A(10) | PGME(180) | IRGACURE184(0.5) | NIKALAC (0.5) | A |
| Example | 21A | BiP-7A(10) | PGME(180) | IRGACURE184(0.5) | NIKALAC (0.5) | A |
| Example | 22A | BiP-8A(10) | PGME(180) | IRGACURE184(0.5) | NIKALAC (0.5) | A |
| Example | 23A | BiP-9A(10) | PGME(180) | IRGACURE184(0.5) | NIKALAC (0.5) | A |
| Example | 24A | R-BiP-2A(10) | PGME(180) | IRGACURE184(0.5) | NIKALAC (0.5) | A |
| Example | 25A | R-BiP-3A(10) | PGME(180) | IRGACURE184(0.5) | NIKALAC (0.5) | A |
| Example | 26A | SBiP-10A(10) | PGME(180) | IRGACURE184(0.5) | NIKALAC (0.5) | A |

### (Examples 27A to 38A)

Next, a SiO₂ substrate with a film thickness of 300 nm was coated with each solution of the underlayer film forming material for lithography containing BiP-1A to BiP-9A, BiP-1A-Prop, R-BiP-2A to 3A or SBiP-10A, and baked at 240°C for 60 seconds and further at 400°C for 120 seconds to prepare each underlayer film with a film thickness of 70 nm. This underlayer film was coated with a resist solution for ArF and baked at 130°C for 60 seconds to form a photoresist layer with a film thickness of 140 nm. The ArF resist solution used was prepared by compounding 5 parts by mass of a compound of the formula (A) given below, 1 part by mass of triphenylsulfonium nonafluoromethanesulfonate, 2 parts by mass of tributylamine, and 92 parts by mass of PGMEA.

In order to prepare the compound of the formula (A), 4.15 g of 2-methyl-2-methacryloyloxyadamantane, 3.00 g of methacryloyloxy-y-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 ml of n-hexane. The product resin thus obtained was solidified and purified, and the resulting white powder was filtered and dried overnight at 40°C under reduced pressure, thereby obtaining the compound of the formula (A).

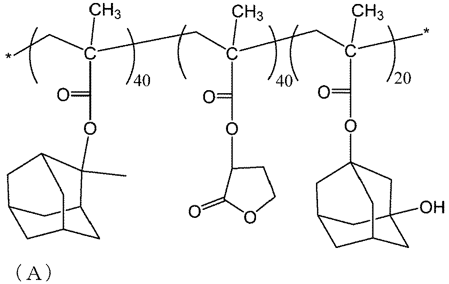

In the above formula (A), 40, 40, and 20 represent the ratio of each constituent unit, and do not mean that the compound is a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern.

The shapes of the resist patterns after development were evaluated as "goodness" when having good rectangularity without pattern collapse, and as "poorness" if this was not the case. The smallest line width having good rectangularity without pattern collapse as a result of this observation was used as an index for "resolution" evaluation. The smallest electron beam energy quantity capable of lithographing good pattern shapes was used as an index for "sensitivity" evaluation.

The evaluation results are shown in Table 4.

### (Comparative Example 2A)

The same operations as in Examples 27A to 39A were performed except that no underlayer film was formed so that a photoresist layer was formed directly on a SiO₂ substrate to obtain a positive type resist pattern. The results are shown in Table 4.

### [Table 4]

**Table 4**

| | No | Underlayer film forming material | Resolution (nm L/S) | Sensitivity (µC/cm²) | Resist pattern shape after development |
|---|---|---|---|---|---|
| Example | 27A | Material described in Example 1 | 45 | 10 | Good |
| Example | 28A | Material described in Example 2 | 45 | 10 | Good |
| Example | 29A | Material described in Example 3 | 45 | 10 | Good |
| Example | 30A | Material described in Example 4 | 45 | 10 | Good |
| Example | 31A | Material described in Example 5 | 45 | 10 | Good |
| Example | 32A | Material described in Example 6 | 45 | 10 | Good |
| Example | 33A | Material described in Example 7 | 45 | 10 | Good |
| Example | 34A | Material described in Example 8 | 45 | 10 | Good |
| Example | 35A | Material described in Example 9 | 45 | 10 | Good |
| Example | 36A | Material described in Example 11 | 45 | 10 | Good |
| Example | 37A | Material described in Example 12 | 45 | 10 | Good |
| Example | 38A | Material described in Example 13 | 45 | 10 | Good |
| Example | 39A | Material described in Example 14 | 45 | 10 | Good |
| Comparative Example | 2A | None | 80 | 26 | Poor |

As is evident from Tables 2 and 3, Examples 1A to 26A using the compound or the resin according to the present embodiment were confirmed to be good in terms of any of solubility and etching resistance. On the other hand, Comparative Example 1A using CR-1 (phenol-modified dimethylnaphthalene formaldehyde resin) resulted in poor etching resistance.

In addition, Examples 27A to 39A were confirmed to be good in terms of the resist pattern shape after development without any defect. These examples were confirmed to be significantly superior in both resolution and sensitivity to Comparative Example 2A in which underlayer film formation was omitted.

The difference in the resist pattern shapes after development demonstrated that the underlayer film forming materials for lithography used in Examples 27A to 39A have good adhesiveness to a resist material.

### (Examples 40A to 53A)

A SiO₂ substrate with a film thickness of 300 nm was coated with the solution of the underlayer film forming materials for lithography of Examples 1A to 14A, and baked at 240°C for 60 seconds and further at 400°C for 120 seconds to form each underlayer film with a film thickness of 80 nm. This underlayer film was coated with a silicon-containing intermediate layer material and baked at 200°C for 60 seconds to form an intermediate layer film with a film thickness of 35 nm. This intermediate layer film was further coated with the above resist solution for ArF and baked at 130°C for 60 seconds to form a photoresist layer with a film thickness of 150 nm. The silicon-containing intermediate layer material used was the silicon atom-containing polymer described in

### <Synthesis Example 1> of Japanese Patent Laid-Open No. 2007-226170.

Subsequently, the photoresist layer was mask exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a 55 nm L/S (1:1) positive type resist pattern.

Then, the silicon-containing intermediate layer film (SOG) was dry etched with the obtained resist pattern as a mask using RIE-10NR manufactured by Samco International, Inc. Subsequently, dry etching of the underlayer film with the obtained silicon-containing intermediate layer film pattern as a mask and dry etching of the SiO₂ substrate with the obtained underlayer film pattern as a mask were performed in order.

Respective etching conditions are as shown below.

### Conditions for etching of resist intermediate layer film with resist pattern

Output: 50 W
Pressure: 20 Pa
Time: 1 min
Etching gas
Ar gas flow rate:CF₄ gas flow rate:O₂ gas flow rate = 50:8:2 (sccm)

### Conditions for etching of resist underlayer film with resist intermediate film pattern

Output: 50 W
Pressure: 20 Pa
Time: 2 min
Etching gas
Ar gas flow rate:CF₄ gas flow rate:O₂ gas flow rate = 50:5:5 (sccm)

### Conditions for etching of SiO₂ substrate with resist underlayer film pattern

Output: 50 W
Pressure: 20 Pa
Time: 2 min
Etching gas
Ar gas flow rate:C₅F₁₂ gas flow rate:C₂F₆ gas flow rate:O₂ gas flow rate = 50:4:3:1 (sccm)

### [Evaluation]

The pattern cross section (the shape of the SiO₂ substrate after etching) obtained as described above was observed under an electron microscope manufactured by Hitachi, Ltd. (S-4800). As a result, it was confirmed that the shape of the SiO₂ substrate after etching in a multilayer resist process is a rectangular shape in Examples using the underlayer film of the present embodiment and is good without defects.

### (Examples 54A to 66A)

An optical component forming composition was prepared according to the recipe shown in Table 5 below using each compound synthesized in the above Synthesis Working Examples or the modified resin CR-1 synthesized in the above Synthesis Comparative Example. For the optical component forming compositions in Table 5, the following acid generating agent, acid crosslinking agent, and solvent were used.
Acid generating agent: di-tertiary butyl diphenyliodonium nonafluoromethanesulfonate (DTDPI) manufactured by Midori Kagaku Co., Ltd.
Acid crosslinking agent: NIKALAC MX270 (NIKALAC) manufactured by Sanwa Chemical Co., Ltd.
Organic solvent: propylene glycol monomethyl ether (PGMEA)

### [Evaluation of film formation]

A clean silicon wafer was spin coated with the homogeneous optical component forming composition, and then prebaked (PB) in an oven of 110°C to form an optical component forming film with a thickness of 1 µm. The prepared optical component forming composition was evaluated as "A" when it formed a good film, and as "C" when the formed film had defects.

### [Evaluation of refractive index and transmittance]

A clean silicon wafer was spin coated with the homogeneous optical component forming composition, and then prebaked (PB) in an oven of 110°C to form a film with a thickness of 1 µm. The refractive index (λ = 550 nm) of the film at 25°C was measured using a variable angle spectroscopic ellipsometer VASE manufactured by J. A. Woollam Co., Inc. The prepared film was evaluated as "A" when the refractive index was 1.70 or more, as "B" when the refractive index was 1.60 or more and less than 1.70, and as "C" when the refractive index was less than 1.60. Also, the film was evaluated as "A" when the transmittance (λ = 400 nm) was 90% or more, and as "C" when the transparency was less than 90%.

### [Table 5]

**Table 5**

| | No | Compound | Composition of optical component forming material | | | | Evaluation | | |
|---|---|---|---|---|---|---|---|---|---|
| | | | Compound (parts by mass) | Solvent PGME (parts by mass) | Acid generating agent DTDPI (parts by mass) | Crosslinking agent NIKALAC (parts by mass) | Film formation | Refractive index | Transmittance |
| Example | 54A | BiP-1A | 10 | 180 | 0.5 | 2 | A | A | A |
| Example | 55A | BiP-2A | 10 | 180 | 0.5 | 2 | A | A | A |
| Example | 56A | BiP-3A | 10 | 180 | 0.5 | 2 | A | A | A |
| Example | 57A | BiP-4A | 10 | 180 | 0.5 | 2 | A | A | A |
| Example | 58A | BiP-5A | 10 | 180 | 0.5 | 2 | A | A | A |
| Example | 59A | BiP-6A | 10 | 180 | 0.5 | 2 | A | A | A |
| Example | 60A | BiP-7A | 10 | 180 | 0.5 | 2 | A | A | A |
| Example | 61A | BiP-8A | 10 | 180 | 0.5 | 2 | A | A | A |
| Example | 62A | BiP-9A | 10 | 180 | 0.5 | 2 | A | A | A |
| Example | 63A | BiP-1A-Prop | 10 | 180 | 0.5 | 2 | A | A | A |
| Example | 64A | R-BiP-2A | 10 | 180 | 0.5 | 2 | A | A | A |
| Example | 65A | R-BiP-3A | 10 | 180 | 0.5 | 2 | A | A | A |
| Example | 66A | SBiP-10A | 10 | 180 | 0.5 | 2 | A | A | A |
| Comparative Example | 3A | CR-1 | 10 | 180 | 0.5 | 2 | A | C | C |
| Comparative Example | 4A | BiP-C2 | 10 | 180 | 0.5 | 2 | A | B | C |

### (Examples 67A to 79A)

Resist compositions were prepared according to the composition shown in Table 6 below using each of the compounds synthesized in the above Synthesis Working Examples. For the resist compositions in Table 6, the following radical generating agent, radical diffusion controlling agent, and solvent were used.
Radical generating agent: IRGACURE 184 manufactured by BASF SE
Radical diffusion controlling agent: IRGACURE 1010 manufactured by BASF SE
Organic solvent: propylene glycol monomethyl ether (PGME)

### [Evaluation method]

### (1) Storage stability and thin film formation of resist composition

The storage stability of the resist composition was evaluated by leaving the resist composition after preparation to stand still at 23°C and 50% RH for 3 days, and visually observing the presence or absence of precipitates. The resist composition after being left to stand still for 3 days was evaluated as "A" when it was a homogeneous solution without precipitates, and "C" when precipitates were present. A clean silicon wafer was spin coated with the homogeneous resist composition, and then prebaked (PB) before exposure in an oven of 110°C to form a resist film with a thickness of 40 nm. The prepared resist film was evaluated as "A" when the thin film formability was good, and "C" when the formed film had defects.

### (2) Pattern evaluation of resist pattern

A clean silicon wafer was spin coated with the homogeneous resist composition, and then prebaked (PB) before exposure in an oven of 110°C to form a resist film with a thickness of 60 nm. The obtained resist film was irradiated with electron beams of 1:1 line and space setting with 50 nm, 40 nm and 30 nm intervals using an electron beam lithography system (ELS-7500 manufactured by ELIONIX INC.). After the irradiation, the resist film was heated at each predetermined temperature for 90 seconds, and immersed in PGME for 60 seconds for development. Subsequently, the resist film was washed with ultrapure water for 30 seconds, and dried to form a negative type resist pattern. Concerning the formed resist pattern, the line and space were observed under a scanning electron microscope (S-4800 manufactured by Hitachi High-Technologies Corporation) to evaluate the reactivity by electron beam irradiation of the resist composition.

The sensitivity was indicated by the smallest energy quantity per unit area necessary for obtaining patterns, and evaluated according to the following criteria.
A: when the pattern was obtained at less than 50 µC/cm²
C: when the pattern was obtained at 50 µC/cm² or more.

As for pattern formation, the obtained pattern shape was observed under SEM (scanning electron microscope), and evaluated according to the following criteria.
A: When a rectangular pattern was obtained
B: When an almost rectangular pattern was obtained
C: When a non-rectangular pattern was obtained

### [Table 6]

**Table 6**

| | No | Compound | Composition of optical component forming material | | | | Evaluation | | | |
|---|---|---|---|---|---|---|---|---|---|---|
| | | | Compound (parts by mass) | Solvent PGME (parts by mass) | Radical generating agent (parts by mass) | Radical diffusion controlling agent (parts by mass) | Storage stability | Film formation | Sensitivity | Pattern formation |
| Example | 67A | BiP-1A | 1 | 50 | 0.1 | 0.01 | A | A | A | A |
| Example | 68A | BiP-2A | 1 | 50 | 0.1 | 0.01 | A | A | A | A |
| Example | 69A | BiP-3A | 1 | 50 | 0.1 | 0.01 | A | A | A | A |
| Example | 70A | BiP-4A | 1 | 50 | 0.1 | 0.01 | A | A | A | A |
| Example | 71A | BiP-5A | 1 | 50 | 0.1 | 0.01 | A | A | A | A |
| Example | 72A | BiP-6A | 1 | 50 | 0.1 | 0.01 | A | A | A | A |
| Example | 73A | BiP-7A | 1 | 50 | 0.1 | 0.01 | A | A | A | A |
| Example | 74A | BiP-8A | 1 | 50 | 0.1 | 0.01 | A | A | A | A |
| Example | 75A | BiP-9A | 1 | 50 | 0.1 | 0.01 | A | A | A | A |
| Example | 76A | BiP-1A-Prop | 1 | 50 | 0.1 | 0.01 | A | A | A | A |
| Example | 77A | R-BiP-2A | 1 | 50 | 0.1 | 0.01 | A | A | A | A |
| Example | 78A | R-BiP-3A | 1 | 50 | 0.1 | 0.01 | A | A | A | A |
| Example | 79A | SBiP-10A | 1 | 50 | 0.1 | 0.01 | A | A | A | A |

### <<Example B>>

### (Carbon concentration and oxygen concentration)

The carbon concentration and the oxygen concentration (% by mass) were measured by organic elemental analysis.

Apparatus: CHN Coder MT-6 (manufactured by Yaic. Yanaco)

### (Molecular weight)

The molecular weight of a compound was measured by LC-MS analysis using Acquity UPLC/MALDI-Synapt HDMS manufactured by Waters Corp.

### (Solubility)

A compound was dissolved at 5% by mass in propylene glycol monomethyl ether (PGME) at 23°C, and the solution was then left for 30 days at 5°C. The results were evaluated according to the following criteria.
Evaluation A: no precipitate was visually confirmed
Evaluation C: precipitates were visually confirmed

### (Synthesis Example 1B) Synthesis of BiP-1B

In a container (internal capacity: 300 mL) equipped with a stirrer, a condenser tube, and a burette, after 12 g (69.0 mmol) of o-phenylphenol (a reagent manufactured by Sigma-Aldrich) was melted at 120°C, 0.27 g of sulfuric acid was added, and 2.7 g (13.8 mmol) of 4-acetylbiphenyl (a reagent manufactured by Sigma-Aldrich) was added, and the contents were reacted by being stirred at 120°C for 6 hours to obtain a reaction liquid. Next, 100 mL of N-methyl-2-pyrrolidone (manufactured by Kanto Chemical Co., Inc.) and 50 mL of pure water were added to the reaction liquid, followed by extraction with ethyl acetate. Next, the mixture was separated until neutral by the addition of pure water, and then concentrated to obtain a solution.

The obtained solution was separated by column chromatography to obtain 5.0 g of the objective compound (BiP-1B) represented by the following formula (BiP-1B).

As a result of measuring the molecular weight of the obtained compound (BiP-1B) by the above method, it was 518. Also, the carbon concentration was 88.0% by mass, and the oxygen concentration was 6.2% by mass.

The following peaks were found by NMR measurement performed on the obtained compound (BiP-1B) using 400 MHz-¹H-NMR with a solvent of DMSO-6, and the compound was confirmed to have a chemical structure of the following formula (BiP-1B).
δ (ppm) 9.48 (2H, O-H), 6.88-7.61 (25H, Ph-H), 3.36 (3H, C-H)

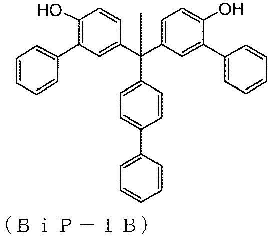

### (Synthesis Example 2B) Synthesis of SBiP-1B

In a container (internal capacity: 300 mL) equipped with a stirrer, a condenser tube, and a burette, BiP-1B obtained as described above was subjected to substitution reaction according to the method described in J. Am. Chem. Soc., Vol. 122, No. 28, 2000, thereby replacing a hydroxy group with a thiol group, and after separation by column chromatography, 1.0 g of the objective compound (SBiP-1B) represented by the following formula (SBiP-1B) was obtained.

As a result of measuring the molecular weight of the obtained compound (SBiP-1B) by the above method, it was 550. Also, the carbon concentration was 82.9% by mass.

The following peaks were found by NMR measurement performed on the obtained compound (SBiP-1B) using 400 MHz-¹H-NMR with a solvent of CDCl₃, and the compound was confirmed to have a chemical structure of the following formula (SBiP-1B).
δ (ppm) 3.40 (2H, S-H), 6.88-7.61 (25H, Ph-H), 3.36 (3H, C-H)

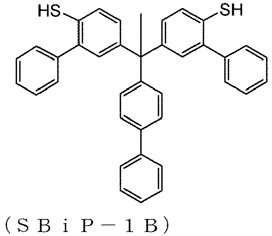

### (Synthesis Example 3B)

A four necked flask (internal capacity: 10 L) equipped with a Dimroth condenser tube, a thermometer, and a stirring blade and having a detachable bottom was prepared. To this four necked flask, 1.09 kg (7 mol) of 1,5-dimethylnaphthalene (manufactured by Mitsubishi Gas Chemical Company, Inc.), 2.1 kg (28 mol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were added in a nitrogen stream, and the mixture was reacted for 7 hours while refluxed at 100°C at normal pressure. Subsequently, 1.8 kg of ethylbenzene (special grade reagent manufactured by Wako Pure Chemical Industries, Ltd.) was added as a diluting solvent to the reaction liquid, and the mixture was left to stand still, followed by removal of an aqueous phase as a lower phase. Neutralization and washing with water were further performed, and ethylbenzene and unreacted 1,5-dimethylnaphthalene were distilled off under reduced pressure to obtain 1.25 kg of a light brown solid dimethylnaphthalene formaldehyde resin.

The molecular weight of the obtained dimethylnaphthalene formaldehyde was as follows: Mn: 562, Mw: 1168, and Mw/Mn: 2.08.

Subsequently, a four necked flask (internal capacity: 0.5 L) equipped with a Dimroth condenser tube, a thermometer, and a stirring blade was prepared. To this four necked flask, 100 g (0.51 mol) of the dimethylnaphthalene formaldehyde resin obtained as described above, and 0.05 g of p-toluenesulfonic acid were added in a nitrogen stream, and the temperature was raised to 190°C at which the mixture was then heated for 2 hours, followed by stirring. Subsequently, 52.0 g (0.36 mol) of 1-naphthol was further added thereto, and the temperature was further raised to 220°C at which the mixture was reacted for 2 hours. After dilution with a solvent, neutralization and washing with water were performed, and the solvent was distilled off under reduced pressure to obtain 126.1 g of a modified resin (CR-1) as a black-brown solid.

The molecular weight of the obtained resin (CR-1) was Mn: 885, Mw: 2220, and Mw/Mn: 4.17. Also, the carbon concentration was 89.1% by mass, and the oxygen concentration was 4.5% by mass.

### [Examples 1B and 2B, and Comparative Example 1B]

Solubility test was conducted for the above SBiP-1B and CR-1. The results are shown in Table 7.

Also, underlayer film forming materials for lithography were each prepared according to the composition shown in Table 7. Next, a silicon substrate was spin coated with each of these underlayer film forming materials for lithography, and then baked at 240°C for 60 seconds and further at 400°C for 120 seconds to prepare each underlayer film with a film thickness of 200 nm. The following acid generating agent, crosslinking agent, and organic solvent were used.
Acid generating agent: di-tertiary butyl diphenyliodonium nonafluoromethanesulfonate (DTDPI) manufactured by Midori Kagaku Co., Ltd.
Crosslinking agent: NIKALAC MX270 (NIKALAC) (Sanwa Chemical Co., Ltd.)
Organic solvent: propylene glycol monomethyl ether acetate (PGMEA)

Then, etching test was conducted under conditions shown below to evaluate etching resistance. The evaluation results are shown in Table 7.

### [Etching test]

Etching apparatus: RIE-10NR manufactured by Samco International, Inc.
Output: 50 W
Pressure: 20 Pa
Time: 2 min
Etching gas
Ar gas flow rate:CF₄ gas flow rate:O₂ gas flow rate = 50:5:5 (sccm)

### [Evaluation of etching resistance]

The evaluation of etching resistance was conducted by the following procedures.

First, an underlayer film of novolac was prepared under the same conditions as in Example 1B except that novolac (PSM4357 manufactured by Gunei Chemical Industry Co., Ltd.) was used instead of the compound (SBip-1B) used in Example 1B. Then, this underlayer film of novolac was subjected to the above etching test, and the etching rate was measured.

Next, underlayer films of Examples 1B and 2B, and Comparative Example 1B were subjected to the above etching test in the same way as above, and the etching rate was measured.

Then, the etching resistance was evaluated according to the following evaluation criteria on the basis of the etching rate of the underlayer film of novolac.

### [Evaluation criteria]

A: The etching rate was less than -10% as compared with the underlayer film of novolac.
B: The etching rate was -10% to +5% as compared with the underlayer film of novolac.
C: The etching rate was more than +5% as compared with the underlayer film of novolac.

### [Table 7]

**Table 7**

| | Underlayer film forming material | | | | Evaluation of solubility | Evaluation of etching resistance |
|---|---|---|---|---|---|---|
| | Compound or resin (parts by mass) | Organic solvent (parts by mass) | Acid generating agent (parts by mass) | Crosslinking agent (parts by mass) | | |
| Example 1B | SBiP-1B (10) | PGMEA (90) | - | - | A | A |
| Example 2B | SBiP-1B (10) | PGMEA (90) | DTDPI (0.5) | NIKALAC (0.5) | A | A |
| Comparative Example 1B | CR-1 (10) | PGMEA (90) | DTDPI (0.5) | NIKALAC (0.5) | A | C |

### [Examples 3B and 4B]

Next, a SiO₂ substrate with a film thickness of 300 nm was coated with each of the solutions of the underlayer film forming materials for lithography containing SBiP-1B of Examples 1B and 2B, and baked at 240°C for 60 seconds and further at 400°C for 120 seconds to form each underlayer film with a film thickness of 70 nm. This underlayer film was coated with a resist solution for ArF and baked at 130°C for 60 seconds to form a photoresist layer with a film thickness of 140 nm. The ArF resist solution used was prepared by compounding 5 parts by mass of a compound of the formula (11) given below, 1 part by mass of triphenylsulfonium nonafluoromethanesulfonate, 2 parts by mass of tributylamine, and 92 parts by mass of PGMEA.

In order to prepare the compound of the formula (11), 4.15 g of 2-methyl-2-methacryloyloxyadamantane, 3.00 g of methacryloyloxy-γ-butyrolactone, 2.08 g of 3-hydroxy-1-adamantyl methacrylate, and 0.38 g of azobisisobutyronitrile were dissolved in 80 mL of tetrahydrofuran to prepare a reaction solution. This reaction solution was polymerized for 22 hours with the reaction temperature kept at 63°C in a nitrogen atmosphere. Then, the reaction solution was added dropwise into 400 mL of n-hexane. The product resin thus obtained was solidified and purified, and the resulting white powder was filtered and dried overnight at 40°C under reduced pressure, thereby obtaining the compound of the formula (11).

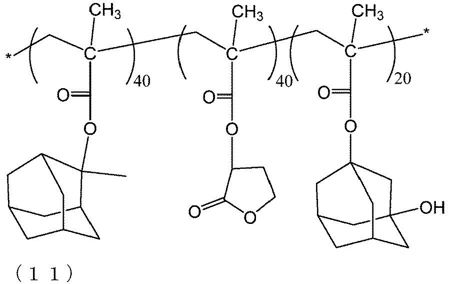

In the above formula (11), 40, 40, and 20 represent the molar ratio of each constituent unit, and do not mean that the compound is a block copolymer.

Subsequently, the photoresist layer was exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a positive type resist pattern.

The shape and defects of the obtained resist patterns of 55 nm L/S (1:1) and 80 nm L/S (1:1) were observed, and the results are shown in Table 8.

### [Comparative Example 2B]

The same operations as in Example 3B were performed except that no underlayer film is formed so that a photoresist layer was formed directly on a SiO₂ substrate to obtain a positive type resist pattern. The results are shown in Table 8.

### [Table 8]

**Table 8**

| | Underlayer film forming material | Resolution (nmL/S) | Sensitivity (µC/cm²) | Resist pattern shape after development |
|---|---|---|---|---|
| Example 3B | Material described in Example 1B | 55 | 10 | Good |
| Example 4B | Material described in Example 2B | 55 | 10 | Good |
| Comparative Example 2B | - | 80 | 26 | Poor |

As is evident from Table 7, Examples 1B and 2B using the compound SBiP-1B of the present embodiment were confirmed to be good in terms of any of solubility and etching resistance. On the other hand, Comparative Example 1B using CR-1 (phenol-modified dimethylnaphthalene formaldehyde resin) resulted in poor etching resistance.

In addition, Examples 3B and 4B were confirmed to be good in terms of the resist pattern shape after development without any defect. Examples 3B and 4B were confirmed to be significantly superior in both resolution and sensitivity to Comparative Example 2B in which underlayer film formation was omitted.

The difference in the resist pattern shapes after development demonstrated that the underlayer film forming materials for lithography used in Examples 3B and 4B have good adhesiveness to a resist material.

### [Examples 5B and 6B]

A SiO₂ substrate with a film thickness of 300 nm was coated with the solution of the underlayer film forming materials for lithography of Examples 1B and 2B, and baked at 240°C for 60 seconds and further at 400°C for 120 seconds to form each underlayer film with a film thickness of 80 nm. This underlayer film was coated with a silicon-containing intermediate layer material and baked at 200°C for 60 seconds to form an intermediate layer film with a film thickness of 35 nm. This intermediate layer film was further coated with the above resist solution for ArF and baked at 130°C for 60 seconds to form a photoresist layer with a film thickness of 150 nm. The silicon-containing intermediate layer material used was the silicon atom-containing polymer described in

### <Synthesis Example 1> of Japanese Patent Laid-Open No. 2007-226170.

Subsequently, the photoresist layer was mask exposed using an electron beam lithography system (manufactured by ELIONIX INC.; ELS-7500, 50 keV), baked (PEB) at 115°C for 90 seconds, and developed for 60 seconds in 2.38% by mass tetramethylammonium hydroxide (TMAH) aqueous solution to obtain a 55 nm L/S (1:1) positive type resist pattern.

Then, the silicon-containing intermediate layer film (SOG) was dry etched with the obtained resist pattern as a mask using RIE-10NR manufactured by Samco International, Inc. Subsequently, dry etching of the underlayer film with the obtained silicon-containing intermediate layer film pattern as a mask and dry etching of the SiO₂ substrate with the obtained underlayer film pattern as a mask were performed in order.

Respective etching conditions are as shown below.

### Conditions for etching of resist intermediate layer film with resist pattern

Output: 50 W
Pressure: 20 Pa
Time: 1 min
Etching gas
Ar gas flow rate:CF₄ gas flow rate:O₂ gas flow rate = 50:8:2 (sccm)

### Conditions for etching of resist underlayer film with resist intermediate film pattern

Output: 50 W
Pressure: 20 Pa
Time: 2 min
Etching gas
Ar gas flow rate:CF₄ gas flow rate:O₂ gas flow rate = 50:5:5 (sccm)

### Conditions for etching of SiO₂ substrate with resist underlayer film pattern

Output: 50 W
Pressure: 20 Pa
Time: 2 min
Etching gas
Ar gas flow rate:C₅F₁₂ gas flow rate:C₂F₆ gas flow rate:O₂ gas flow rate = 50:4:3:1 (sccm)

### [Evaluation]

The pattern cross section (the shape of the SiO₂ substrate after etching) obtained as described above was observed under an electron microscope manufactured by Hitachi, Ltd. (S-4800). As a result, it was confirmed that the shape of the SiO₂ substrate after etching in a multilayer resist process is a rectangular shape in Examples using the underlayer film of the present embodiment and is good without defects.

### [Examples 7B and 8B]

A SiO₂ substrate with a film thickness of 300 nm was coated with the solution of the optical component forming compositions having the same composition as the underlayer film forming materials for lithography of Examples 1B and 2B, and baked at 260°C for 300 seconds to form each optical component forming film with a film thickness of 100 nm.

Then, test for the refractive index at a wavelength of 550 nm and the transparency at a wave length of 400 nm was conducted using a vacuum ultraviolet with variable angle spectroscopic ellipsometer (VUV-VASE) manufactured by J.A. Woollam Japan, and the refractive index and transparency were evaluated according to the following criteria.

### [Evaluation criteria for refractive index]

A: the refractive index is 1.65 or more
C: the refractive index is less than 1.65

### [Evaluation criteria for transparency]

A: the absorption coefficient is less than 0.03
C: the absorption coefficient is 0.03 or more

As a result, both Examples 7B and 8B were confirmed to have a refractive index of A and a transparency of A, and be useful as an optical component forming composition.

### [Example 9B and Comparative Example 3B]

The compound represented by the above formula (SBiP-1B) or the following formula (BiP-C2) was dissolved at 5% in PGMEA. Next, a SiO₂ substrate with a film thickness of 300 nm was coated with the above solution, and baked at 260°C for 300 seconds to form a film with a film thickness of 100 nm.

Then, test for the refractive index at a wavelength of 550 nm and the transparency at a wave length of 400 nm was conducted using a vacuum ultraviolet with variable angle spectroscopic ellipsometer (VUV-VASE) manufactured by J.A. Woollam Japan, and the refractive index and transparency were evaluated according to the following criteria.

### [Evaluation criteria for refractive index]

A: the refractive index is 1.65 or more
C: the refractive index is less than 1.65
[Evaluation criteria for transparency]
A: the absorption coefficient is less than 0.03
C: the absorption coefficient is 0.03 or more

As a result, when the compound represented by the above formula (SBiP-1B) was used, both refractive index and transparency were A, but when the compound represented by the above formula (BiP-C2) was used, the refractive index was A and the transparency was C. The compound represented by the formula (SBiP-1B) was confirmed to be excellent in coloration resistance.

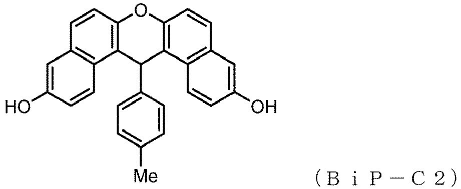

As mentioned above, the present invention is not limited to the above embodiments and examples, and changes or modifications can be arbitrarily made without departing from the spirit of the present invention.

The compound and the resin of the present embodiment have high solubility in a safe solvent and have good heat resistance and etching resistance. The resist composition of the present embodiment imparts a good shape to a resist pattern.

Also, the compound and the resin of the present embodiment are applicable to a wet process and can achieve a compound, a resin, and a film forming composition for lithography useful for forming a photoresist underlayer film excellent in heat resistance and etching resistance. Furthermore, this film forming composition for lithography employs the compound or the resin having high heat resistance and also high solvent solubility and having a specific structure and can therefore form a resist and an underlayer film that is prevented from deteriorating during high temperature baking and is also excellent in etching resistance against oxygen plasma etching or the like. In addition, the underlayer film thus formed is also excellent in adhesiveness to a resist layer and can therefore form an excellent resist pattern.

Moreover, the composition of the present embodiment has high refractive index and is prevented from being stained by low temperature to high temperature treatments. Therefore, the composition is also useful as various optical component forming compositions.

Accordingly, the present invention is used in for example, electrical insulating materials, resins for resists, encapsulation resins for semiconductors, adhesives for printed circuit boards, electrical laminates mounted in electric equipment, electronic equipment, industrial equipment, and the like, matrix resins of prepregs mounted in electric equipment, electronic equipment, industrial equipment, and the like, buildup laminate materials, resins for fiber-reinforced plastics, resins for encapsulation of liquid crystal display panels, coating materials, various coating agents, adhesives, coating agents for semiconductors, resins for resists for semiconductors, resins for underlayer film formation, and in the form of a film or a sheet, and additionally, can be used widely and effectively in optical components such as plastic lenses (prism lenses, lenticular lenses, microlenses, Fresnel lenses, viewing angle control lenses, contrast improving lenses, etc.), phase difference films, films for electromagnetic wave shielding, prisms, optical fibers, solder resists for flexible printed wiring, plating resists, interlayer insulating films for multilayer printed circuit boards, and photosensitive optical waveguides.

### Industrial applicability

The present invention has industrial applicability in the fields of resists for lithography, underlayer films for lithography, underlayer films for multilayer resists, and optical components.

## Claims

1. A compound represented by the following formula (1) or (1'): 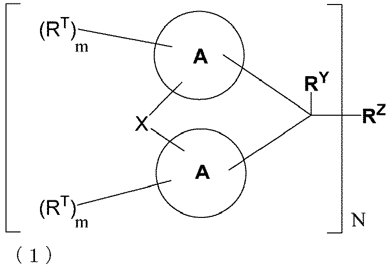 wherein
R^{Y} is a hydrogen atom, an alkyl group having 1 to 60 carbon atoms and optionally having a substituent and/or a heteroatom, or an aryl group having 6 to 60 carbon atoms and optionally having a substituent and/or a heteroatom;
R^{Z} is an N-valent group having 1 to 60 carbon atoms and optionally having a substituent and/or a heteroatom, or a single bond;
or alternatively, R^{Y} and R^{Z} may form, including a carbon atom to which they are bonded, a 4-membered to 30-membered ring optionally having a substituent and/or a heteroatom;
A is a group exhibiting aromaticity and having 1 to 60 carbon atoms and optionally having a substituent and/or a heteroatom;
each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a carboxylic acid group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond; and
X is an oxygen atom, a sulfur atom or not a crosslink;
wherein
at least one selected from A, R^{Y}, R^{Z}, R^{T} and X contains a sulfur atom;
at least one R^{T} contains a thiol group, a hydroxy group, or a group in which a hydrogen atom of a thiol group or a hydroxy group is replaced with an acid crosslinking group or an acid dissociation group;
each m is independently an integer of 0 to 9; and
N is an integer of 1 to 4, wherein when N is an integer of 2 or larger, N structural formulas within the parentheses [ ] are the same or different, or 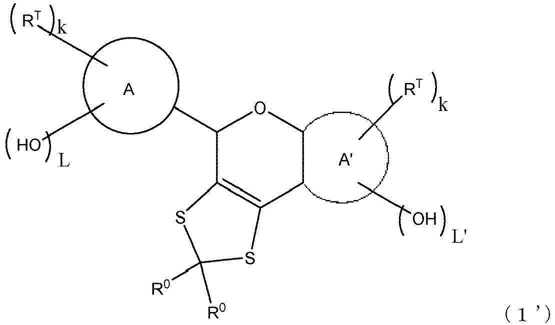 wherein
each R^{T} is independently an alkyl group having 1 to 30 carbon atoms and optionally having a substituent and/or a heteroatom, an aryl group having 6 to 40 carbon atoms and optionally having a substituent and/or a heteroatom, an alkenyl group having 2 to 30 carbon atoms and optionally having a substituent and/or a heteroatom, an alkynyl group having 2 to 30 carbon atoms and optionally having a substituent and/or a heteroatom, an alkoxy group having 1 to 30 carbon atoms and optionally having a substituent and/or a heteroatom, an alkylthio group having 1 to 30 carbon atoms and optionally having a substituent and/or a heteroatom, a halogen atom, a nitro group, an amino group, a cyano group, a thiol group, a hydroxy group, or a group in which a hydrogen atom of a thiol group or a hydroxy group is replaced with an acid crosslinking group or an acid dissociation group, wherein the alkyl group, the alkenyl group, the alkynyl group and the aryl group each optionally contain an ether bond, a ketone bond or an ester bond;
each R⁰ is independently a hydrogen atom, an alkyl group having 1 to 30 carbon atoms and optionally having a substituent and/or a heteroatom, or an aryl group having 6 to 30 carbon atoms and optionally having a substituent and/or a heteroatom;
or alternatively, two R⁰ may form, including a carbon atom to which they are bonded, a 4-membered to 30-membered ring optionally having a substituent and/or a heteroatom; or two R⁰ are a double bond bonded to a carbon atom to which they are bonded, wherein an alkyl group having 1 to 30 carbon atoms and optionally having a substituent and/or a heteroatom, or an aryl group having 6 to 30 carbon atoms and optionally having a substituent and/or a heteroatom may be bonded to the double bond;
A and A' are each a group exhibiting aromaticity and having 1 to 60 carbon atoms and optionally having a substituent and/or a heteroatom;
L is an integer of 1 to 9; and
k and L' are each independently an integer of 0 to 9.

2. The compound according to claim 1, wherein the compound represented by the above formula (1) is a compound represented by the following formula (1-1): 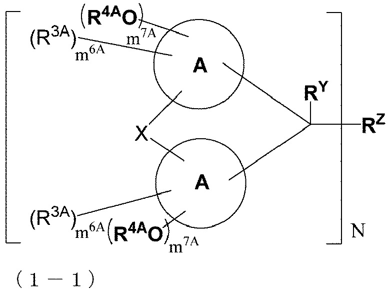 wherein
R^{Y}, R^{Z}, A, X, and N are as defined in claim 1;
each R^{3A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, a halogen atom, a nitro group, an amino group, a carboxylic acid group, or a thiol group; and
each R^{4A} is independently a hydrogen atom, an acid crosslinking group, or an acid dissociation group;
wherein, at least one selected from A, R^{Y}, R^{Z}, R^{3A}, R^{4A}, and X contains a sulfur atom;
each m^{6A} is independently an integer of 0 to 5; and
each m^{7A} is independently an integer of 0 to 5,
provided that two m^{7A} are not 0 at the same time.

3. The compound according to claim 1, wherein the compound represented by the above formula (1) is a compound represented by the following formula (2): 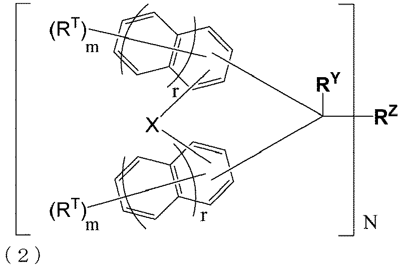 wherein
R^{Y} is a hydrogen atom, an alkyl group having 1 to 30 carbon atoms and optionally having a substituent, or an aryl group having 6 to 30 carbon atoms and optionally having a substituent;
R^{Z} is an N-valent group having 1 to 60 carbon atoms or a single bond;
each R^{T} is independently an alkyl group having 1 to 30 carbon atoms and optionally having a substituent, an aryl group having 6 to 40 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a nitro group, an amino group, a cyano group, a thiol group, a hydroxy group, or a group in which a hydrogen atom of a hydroxy group is replaced with an acid dissociation group, wherein the alkyl group, the alkenyl group, the alkynyl group and the aryl group each optionally contain an ether bond, a ketone bond or an ester bond and wherein at least one R^{T} is a thiol group;
X is an oxygen atom, a sulfur atom or not a crosslink;
each m is independently an integer of 0 to 9, wherein at least one m is an integer of 1 to 9;
N is an integer of 1 to 4, wherein when N is an integer of 2 or larger, N structural formulas within the parentheses [ ] are the same or different; and
each r is independently an integer of 0 to 2.

4. The compound according to claim 3, wherein the compound represented by the above formula (2) is a compound represented by the following formula (3): 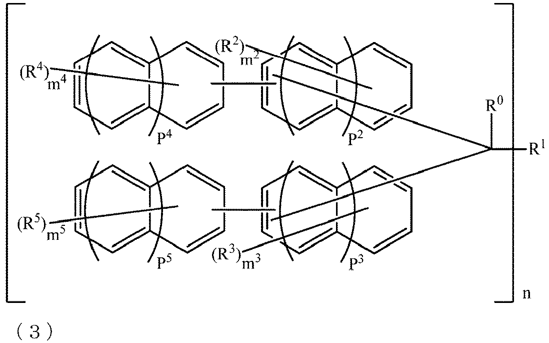 wherein
R⁰ is as defined in R^{Y} in claim 3;
R¹ is an n-valent group having 1 to 60 carbon atoms or a single bond;
R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one of R² to R⁵ is a thiol group;
m² and m³ are each independently an integer of 0 to 8;
m⁴ and m⁵ are each independently an integer of 0 to 9,
provided that m², m³, m⁴, and m⁵ are not 0 at the same time;
n is as defined in N in claim 3, wherein when n is an integer of 2 or larger, n structural formulas within the parentheses [ ] are the same or different; and
p² to p⁵ are as defined in r in claim 3.

5. The compound according to claim 3, wherein the compound represented by the above formula (2) is a compound represented by the following formula (4): 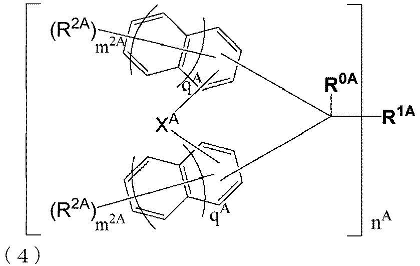 wherein
R^{0A} is as defined in R^{Y} in claim 3;
R^{1A} is an n^{A}-valent group having 1 to 60 carbon atoms or a single bond;
each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one R^{2A} is a thiol group;
n^{A} is as defined in N in claim 3, where when n^{A} is an integer of 2 or larger, n^{A} structural formulas within the parentheses [ ] are the same or different;
X^{A} is an oxygen atom, a sulfur atom or not a crosslink;
each m^{2A} is independently an integer of 0 to 7, provided that at least one m^{2A} is an integer of 1 to 7; and
each q^{A} is independently 0 or 1.

6. The compound according to claim 4, wherein the compound represented by the above formula (3) is a compound represented by the following formula (3-1): 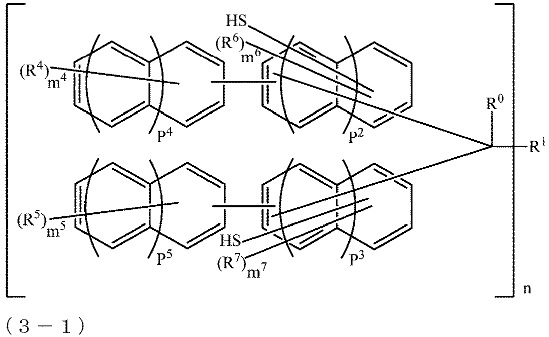 wherein
R⁰, R¹, R⁴, R⁵, n, p² to p⁵, m⁴, and m⁵ are as defined in claim 4;
R⁶ and R⁷ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, or a thiol group; and
m⁶ and m⁷ are each independently an integer of 0 to 7.

7. The compound according to claim 6, wherein the compound represented by the above formula (3-1) is a compound represented by the following formula (3-2): 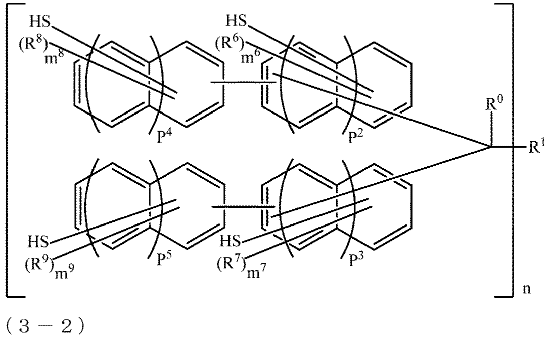 wherein
R⁰, R¹, n, and p² to p⁵ are as defined in claim 4;
R⁶, R⁷, m⁶, and m⁷ are as defined in claim 6;
R⁸ and R⁹ are as defined in R⁶ and R⁷; and
m⁸ and m⁹ are each independently an integer of 0 to 8.

8. The compound according to claim 5, wherein the compound represented by the above formula (4) is a compound represented by the following formula (4-1): 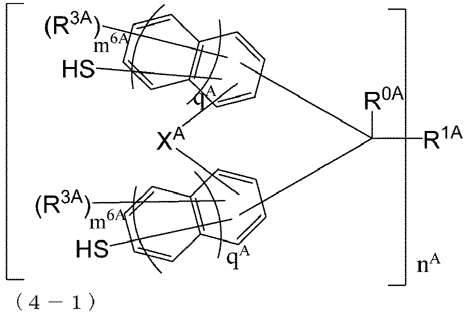 wherein
R^{0A}, R^{1A}, n^{A}, q^{A}, and X^{A} are as defined in claim 5;
each R^{3A} is independently a halogen atom, an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, or an alkoxy group having 1 to 30 carbon atoms and optionally having a substituent; and
each m^{6A} is independently an integer of 0 to 5.

9. A resin comprising the compound according to claim 1 or 2 as a constituent unit.

10. The resin according to claim 9, wherein the resin is represented by the following formula (5): 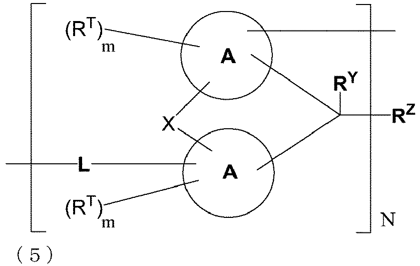 wherein
R^{Y}, R^{Z}, A, R^{T}, X, and N are as defined in claim 1; and
each m is independently an integer of 0 to 8;
wherein
at least one selected from A, R^{Y}, R^{Z}, R^{T} and X contains a sulfur atom;
at least one R^{T} contains a thiol group, a hydroxy group, or a group in which a hydrogen atom of a thiol group or a hydroxy group is replaced with an acid crosslinking group or an acid dissociation group;
when N is an integer of 2 or larger, N structural formulas within the parentheses [ ] are the same or different; and
L is an alkylene group having 1 to 30 carbon atoms and optionally having a substituent and/or a heteroatom, an arylene group having 6 to 30 carbon atoms and optionally having a substituent and/or a heteroatom, an alkoxylene group having 1 to 30 carbon atoms and optionally having a substituent and/or a heteroatom, or a single bond, wherein the alkylene group, the arylene group and the alkoxylene group each optionally contain an ether bond, a thioether bond, a ketone bond or an ester bond.

11. A resin comprising the compound according to any one of claims 3 to 8 as a constituent unit.

12. The resin according to claim 11, wherein the resin has a structure represented by the following formula (6): 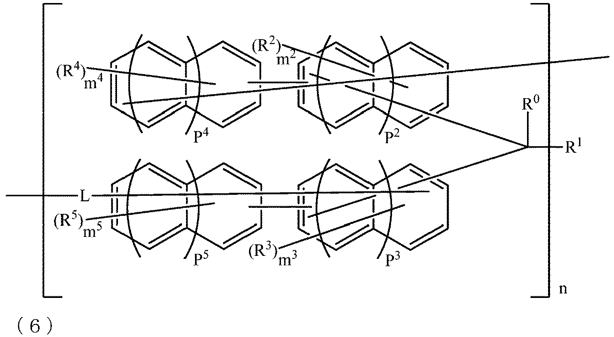 wherein
L is a linear or branched alkylene group having 1 to 30 carbon atoms and optionally having a substituent, or a single bond;
R⁰ is as defined in R^{Y} in claim 3;
R¹ is an n-valent group having 1 to 60 carbon atoms or a single bond;
R² to R⁵ are each independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one of R² to R⁵ is a thiol group;
m² and m³ are each independently an integer of 0 to 8;
m⁴ and m⁵ are each independently an integer of 0 to 9,
provided that m², m³, m⁴, and m⁵ are not 0 at the same time;
n is as defined in N in claim 3, wherein when n is an integer of 2 or larger, n structural formulas within the parentheses [ ] are the same or different; and
p² to p⁵ are as defined in r in claim 3.

13. The resin according to claim 11, wherein the resin has a structure represented by the following formula (7): 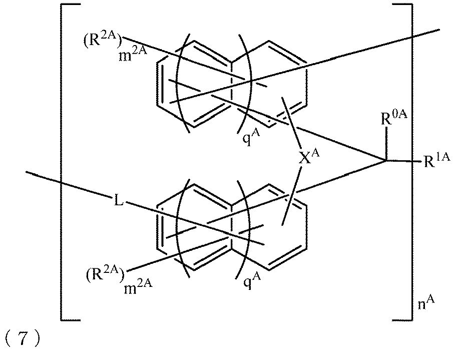 wherein
L is a linear or branched alkylene group having 1 to 30 carbon atoms and optionally having a substituent, or a single bond;
R^{0A} is as defined in R^{Y} in claim 3;
R^{1A} is an n^{A}-valent group having 1 to 60 carbon atoms or a single bond;
each R^{2A} is independently an alkyl group having 1 to 30 carbon atoms and optionally having a substituent, an aryl group having 6 to 30 carbon atoms and optionally having a substituent, an alkenyl group having 2 to 30 carbon atoms and optionally having a substituent, an alkynyl group having 2 to 30 carbon atoms and optionally having a substituent, an alkoxy group having 1 to 30 carbon atoms and optionally having a substituent, a halogen atom, a cyano group, a thiol group, a hydroxy group, or a group in which a hydrogen atom of a hydroxy group is replaced with an acid dissociation group, wherein at least one R^{2A} is a thiol group;
n^{A} is as defined in N in claim 3, where when n^{A} is an integer of 2 or larger, n^{A} structural formulas within the parentheses [ ] are the same or different;
X^{A} is an oxygen atom, a sulfur atom or not a crosslink;
each m^{2A} is independently an integer of 0 to 7, provided that at least one m^{2A} is an integer of 1 to 6; and
each q^{A} is independently 0 or 1.

14. A composition comprising one or more selected from the group consisting of the compound according to any one of claims 1 to 8 and the resin according to any one of claims 9 to 13.

15. The composition according to claim 14, further comprising a solvent.

16. The composition according to claim 14 or 15, further comprising an acid generating agent.

17. The composition according to any one of claims 14 to 16, further comprising an acid crosslinking agent.

18. The composition according to any one of claims 14 to 17, further comprising a radical generating agent.

19. The composition according to any one of claims 14 to 18, wherein the composition is used in film formation for lithography.

20. The composition according to claim 19, wherein the composition is used in resist underlayer film formation.

21. The composition according to claim 19, wherein the composition is used in resist film formation.

22. The composition according to claim 19, wherein the composition is used in resist permanent film formation.

23. The composition according to any one of claims 14 to 18, wherein the composition is used in optical component formation.

24. A method for forming a resist pattern, comprising the steps of:
forming a photoresist layer on a substrate using the composition according to claim 19; and
irradiating a predetermined region of the photoresist layer with radiation for development.

25. A method for forming an insulating film, comprising the steps of:
forming a photoresist layer on a substrate using the composition according to claim 19; and
irradiating a predetermined region of the photoresist layer with radiation for development.

26. A method for forming a resist pattern, comprising the steps of:
forming an underlayer film on a substrate using the composition according to claim 19;
forming at least one photoresist layer on the underlayer film; and
irradiating a predetermined region of the photoresist layer with radiation for development.

27. A method for forming a circuit pattern, comprising the steps of:
forming an underlayer film on a substrate using the composition according to claim 19;
forming an intermediate layer film on the underlayer film using a resist intermediate layer film material;
forming at least one photoresist layer on the intermediate layer film;
irradiating a predetermined region of the photoresist layer with radiation for development, thereby forming a resist pattern;
etching the intermediate layer film with the resist pattern as a mask;
etching the underlayer film with the obtained intermediate layer film pattern as an etching mask; and
etching the substrate with the obtained underlayer film pattern as an etching mask, thereby forming a pattern on the substrate.

28. A method for purifying the compound according to any one of claims 1 to 8 or the resin according to any one of claims 9 to 13, comprising:
an extraction step in which extraction is carried out by bringing a solution containing the compound or resin, and an organic solvent that does not inadvertently mix with water into contact with an acidic aqueous solution.
